# EUROPEAN PATENT APPLICATION

(11) **EP 4 212 528 A1**
(43) Date of publication of application: **19.07.2023**
(21) Application number: 21866065.2
(22) Date of filing: 10.09.2021
(51) Int. Cl.: C07D 407/12, A61K 31/19, C07D 405/00, C07D 407/00, C07D 307/00, A61P 3/08, A61P 3/10

(54) **BENZO OXYGEN-CONTAINING HETEROCYCLIC COMPOUND AND MEDICAL APPLICATION THEREOF**

(30) Priority: 10.09.2020 CN 202010947909
(71) Applicant: AB Pharma Ltd., Shanghai 201108 (CN)
(72) Inventor: ZHAN, Zhengyun, Shanghai 201108 (CN)
(74) Representative: Biggi, Cristina
(86) International application number: PCT/CN2021/117623
(87) International publication number: WO 2022/053013

(57) **Abstract**

Disclosed in the present invention are a compound represented by formula Ib or lib, a cis or trans isomer, enantiomer, diastereomer, racemate, tautomer, solvate, hydrate, and pharmaceutically acceptable salt thereof, or a mixture thereof, a pharmaceutical composition containing the compound, and a use of the compound as a GPR40 agonist, wherein n, E, G¹, L¹, L², R¹, R², R³, R⁴, R⁵, R^{5b}, R⁶, R⁷, X⁵, X⁶, X⁷, Y, Y¹, Z, Z¹, and the possible isotopic substitution label of each element in the compound are as defined in the description.

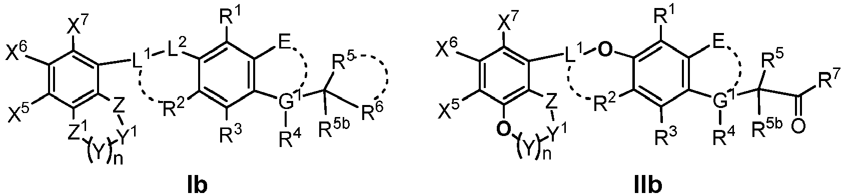

## Description

### TECHNOLOGY FIELD OF THE INVENTION

The present invention relates to a new benzo oxygen-containing heterocyclic compounds, especially for a benzo oxygen-containing five-membered heterocyclic compounds. The described compounds can be used as agonists of the GPR40 target to lower the blood glucose levels by stimulating the release of insulin from pancreatic β cells for the safe and effective treatment of diseases such as type II diabetes.

### BACKGROUND OF THE INVENTION

Diabetes is a chronic endocrine and metabolic disease characterised by hyperglycaemia due to defective insulin secretion, insulin resistance or both. Diabetes can severely damage all major organ systems in the body, causing heart disease, stroke, nerve damage, kidney failure, blindness and infections that may lead to amputation, and the disease is associated with numerous complications and high rates of disability and mortality.

Type II diabetes accounts for about 90% of all cases of diabetes and patients are usually able to produce their own insulin, but are unable to produce enough or to utilise it properly. The medications commonly used clinically to treat diabetes include insulin stimulating agents which are currently the first line hypoglycemic agents, such as sulphonylureas such as glipizide and non-sulphonylurea drugs such as metformin. The main side effects common to glucose-lowering drugs are gastrointestinal discomfort, oedema and hypoglycaemia. Some patients may suffer from a strong feeling of fasting, cold sweats, general weakness, palpitations, trembling hands and feet, blurred eyes, headaches, dizziness and, in severe cases, coma due to the side effects of hypoglycaemia. Therefore, the development of new drugs against type II diabetes that are safe and effective, free of hypoglycaemic side effects, easy to take orally is still the direction scientists are trying to explore.

Recent studies have identified free fatty acid receptor 1 (FFAR1), or G protein-coupled receptor 40 (GPR40), as playing a key role in the stimulation and regulation of insulin production. When glucose and fatty acids are elevated in the blood after a meal, GPR40 agonists lower blood glucose levels by stimulating insulin release from pancreatic beta cells. Drugs that activate GPR40 are therefore effective in controlling blood glucose levels by helping diabetics to release more insulin. These drugs have the characteristic of promoting insulin secretion only at high blood glucose levels, thus greatly reducing the risk of hypoglycaemia. A representative of this group is TAK-875, a selective GPR40 agonist developed by Takeda, Japan, which has entered phase III clinical trials and has shown no effect on insulin secretion when blood glucose levels are normal. Clinically, the dose of 50 mg administered once daily was more effective and well tolerated by patients and its risk of causing hypoglycaemia was significantly lower than that of the control sulphonylureas, verifying that TAK-875-induced insulin secretion is glucose-dependent. Although TAK-875 has shown good efficacy, Takeda Japan terminated its clinical studies in 2013 due to problems with hepatotoxicity from the drug. It is worth mentioning that studies on the hepatotoxicity of TAK-875 have shown that the problem is not related to the mechanism of action of the drug, but is mainly a problem caused by the lack of safety in the design of its molecular structure. Therefore, this GPR40 target is still a good development idea with very important application prospects.

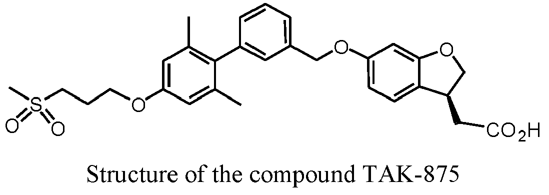

A series of patent applications for GPR40 agonists are currently disclosed, including WO2005087710, WO2007106469A1, WO2004106276A1, WO2010143733A1, CN103030646A1, WO2013104257A1, WO2015062486A1 and others.

Thus, despite the advances that have been made in the field, there remains a critical need in the field for GPR40 agonists that specifically activate GPR40 targets for the treatment of diabetes and related metabolic diseases, as well as associated compositions and methods of disease treatment. The present invention is dedicated to meeting that need and providing other related benefits.

### SUMMARY OF THE INVENTION

The present invention relates to compounds that activate the GPR40 target, as well as cis-trans isomers, enantiomers, diastereoisomers, racemates, tautomer, solvate, hydrates, or pharmaceutically acceptable salts of said compounds or mixtures thereof. The invention also relates to pharmaceutically acceptable compositions comprising said compounds, and related methods for the treatment of conditions that derive beneficial effects from activation of the GPR40 target, such as diabetes and related metabolic diseases.

The present invention provides a benzo oxygen-containing heterocyclic compound with a different structure from the existing ones. The benzo oxygen-containing five-membered heterocyclic compound of the present invention has better inhibitory activity against type II diabetes and can be used more effectively in the treatment of patients with type II diabetes and has a better safety profile.

In the first aspect,the present invention provides a compound represented by formula Ib, its cis-trans isomer, enantiomer, diastereoisomer, racemate, tautomer, solvate, hydrate, or pharmaceutically acceptable salt, or mixtures thereof; 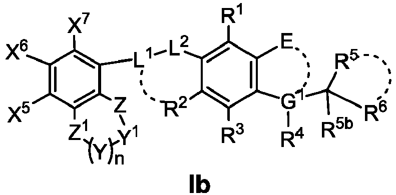 wherein,
n = 0, 1 or 2; n = 0, 1 or 2;
When n = 0, Y does not exist and Y¹ is directly single bonded to Z¹ adjacent (ortho-) to Y to form a five-membered heterocyclic group;
When E is not directly linked to G¹ to form a cyclic compound, E is selected from: hydrogen, deuterium(D), halogen, trifluoromethyl, trifluoromethoxy, nitrile, hydroxyl, carboxyl, amino(NH₂), aminocarbonyl (H₂NCO), alkyl, alkoxy, alkoxycarbonyl, alkylcarbonylamino, alkylaminocarbonyl, aryl, aryloxy, or heterocyclic aryl; G¹ is CH, or C(Rb), wherein Rb is hydrogen, deuterium(D), alkyl, cycloalkyl, alkenyl, alkynyl alkoxy, cycloalkoxy, alkoxycarbonyl, alkylaminyl, cycloalkylaminyl, alkylaminylcarbonyl, cycloalkylaminylcarbonyl, aryl, aryloxy, heterocyclic, heterocyclic aryl, or heterocyclic aryloxy, or Rb and R⁴ may be interconnected to form a cycloalkyl, or heterocyclic group;
When E is linked to G1 to form a cyclic compound, G¹ is -C-; E is -O-, -C(RcRd)-, -OC(RcRd)-, -C(RcRd)O-, or -NRe-, wherein Rc and Rd are hydrogen, deuterium(D), alkyl, cycloalkyl, alkenyl, alkyl, alkoxy, cycloalkoxy, alkoxycarbonyl, alkylamino, cycloalkylamino, alkylaminocarbonyl, cycloalkylaminocarbonyl, aryl, aryloxy heterocyclic, heterocyclic aryl, or heterocyclic aryloxy, Rc and Rd may be interconnected to form a cycloalkyl, or heterocyclic group; Re is hydrogen, deuterium(D), alkyl, cycloalkyl, alkylcarbonyl, alkoxycarbonyl, cycloalkoxycarbonyl, alkylaminocarbonyl, cycloalkylaminocarbonyl, alkylsulfonyl, or aryl sulfonyl group.
L¹ and L² are each independently -O-, -S-, -C(O)-, -S(O)₂-,-CH₂-, -C(R_{f}R_{g})-, -OC(R_{f}R_{g})-, -C(R_{f}R_{g})O-, -N(Re)-, -N(Rc)C(R_{f}R_{g})-, or-C(R_{f}R_{g})N(Re)-; wherein R_{f} and R_{g} are hydrogen, deuterium(D), alkyl, cycloalkyl, alkenyl, alkynyl, alkoxy, cycloalkoxy, alkoxycarbonyl, alkylaminyl, cycloalkylaminyl, alkylaminylcarbonyl, cycloalkylaminylcarbonyl, aryl, aryloxy, heterocyclic, heterocyclic aryl, or heterocyclic aryloxy, respectively, and Re is defined as the same as that described for Re in E above;
R¹, R²and R³ are each independently hydrogen, deuterium(D), halogen, trifluoromethyl, trifluoromethoxy, nitrile, hydroxyl, aminocarbonyl (H₂NCO), alkyl, alkoxy, alkoxycarbonyl, alkylaminocarbonyl, alkylcarbonylamino, aryl, aryloxy, or heterocyclic aryl group; wherein R² and L¹ in formula **Ib** may be interconnected to form a 4~8-membered heterocyclic compound, and L¹ is -CH-.
R⁴, R⁵ and R^{5b} are each independently hydrogen, deuterium(D), halogen, hydroxyl, amino, nitrile, alkyl, alkoxy, cycloalkyl, heterocycloalkyl, cycloalkoxy, optionally substituted alkenyl, optionally substituted alkynyl, alkoxycarbonyl, alkylaminocarbonyl, alkylcarbonylamino, alkoxycarbonylamino, aryl, aryloxy, or heterocycloaryl; wherein R⁵ and R^{5b} may be interconnected as cycloalkyl, heterocycloalkyl, or heterocycloaryl group;
R⁶ is a carboxyl group, an alkoxycarbonyl group, an aryloxycarbonyl group, an alkylaminocarbonyl group, a cycloalkylaminocarbonyl group, an alkylsulfonamido carbonyl group, a cycloalkylsulfonamido carbonyl group, a heterocyclic group, or a heterocyclic aryl group; or R⁶ and the adjacent (ortho-) substituent R⁵ may be interconnected to become a heterocyclic group, or a heterocyclic aryl group.
X⁵, X⁶ and X⁷ are each independently hydrogen, deuterium(D), halogen, nitrile, amino, trifluoromethyl, trifluoromethoxy, aminocarbonyl (H₂NCO), alkyl, heterocycloalkyl, alkoxy, heteroatomically substituted alkoxy, alkylaminyl (NRᵢRⱼ, heteroatomically substituted alkylaminyl, alkoxycarbonyl, alkylaminocarbonyl, alkylcarbonylaminyl, alkoxycarbonylaminyl, cyclo alkoxycarbonylamino group, alkyl sulfonamido group, cycloalkyl sulfonamido group, aryl group, aryloxy group, aryl amido carbonyl group, aryl carbonyl amido group, aryloxy carbonyl amido group, heterocyclic aryl group, heterocyclic aryloxy group, or heterocyclic aryl amido group, wherein Rᵢ and Rⱼare each independently hydrogen, deuterium(D), alkyl, heterocycloalkyl, alkylcarbonyl, alkoxycarbonyl, cycloalkoxycarbonyl, alkylaminocarbonyl, alkylsulfonyl, cycloalkylsulfonyl, aryl, aryloxycarbonyl, arylaminocarbonyl, heterocycloaryl group, or Rᵢ and Rⱼ are interconnected to form a 3-8-membered heterocyclic group containing 1-3 heteroatoms;
Y and Y¹ are each independently -O-, -S-, -CH₂-, -CHF-, -CF₂-, -CCl₂-, -C(R_{f}R_{g})-, or -N(Re)-; wherein R_{f} and R_{g} are defined as described above for R_{f} and R_{g} in L¹, respectively, and Re is defined as described above for Re in E.
Z and Z¹ are each independently selected from: -O-, -S-, -CF₂-, -CHF-, -CF₂-, -C(R_{f}R_{g})-, -N(Re)-, or -C(O)-; wherein R_{f} and R_{g} are defined as described above for R_{f} and R_{g} in L¹, respectively, and Re is defined as described above for Re in E.

In the second aspect, the present invention provides a compound of formula **IIb**: 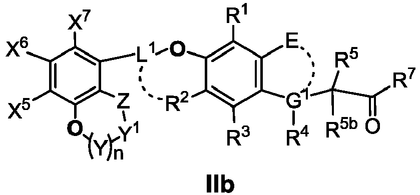 wherein;
n, E, G¹, L¹, R¹, R², R³, R⁴, R⁵, R^{5b}, X⁵, X⁶, X⁷, Y, Y¹, Z have the same definitions as n, E, G¹, L¹, R¹, R², R³, R⁴, R⁵, R^{5b}, X⁵, X⁶, X⁷, Y, Y¹, Z in claim 1.

R⁷ is hydroxy, alkoxy, alkylamino, cycloalkylamino, heterocyclicamino, alkylsulfonamido, cycloalkylsulfonamido, aryloxy, heterocyclic aryloxy, arylamido, or heterocyclic arylamido; or R⁷ and the adjacent (ortho-) substituent R⁵ may be interconnected to form a heterocyclic group.

In some preferred embodiments, n = 0 or 1;
when n = 0, Y does not exist and Y¹ is directly single bonded to the oxygen adjacent (ortho-) to Y to form a five-membered heterocyclic group;
When n = 1, Y is -CH2-.

When E is not directly linked to G¹ to form a cyclic compound, E is hydrogen, halogen, trifluoromethyl, trifluoromethoxy, or alkoxy; G¹ is -CH-, or -C(Rb)-, wherein Rb is hydrogen, an alkyl group, an optionally substituted alkenyl group, an optionally substituted alkynyl group, an alkoxy group, or Rb and R⁴ are interconnected to form an oxygen-containing heterocyclic group; R⁴ is hydrogen, an alkyl group, an alkoxy group, an optionally substituted alkynyl group, or R⁴ and Rb are interconnected to form an oxygen-containing heterocyclic group.
When E is directly linked to G¹ to form a cyclic compound, E is -OC(RcRd)-, G¹ is -C-, and R⁴ is hydrogen, wherein Rc and Rd are each independently hydrogen;
L¹ is-CH₂-, or when R² and L¹ in formula **IIb** are interconnected to form a 5-6-membered heterocyclic compound, L¹ is -OCH-;
R¹, R²and R³ are each independently hydrogen, halogen, alkyl or alkoxy;
R⁵ is hydrogen, halogen, hydroxyl, amino, alkylamino, alkyl or alkoxy;
R^{5b}is hydrogen, halogen, alkyl or alkoxy;
R⁷is an alkoxy, hydroxy, alkylsulfonamido, or cycloalkylsulfonamido group;
X⁵ is hydrogen, deuterium(D), halogen, nitrile, amino, trifluoromethyl, trifluoromethoxy, alkyl, alkoxy, alkylamino (NRᵢRⱼ), alkylcarbonylamino, alkylaminocarbonylamino, alkoxycarbonylamino, cycloalkoxycarbonylamino, alkylsulfonamido, cycloalkylsulfonamido, aryl, or aryloxycarbonylamino; wherein Rᵢ and Rⱼ are each independently hydrogen, alkyl, alkylcarbonyl, alkoxycarbonyl, or cycloalkoxycarbonyl group;
X⁶ and X⁷ are each independently hydrogen, deuterium(D), halogen, C₁-C₈ alkylamino group or C₁-C₈ alkoxycarbonylamino group;
Y¹ is -CH₂-, -CHF-, -CH₂-, or -C(CH₃)₂-;
Z is -O-, or -CH₂-.

In some more preferred embodiments, n = 0 and Y does not exist;
When E is not directly connected to G¹ to form a cyclic compound, E is hydrogen, or halogen; G¹ is -CH-, or -C(Rb)-, wherein Rb is alkyl, alkenyl, alkynyl or alkoxy; and R⁴ is hydrogen, alkyl or alkoxy group;
E is directly linked to G¹ in a cyclic compound, E is -OC(RcRd)-, G¹ is -C- and R⁴ is hydrogen, where Rc and Rd are each independently hydrogen;
L¹ is -CH₂-, or L¹ is -OCH- when R² and L¹ in formula **IIb** are interconnected to form a 5-6-membered heterocyclic compound;
R¹, R² and R³ are each independently hydrogen, halogen, or alkoxy group;
R⁵ is hydrogen, halogen, hydroxyl, amino, alkyl or alkoxy group;
R^{5b} is hydrogen, halogen, alkyl or alkoxy group;
R⁷ is selected from: alkoxy, hydroxy, alkylsulfonamido, or cycloalkylsulfonamido group;
X⁵ is hydrogen, deuterium(D), halogen, nitrile, amino, trifluoromethyl, trifluoromethoxy, alkyl, alkoxy, alkylamino (NRᵢRⱼ), alkylcarbonylamino, alkylaminocarbonylamino, alkoxycarbonylamino, cycloalkoxycarbonylamino, alkylsulfonamido, cycloalkylsulfonamido, aryl, or aryloxycarbonylamino; wherein Rᵢ and Rⱼare each independently hydrogen, alkyl, alkylcarbonyl, alkoxycarbonyl, or cycloalkoxycarbonyl group;
X⁶ is hydrogen, deuterium(D), halogen, C₁-C₈ alkylamino group or C₁-C₈ alkoxycarbonylamino group;
X⁷is hydrogen;
Y¹ is -CH₂-, -CHF-, -CF₂-, or -C(CH₃)₂-;
Z is -O-.

In some specific embodiments, the compound is represented by one of the following structures:

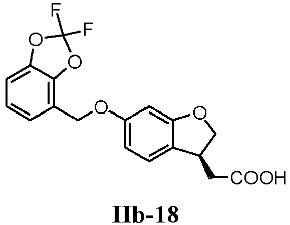

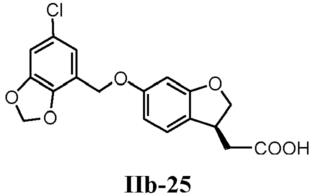

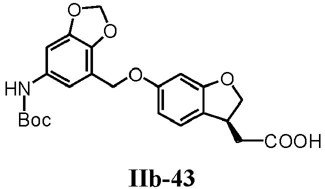

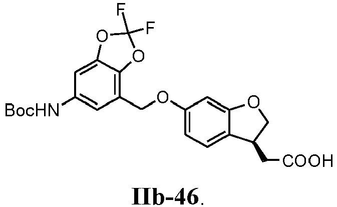

Another aspect of the invention provides a composition comprising (i) a therapeutically effective amount of at least one compound of formula Ib or IIb, its cis-trans isomer, enantiomer, diastereoisomer, racemate, tautomer, solvate, hydrate, or pharmaceutically acceptable salt or a mixture thereof; and (ii) a pharmaceutically acceptable diluent and/or excipient.

The invention also relates to use of the compounds or compositions as GPR40 agonists.

The invention also provides methods for treating or preventing diabetes or related metabolic syndromes, characterized by comprising administering to a patient a therapeutically effective amount of said compound or composition.

The invention also relates to the use of said compounds or compositions in the preparation of pharmaceuticals for the treatment or prevention of diabetes mellitus or related metabolic syndromes.

In preferred embodiments, the compounds or compositions of the present invention are particularly suitable for the treatment of type II diabetes.

The compounds of the invention promote the secretion of insulin only at high blood glucose concentrations in patients with type II diabetes,thus effectively reduce the risk of hypoglycaemia in patients. At the same time, the compounds of the present invention have a better GPR40 target selectivity and safety profile relative to commercially available drugs.

The foregoing embodiments and other aspects of the present invention will be apparent in the following detailed description. Wherefore, various references are set forth herein which describe in greater detail certain background information, processes, compounds and/or compositions and are each incorporated herein by reference in their entirety.

### DETAILED DESCRIPTION OF THE INVENTIONS

In the following description, a number of specific details are stated in order to provide a thorough understanding of the various embodiments of the invention. However, it should be understood by those skilled in the art that the invention can be implemented without requiring these details. Unless the context indicates otherwise, throughout the specification and claims of the invention, the word "comprising" and variations thereof. For example, the words "comprising" and "including" are understood to be in an open, combinable form (i.e., "including (comprising) but not limited to").

References in the specification to "an embodiment" or "an embodiment" indicate that the specific features, structures or characteristics described together with the embodiment are included in at least one embodiment of the invention. Thus, the phrases "in an embodiment" or "in an embodiment" appearing throughout the specification do not necessarily all relate to the same embodiment. Furthermore, specific features, structures, or properties may be combined in any suitable manner in one or more embodiments.

### I. DEFINITIONS

In the present invention, "alkyl", when not specifically designated, means a branched and straight chain saturated aliphatic hydrocarbon group comprising from one to twenty carbon atoms and consisting only of carbon and hydrogen atoms.In preferred embodiments, said alkyl groups have one to twelve carbon atoms (C₁-C₁₂ alkyl), one to eight carbon atoms (C₁-C₈ alkyl) or one to six carbon atoms (C₁-C₆ alkyl) and are attached to the remainder of the molecule by a single bond. Exemplary alkyl groups include: methyl, ethyl, n-propyl, isopropyl, n-butyl, tert-butyl, isobutyl, pentyl, hexyl, heptyl, octyl, and their various isomers, etc. The alkyl groups are optionally substituted with groups selected from the group consisting of: deuterium (D), halogen, trifluoromethyl, trifluoromethoxy, nitrile, hydroxyl, carboxyl, amino (NH₂), aminocarbonyl (H₂NCO), alkyl, alkoxy, alkoxycarbonyl, alkylcarbonylamino, alkylaminocarbonyl, cycloalkyl, cycloalkoxy, cycloalkoxycarbonyl, cycloalkylamino, cycloalkylaminocarbonyl, cycloalkenyl, cycloetheryl, heterocyclic, alkylureido, aryl, aryloxy, heterocyclic aryl, heterocyclic aryloxy, fused-ring aryl, fused hetero-ring aryl, fused-ring oxy, fused-ring aryloxy, fused-ring heterocyclic aryloxy, aryl ureido, or heterocyclic aryl ureido.

In the present invention, when not specifically designated, the term "alkylidene", where "alkylidene" means a second-order group derived from an alkyl group comprising 1 to 20 carbon atoms with one hydrogen atom removed, such as methylene, ethylene, propylidene, etc., is used when not specifically specified.

In the present invention, when not specifically designated, the term "aryl" is meant any stable monocyclic, bicyclic, tricyclic or tetracyclic hydrocarbon ring group comprising up to seven carbon atoms per ring, at least one of which is an aromatic ring. Exemplary aryl groups are hydrocarbon ring system groups comprising hydrogen and 6-9 carbon atoms and at least one aromatic ring; hydrocarbon ring system groups comprising hydrogen and 9-12 carbon atoms and at least one aromatic ring; hydrocarbon ring system groups comprising hydrogen and 12-15 carbon atoms and at least one aromatic ring; or hydrocarbon ring system groups comprising hydrogen and 15-18 carbon atoms and at least one aromatic ring. For the purposes of the present invention, aryl groups may be monocyclic, bicyclic, tricyclic or tetracyclic ring systems, which may include dense or bridged ring systems. Aryl groups include, but are not limited to, aryl groups derived from the following compositions: benzene, biphenyl, anthracene, azulene, fluorene, indene, indene, naphthalene, phenanthrene, pyrene, etc. "optionally substituted aryl" means: an aryl group or a substituted aryl group. The term aryl group may optionally be substituted with a group selected from the group consisting of: deuterium(D), halogen, trifluoromethyl, trifluoromethoxy, nitrile, hydroxyl, carboxyl, amino(NH₂), aminocarbonyl (H₂NCO), alkyl, alkoxy, alkoxycarbonyl, alkylcarbonylamino, alkylaminocarbonyl, alkyl-sulfonyl-alkoxy, cycloalkyl, cycloalkoxy, cycloalkoxycarbonyl, cycloalkylamino, cycloalkyl aminocarbonyl, cycloalkenyl, cycloetheryl, heterocyclic, aryl, aryloxy, heterocyclic aryl, fused-ring aryl, fused-ring alkyl, fused-ring oxy, unsubstituted or benzene or biphenyl groups containing 1 to 4 of the above optional substituents.

In the present invention, when not specifically designated, the term"heterocyclic aryl" means a stable monocyclic, bicyclic or tricyclic ring comprising up to 7 atoms each, at least one of which is an aromatic ring, and at least one of which contains 1-4 heteroatoms selected from O, N, and/or S. Heterocyclic aryl groups within this definition include, but are not limited to: acridinyl, carbazolyl, cinnolinyl, quinoxalinyl, pyrazolyl, indolyl, benzotriazolyl, furanyl, thiophenyl, benzothiazolyl, benzothiophenyl, benzofuranyl, quinolinyl, isoquinolinyl, oxazolyl, isoxazolyl, pyrazinyl, pyridazinyl, pyridinyl, pyrimidinyl, pyrrole, tetrahydroquinoline. In addition, "heterocyclic aryl" should also be understood to include any quaternary ammonium salt or N-oxide derivative of a nitrogen-containing heterocyclic aryl group. Said heterocyclic aryl group may optionally be substituted with a group selected from the group consisting of: deuterium (D), halogen, trifluoromethyl, trifluoromethoxy, nitrile, hydroxyl, carboxyl, amino (NH₂), aminocarbonyl (H₂NCO), alkyl, alkoxy, alkoxycarbonyl, alkylcarbonylamino, alkylaminocarbonyl, cycloalkyl, cycloalkoxy, cycloalkoxycarbonyl, cycloalkylamino, cycloalkylaminocarbonyl, cycloalkenyl, cycloalkenyl, cycloetheryl, heterocyclic, heterocyclic, heterocyclic. cyclic ether group, heterocyclic group, aryl group, aryloxy group, heterocyclic aryl group, fused-ring aryl group, fused-ring alkyl group, fused-ring oxy group, unsubstituted or benzene or biphenyl group containing 1 to 4 of the above optional substituents.

In the present invention, when not specifically designated, the term "fused-ring aryl "means a stable bicyclic or tricyclic ring comprising up to 7 atoms each, at least one of which is an aromatic ring. Said fused-ring aryl groups may optionally be substituted with a group selected from the group consisting of: Deuterium (D), halogen, trifluoromethyl, trifluoromethoxy, nitrile, hydroxyl, carboxyl, amino (NH₂), aminocarbonyl (H₂NCO), alkyl, alkoxy, alkoxycarbonyl, alkylcarbonylamino, alkylaminocarbonyl, cycloalkyl, cycloalkoxy, cycloalkoxycarbonyl, cycloalkylamino, cycloalkylaminocarbonyl, cycloalkenyl, cycloether, heterocyclic, aryl, aryloxy, heterocyclic aryl, fused-ring aryl, fused-ring alkyl, fused-ring oxy, unsubstituted or benzene or biphenyl groups containing 1 to 4 of the above optional substituents.

In the present invention, when not specifically designated, the term "dense ring heterocyclic aryl" means a stable bicyclic or tricyclic ring comprising up to 7 atoms each, wherein at least one ring is an aromatic ring and contains 1-4 heteroatoms selected from O, N, and/or S. The fused-ring heterocyclic aryl groups may optionally be substituted with a group selected from the group consisting of: Deuterium (D), halogen, trifluoromethyl, trifluoromethoxy, nitrile, hydroxyl, carboxyl, amino (NH₂), aminocarbonyl (H₂NCO), alkyl, alkoxy, alkoxycarbonyl, alkylcarbonylamino, alkylaminocarbonyl, cycloalkyl, cycloalkoxy, cycloalkoxycarbonyl, cycloalkylamino, cycloalkylaminocarbonyl, cycloalkenyl, cycloether, heterocyclic, aryl, aryloxy, heterocyclic aryl, fused-ring aryl, fused-ring alkyl, fused-ring oxy, unsubstituted or benzene or biphenyl groups containing 1 to 4 of the above optional substituents.

In the present invention, when not specifically designated, the term "alkoxy" refers to the group formed when an alkyl group is attached to an oxygen atom, i.e. " 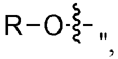 and R is an alkyl group, which is defined as described above for alkyl groups. Examples of alkoxy groups include, but are not limited to: -O-methyl (methoxy), -O-ethyl (ethoxy), -O-propyl (propoxy), -O-isopropyl (isopropoxy), -O-tert-butyl (tert-butoxy), etc.

In the present invention, when not specifically designated, the term"Alkenyl" means unsaturated aliphatic alkenyl containing 1 to 3 "carbon-carbon double bonds" in branched and straight chains of 2 to 20 carbon atoms, preferably 2 to 10 carbon atoms (C₂-C₁₀ alkenyl), more preferably 2 to 8 carbon atoms (C₂-C₈ alkenyl) or 2 to 6 carbon atoms (C₂-C₆ alkenyl), and their various isomers, etc., which are attached to the remaining part of the molecule by single bonds. Examples of alkenyl groups include, but are not limited to: vinyl, propylenyl, butenyl, pentenyl, hexenyl, etc. Said alkenyl may optionally be substituted with a group selected from the group consisting of: deuterium (D), halogen, trifluoromethyl, trifluoromethoxy, nitrile, hydroxyl, carboxyl, amino (NH₂), aminocarbonyl (H₂NCO), alkyl, alkoxycarbonyl, alkylcarbonylamino, alkylaminocarbonyl, cycloalkyl, cycloalkoxy, cycloalkoxycarbonyl, cycloalkylamino, cycloalkylaminocarbonyl, cycloalkenyl, cycloetheryl, heterocyclic alkyl, alkyl ureido, aryl, heterocyclic aryl, fused-ring aryl, fused-ring heterocyclic aryl, aryl ureido, or heterocyclic aryl ureido.

In the present invention, when not specifically designated, the term "alkynyl" means unsaturated aliphatic alkynyl containing 1 to 2 "carbon-carbon triple bonds" in branched and straight chains including 2 to 20 carbon atoms, preferably 2 to 10 carbon atoms (C₂-C₁₀ alkynyl), more preferably 2 to 8 carbon atoms (C₂-C₈ alkynyl) or 2 to 6 carbon atoms (C₂-C₆ alkynyl), and their various isomers, etc., which are connected to the remaining part of the molecule by single bonds. Examples of alkynyl groups include, but are not limited to: ethynyl groups, propargyl groups, butynyl groups, pentynyl groups, hexynyl groups, etc. The alkynyl group may optionally be substituted with a group selected from the group consisting of: deuterium(D), halogen, trifluoromethyl, trifluoromethoxy, nitrile, hydroxyl, carboxyl, amino(NH₂), aminocarbonyl (H₂NCO), alkyl, alkoxycarbonyl, alkylcarbonylamino, alkylaminocarbonyl, cycloalkyl, cycloalkoxy, cycloalkoxycarbonyl, cycloalkylamino, cycloalkylaminocarbonyl, cycloalkenyl, cycloether, heterocyclic, alkylureido, aryl, heterocyclic aryl, fused-ring aryl, fused-ring heterocyclic aryl, arylureido, or heterocyclic arylureido.

In the present invention, unless otherwise specified, "alkylthio" denotes the group formed when an alkyl group is attached to a sulfur atom, i.e. " 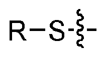 ", and R is an alkyl group, which is defined in the same way as described above for alkyl groups.

In the present invention, when not specifically designated, the term "aryloxy" means the group formed by the attachment of an aryl group to an oxygen atom, i.e. " 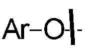 ", and Ar is an aryl group, which is defined in the same way as described above for aryl groups.

In the present invention, unless otherwise specified, the term "arylamino group" refers to an amino group in which one of the hydrogens in "NH3" is replaced by an aryl group, where aryl is defined in the same way as described above for aryl.

In the present invention, unless otherwise specified, "heterocyclic aromatic amino group" means an amino group in which one of the hydrogens in "NH3" is replaced by a heterocyclic aromatic group, where the definition of heterocyclic aromatic group is the same as described above for heterocyclic aromatic groups.

In the present invention, when not specifically designated, the term "dense-ring aryl"meansan all-carbon monocyclic or polycyclic group, wherein each ring does not contain a double or triple bond. Preferably, the ring has 3 to 20 carbon atoms, has 3 to 15 carbon atoms, preferably has 3 to 10 carbon atoms, 3 to 8 carbon atoms, 3 to 6 carbon atoms, 3 to 5 carbon atoms, a ring with 4 carbon atoms, or a ring with 3 carbon atoms. Examples of cycloalkyl include, for example, cyclopropyl, cyclobutyl, cyclopentyl, cyclohexyl, cycloheptyl, cyclooctyl, and the like. Said cycloalkyl group may optionally be substituted with a group selected from the group consisting of deuterium (D), halogen, trifluoromethyl, trifluoromethoxy, nitrile, hydroxyl, carboxyl, amino (NH₂), aminocarbonyl (H₂NCO), alkyl, alkoxy, alkoxycarbonyl, alkylcarbonylamino, alkylaminocarbonyl, cycloalkyl, cycloalkoxy, cycloalkoxycarbonyl, cycloalkylamino, cycloalkylaminocarbonyl, cycloalkenyl, cycloalkenyl cyclic ether group, heterocyclic group, alkyl ureido group, aryl group, aryloxy group, heterocyclic aryl group, heterocyclic aryl oxy group, fused-ring aryl group, fused-ring heterocyclic aryl group, fused-ring oxy group, fused-ring aryl oxy group, fused-ring heterocyclic aryl oxy group, aryl ureido group, or heterocyclic aryl ureido group, wherein the definition of aryl group is the same as described above for aryl group.

In the present invention, when not specifically designated, the term"cycloalkenyl" means an all-carbon monocyclic or polycyclic group in which one or each ring may contain one or more "carbon-carbon double bonds". Preferably, the ring has 3 to 20 carbon atoms, has 3 to 15 carbon atoms, preferably has 3 to 10 carbon atoms, 3 to 8 carbon atoms, 3 to 6 carbon atoms, 3 to 5 carbon atoms, a ring with 4 carbon atoms, or a ring with 3 carbon atoms. Examples of cycloalkenyl groups include, for example: cyclopropenyl, cyclobutenyl, cyclopentenyl, cyclohexenyl, cycloheptenyl, cyclooctenyl. Said cycloalkenyl may optionally be substituted with a group selected from the group consisting of: deuterium (D), halogen, trifluoromethyl, trifluoromethoxy, nitrile, hydroxyl, carboxyl, amino (NH2), aminocarbonyl (H2NCO), alkyl, alkoxy, alkoxycarbonyl, alkylcarbonyl, alkylaminocarbonyl, cycloalkyl, cycloalkoxy, cycloalkoxycarbonyl, cycloalkylaminocarbonyl, cycloalkylaminocarbonyl, cycloalkenyl, cycloalkenyl cyclic ether group, heterocyclic group, alkyl ureido group, aryl group, aryloxy group, heterocyclic aryl group, heterocyclic aryl oxy group, fused-ring aryl group, fused-ring heterocyclic aryl group, fused-ring oxy group, fused-ring aryl-oxy group, fused-ring heterocyclic aryl-oxy group, aryl ureido group, or heterocyclic aryl ureido group. Cycloalkyl can be formed when the substituent of the cycloalkenyl group is substituted on the carbon-carbon double bond and saturates the double bond.

In the present invention, when not specifically designated, the term "cycloether group" means to a cycloalkyl group with an ether group on the ring.

In the present invention, when not specifically designated, the term "heterocyclic group" means an aromatic or non-aromatic heterocyclic group containing one or more heteroatoms selected from O, N and S, and includes a bicyclic group. Thus, "heterocyclic group" includes the above-mentioned heterocyclic aromatic groups and their di- or tetrahydro analogs, and includes, but is not limited to, the following "heterocyclic groups": Benzimidazolyl, benzofuranyl, benzopyrazolyl, benzotriazolyl, benzothiazolyl, benzothiophenyl, benzoxazolyl, isobenzofuranyl, pyridinopyridinyl, and heterocyclic groups can be linked to other organic small molecule groups by carbon atoms or heteroatoms to form new compounds with medicinal effects.

In the present invention, when not specifically designated, the term "dense ring aryl" means two or more aryl and/or heterocyclic aryl groups, formed by fused-ring of polycyclic organic compounds, said dense ring aryl groups may also be substituted in a reasonable manner by alkyl, alkoxy, alkylthio, aryloxy, arylamino, heterocyclic, cycloalkyl, cyclic alkoxy, cycloalkoxycarbonyl, cycloalkylaminyl, cycloalkylaminylcarbonyl, cycloalkenyl, cycloetheryl, aryl, halogen, carbonyl, hydroxyl, heterocyclic aryl groups as defined in the present invention in a reasonable manner-; wherein- includes, but is not limited to, naphthalene, anthracene, quinone, phenanthrene, fluorene, benzimidazolyl, furanyl and furanyl, thiophenyl and thiophenyl, acenaphthyl, 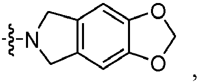 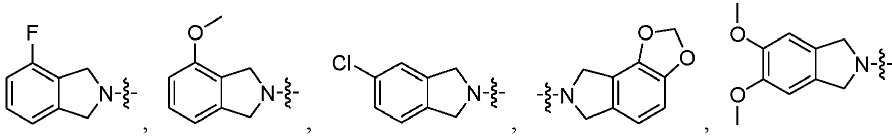 or

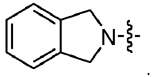

In the present invention, when not specifically designated, the term "fused cycloalkyl" refers a non-aromatic polycyclic system resulting from the reduction of one or more double bonds in a fused-ring aryl group, where the carbon number is " C₁₀-₂₀".

In the present invention, when not specifically designated, the term "fused cycloalkyl aryl" refers the group resulting from the substitution of hydrogen on the carbon of the aryl group by a fused cycloalkyl group, where the carbon number is " C₁₅-₂₀".

In the present invention, when not specifically designated, the term"fused-ring oxy group" refers a group formed by a fused-ring aryl or fused-ring alkyl group attached to oxygen, i.e. " 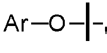 ", Ar is C₁₀-₂₀ fused-ring aryl or fused-ring alkyl group.

In the present invention, when not specifically designated, the term "alkoxycarbonyl" means the group formed when an alkoxy group is attached to a carbonyl group, i.e. 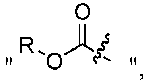 and R is a C₁-₂₀ alkyl group.

In the present invention, when not specifically designated, the term "aryloxycarbonyl" means the group formed by the attachment of an aryloxy group to a carbonyl group, i.e., 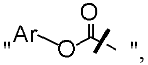 and Ar is a C₆-₂₀ aryl group.

In the present invention, when not specifically designated, the term "heterocyclic oxy" indicates the group formed by the heterocyclic group attached to oxygen, i.e. 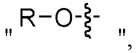 and R is a C₂-₂₀ heterocyclic group.

In the present invention, when not specifically designated, the term "alkylamino group" means to the group formed when an alkyl group is attached to an amino group, i.e., " 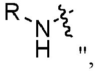 and R is a C₁-₂₀ alkyl group.

In the present invention, when not specifically designated, the term "alkylaminocarbonyl" refers to the group formed when an alkylamino group is attached to a carbonyl group, i.e., " 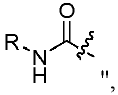 and R is a C₁-₂₀ alkyl group.

In the present invention, when not specifically designated, the term "arylamino group" refers to the group formed when an aryl group is attached to an amino group, i.e., 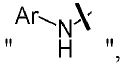 and Ar is aC₆-₂₀ aryl group.

In the present invention, when not specifically designated, the term "heterocyclic amino group" refers to the group formed when the heterocyclic group is attached to the amino group, i.e. " 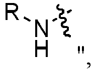 and R is aC₂-₂₀ heterocyclic group.

In the present invention, when not specifically designated, the term "aryl sulfonyl" indicates the group formed by the attachment of an arylamino group to a sulfonyl group, i.e., 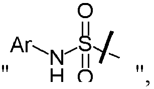 and Ar is aC₆-₂₀aryl group.

In the present invention, when not specifically designated, the term "alkylaminosulfonyl" refers to the group formed when an alkylamino group is attached to a sulfonyl group, i.e., " 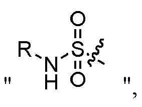 and R is a C₁-₂₀ alkyl group.

In the present invention, when not specifically designated, the term "heterocyclic amino sulfonyl" refers the group formed by the attachment of a heterocyclic amino group to a sulfonyl group, i.e., " 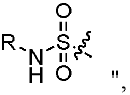 and R is a C₂-₂₀heterocyclic group.

In the present invention, when not specifically designated, the term "alkyl sulfonamido group" refers the group formed by the attachment of an alkyl group to a sulfonamido group, i.e. 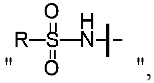 and R is a C₁-₂₀alkyl group.

In the present invention, when not specifically designated, the term "heterocyclic sulfonamido group" refers the group formed by the attachment of the heterocyclic group to the sulfonamido group, i.e. " 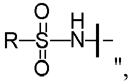 and R is a C₂-₂₀heterocyclic group.

In the present invention, when not specifically designated, the term "aryl sulfonamido group" refers to the group formed by the attachment of an aryl group to a sulfonamido group, i.e., 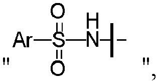 and Ar is a C₆-₂₀ aryl group.

In the present invention, when not specifically designated, the term "alkylamino sulfonamido group" refers to the group formed when an alkylamino group is attached to a sulfonamido group, i.e., " 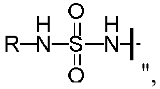 and R is a C₁-₂₀ alkyl group.

In the present invention, when not specifically designated, the term "alkyl carbonyl amino group" refers to a group formed by linking an alkyl group to a carbonyl group and then to an amino group, i.e. 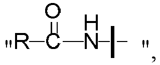 with R being a C₁-₂₀alkyl group.

In the present invention, when not specifically designated, the term "alkyl ureido" refers to the group formed by the attachment of an alkyl group to a ureido group, i.e. " 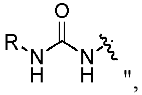 with R being a C₁-₂₀ alkyl group.

In the present invention, when not specifically designated, the term "aryl ureido" refers to the group formed by joining an aryl group with a ureido group, i.e., " 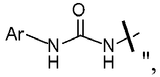 with Ar being aC₆-₂₀ aryl group.

In the present invention, when not specifically designated, the term "alkylthiourea group" refers to the group formed by joining an alkyl group with a thiourea group, i.e., 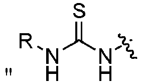 ", with R being aC₁-₂₀ alkyl group.

In the present invention, the term "halogen" refers to "fluorine, chlorine, bromine, or iodine atom".

In the present invention, the term "hydroxyl"refers to

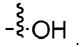

In the present invention, the term"amino group"refers to

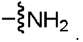

In the present invention, the term"cyano"refers to

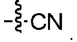

In the present invention, the term" carboxyl " refers to

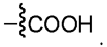

In the present invention, the term"sulfonyl" refers to

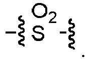

In the present invention, the term" Sulfonamido" refers to

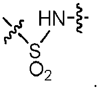

In the present invention, the term"Carbonyl" refers to

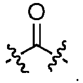

In the present invention, the term"Urea-based" refers to

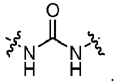

In the present invention, the term" Thiourea-based" refers to

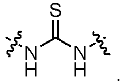

The term "halogen" refers to fluorine, chlorine, bromine and iodine.

### II. COMPOUNDS OF THE PRESENT INVENTION

The present invention was innovatively designed to introduce a heterocyclic functional group containing the following novel benzo-oxo-containing heterocyclic group: 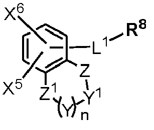 (where R⁸ is a halogen, hydroxyl, amino, carboxyl, alkyl sulfonyloxy, aryl sulfonyloxy, or a leaving group that can be substituted), and to synthesize a class of benzo oxygen-containing five-membered heterocyclic compounds that are effective as novel GPR40 target agonists for the treatment of type II diabetes.

The present invention relates generally to compounds covered by formula Ib, their cis-trans isomers, enantiomers, diastereoisomers, racemates, tautomer, solvate, hydrates, or pharmaceutically acceptable salts, or mixtures thereof:

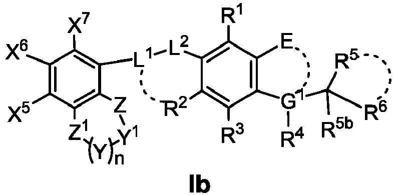

For compounds of formula Ib, n, E, G¹, L¹, L², R¹, R², R³, R⁴, R⁵, R^{5b}, R⁶, Ra, Rb, Rc, Rd, Re, Rf, Rg, Ri, Rj, X⁵, X⁶, X⁷, Y, Y¹, Z and Z 1 are as defined in the specification.

Specific embodiments of compounds of Formula Ib are also described below.

In one embodiment, n = 0, Y does not exist, and Y¹ is directly connected to Z¹ with a single bond.

In one embodiment, E is linked to G¹ to form a cyclic compound. In a preferred embodiment, E is linked to G¹ as a five-membered cyclic compound. In a preferred embodiment, G¹ is -C- and E is -O-, -C(RcRd)-, -OC(RcRd)-, or -C(RcRd)O-. In one embodiment, Rc and Rd are independently selected from: Hydrogen, deuterium(D), alkyl, cycloalkyl, alkenyl, alkynyl, alkoxy, cycloalkoxy, alkoxycarbonyl, alkylaminyl, cycloalkylaminyl, alkylaminylcarbonyl, cycloalkylaminylcarbonyl, aryl, aryloxy, heterocyclic, heterocyclic aryl, or heterocyclic aryloxy. In another embodiment, Rc and Rd may be connected to each other as a cycloalkyl or heterocyclic group. In a preferred embodiment, G1 is -C- and E is -OC(RcRd)- or -C(RcRd)O-, wherein Rc and Rd are independently selected from: hydrogen, deuterium (D), C₁-C₈ alkyl, C3-C8 cycloalkyl, C₂-C₈ alkenyl, C₂-C₈alkynyl, C₁-C₈ alkoxy, C₃-C₈cycloalkoxy, C₁-C₈ alkoxycarbonyl, C₁-C₈ alkylamino, C₃-C₈ cycloalkylamino, C₁-C₈ alkylamino carbonyl, C₃-C₈ cycloalkylamino carbonyl, aryl, aryloxy, heterocyclic, heterocyclic aryl, heterocyclic, heterocyclic aryl, or heterocyclic aryloxy. In a more preferred embodiment, G¹is -C- and E is -OC(RcRd)- or -C(RcRd)O-, wherein Rc and Rd are hydrogen, respectively.

In one embodiment, L¹ and L² are each independently selected from: -O-, -S-, -C(O)-, -SO2-, -CH2-, -C(RfRg)-, -OC(RfRg)-, -C(RfRg)O-, -N(Re)-, -N(Rc)C(RfRg)-, or -C(RfRg)N(Re)-. In a preferred embodiment, one of L¹ and L²is -CH2- and the other is -O-. In another preferred embodiment, R2 and L¹ in Formula Ib are interconnected into a 5-6-membered heterocyclic compound, with at least one of L¹and L² being -OCH-.

In one embodiment, R¹, R², and R³ are each independently hydrogen, deuterium(D), halogen, trifluoromethyl, trifluoromethoxy, nitrile, hydroxy, aminocarbonyl (H2NCO), C₁-C₈ alkyl, C₁-C₈ alkoxy, C₁-C₈ alkoxycarbonyl, C₁-C₈alkylaminocarbonyl, C₁-C₈ alkylcarbonylamino, aryl, aryloxy, or heterocyclic aryl. In preferred embodiments, R¹, R², and R³ are each independently hydrogen, halogen, or C₁-C₈ alkoxy group.

In one embodiment, R⁴and R⁵ are each independently selected from: hydrogen, halogen, hydroxyl, amino, or alkoxy. In preferred embodiments, R⁴and R⁵ are each independently hydrogen, halogen, or C₁-C₈ alkoxy group.

In one embodiment, R⁶ is -COR⁷. In a preferred embodiment, R⁷ is -OH, an alkoxy, an alkyl amino, a cycloalkyl amino, a heterocyclic amino, an alkyl sulfonamido, a cycloalkyl sulfonamido, an aryloxy, a heterocyclic aryloxy, an aryl amino, or a heterocyclic aryl amino group; or R⁷ and a adjacent (ortho-) substituent R⁵ may be interconnected to become a heterocyclic group. In a preferred embodiment, R⁷ is a C₁-C₈alkoxy, hydroxy, C₁-C₈ alkyl sulfonamido, or C₃-C₈cycloalkyl sulfonamido group.

In one embodiment, Y¹ is selected from: -oxygen-, -sulfur-, -CH₂-, -CHF-, -CF₂--CCl₂-, , -C(R_{f}R_{g})-, or -N(Re)-. In a preferred embodiment, Y¹ is selected from: CH₂, -CHF-, CF₂, or C(CH₃)₂.

In one embodiment, Z and Z¹ are independently selected from: -O-, -S-, -CH₂-, -CHF-, -CF₂-, -C(R_{f}R_{g})-, -N(Re)-, or -C(O)-; wherein R_{f}and R_{g}are defined as described above for R_{f}and R_{g}in L1, respectively, and Re is defined is the same as that described for Re in E above. In a more preferred embodiment, at least one of Z and Z¹ is -O-. In a more preferred embodiment, both Z and Z¹ are -O-.

In one embodiment, Z is -O-.

In one embodiment, Z¹ is -O-.

In one embodiment, X⁵, X⁶ and X⁷ are each independently hydrogen, deuterium(D), halogen, nitrile, amino, trifluoromethyl, trifluoromethoxy, aminocarbonyl (H₂NCO), alkyl, heterocycloalkyl, alkoxy, heteroatomically substituted alkoxy, alkylamino (NRᵢRⱼ), heteroatomically substituted alkylamino, alkoxycarbonyl, alkylaminocarbonyl, alkylcarbonylamino, alkoxycarbonyl amino group, cycloalkoxycarbonyl amino group, alkyl sulfonamido group, cycloalkyl sulfonamido group, aryl, aryloxy, aryl amino carbonyl, aryl carbonyl amino group, aryloxy carbonyl amino group, heterocyclic aryl, heterocyclic aryl oxy, or heterocyclic aryl amino group; wherein Rᵢ and Rⱼ are each independently hydrogen, deuterium(D), alkyl, heterocycloalkyl, alkyl carbonyl, alkoxy carbonyl, cycloalkoxy carbonyl, alkyl amino carbonyl, alkyl sulfonyl, cycloalkyl sulfonyl, aryl, aryloxy carbonyl, aryl amino carbonyl, heterocyclic aryl, or Rᵢand Rⱼ are interconnected to form a 3-8-membered heterocyclic group containing 1-3 heteroatoms.

In one embodiment, X⁵ is selected from: hydrogen, deuterium(D), halogen, nitrile, amino(NH2), trifluoromethyl, trifluoromethoxy, C₁-C₈ alkyl, C₁-C₈ alkoxy, C₁-C₈ alkylamino, C₁-C₈ alkylcarbonylamino, C₁-C₈ alkylamino carbonylamino, C₁-C₈ alkoxycarbonylamino, C₃-C₈ cycloalkoxycarbonylamino, C₁-C₈ alkyl sulfonamido, C₃-C₈ cycloalkyl sulfonamido, aryl, or aryloxycarbonyl amino groups. In a preferred embodiment, X⁵ is selected from: hydrogen, deuterium(D), halogen, nitrile, trifluoromethyl, trifluoromethoxy, C₁-C₈ alkoxycarbonyl amino group.

In one embodiment, X⁶ and X⁷ are each independently selected from: hydrogen, deuterium(D), halogen, C₁-C₈ alkylamino group, or C₁-C₈ alkoxycarbonylamino group. In a preferred embodiment, X⁶ is selected from: hydrogen, deuterium(D), halogen, C₁-C₈ alkylamino group or C₁-C₈ alkoxycarbonylamino group, and X⁷ is selected from: hydrogen.

The compounds of the present invention may exist in multiple isomeric forms, as well as one or more mutually variable isomeric forms, including two single mutually variable isomers, and mixtures of mutually variable isomers. The term "isomer" is intended to cover the entire range of isomeric forms of the compounds of the present invention, including the mutually variable isomeric forms of said compounds.

Some of the compounds described herein may have asymmetric centers and thus exist in different enantiomeric and diastereoisomeric forms. The compounds of the present invention may be in the form of optical isomers or diastereoisomers. Thus, the present invention covers the compounds of the present invention and their application in their optical isomeric, diastereoisomeric and mixtures thereof, including racemic mixtures, as described herein. The optical isomers of the compounds of the present invention can be obtained by known techniques, such as asymmetric synthesis, chiral chromatography, or by chemical separation of the stereoisomers using optically active splitting agents.

Unless otherwise indicated, "stereoisomer" means a stereoisomer of a compound that is substantially free of other stereoisomers of the compound. Thus, a stereoisomerically pure compound having a chiral center is substantially free of the opposite enantiomer of the compound. A stereoisomerically pure compound having two chiral centers is substantially free of other diastereoisomers of the compound. A typical stereoisomerically pure compound comprises greater than about 80 wt% of one stereoisomer of the compound and less than about 20 wt% of the other stereoisomers of the compound. For example, greater than about 90 wt% of one stereoisomer of the compound and less than about 10 wt% of the other stereoisomers of the compound, or greater than about 95 wt% of one stereoisomer of the compound and less than about 5 wt% of the other stereoisomers of the compound, or greater than about 97 wt% of one stereoisomer of the compound and less than about 3 wt% of the other stereoisomers of the compound.

If there is a discrepancy between the described structure and the name given to that structure, the described structure prevails. In addition, if the stereochemistry of a structure or part of a structure is not indicated by, for example, a bold or dashed line, the structure or part of a structure should be understood to cover its entire stereoisomerism. However, in some cases where more than one chiral center is present, the structure and name may be indicated in the form of a single enantiomer to aid in describing the relevant stereochemistry. Those skilled in the field of organic synthesis should be aware of their preparation by means of the preparation of single enantiomers of said compounds.

In this specification, the"pharmaceutically acceptable salt" is a pharmaceutically acceptable, organic or inorganic acid or base salt of a compound of the present invention. Typical pharmaceutically acceptable salts include, for example, alkali metal salts, alkaline earth metal salts, ammonium salts, water-soluble salts, and water-insoluble salts. For example, acetate, aminostilbene sulfonate (4,4-diaminostilbene 2,2-disulfonate), benzenesulfonate, benzoate, bicarbonate, hydrogen sulfate, hydrogen tartrate, borate, bromide, butyrate, calcium, calcium edetate, camphor sulfonate, carbonate, chloride, citrate, clavulanate, dihydrochloride, edetate, ethyl disulfonate, propionate lauryl sulfate, ethanesulfonate fumarate, glucosinolate, glucosinolate, glutamate, ethanoic acid asanate, hexafluorophosphate, hexylresorcinol, haramino, hydrobromide, hydrochloride, hydroxynaphthoic acid salt, iodate, isothiosulfate, lactate, lactobionate, laurate, malate, maleate, lentilate, methanesulfonate, methyl bromide, methyl nitrate, methanesulfonate, mucilage Naphthalene sulfonate, nitrate, N-methyl glucosamino ammonium salt, 3-hydroxy-2-naphthoic acid salt, oleate, oxalate, palmitate, bis-hydroxynaphthalate (1,1-methylene-bis-2-hydroxy- 3-naphthoic acid salt, bis-hydroxynaphthalate, pantothenate, phosphate/diphosphate, picric acid salt, polygalacturonate, propionate, p-toluenesulfonate, salicylate, stearate, alkali acetate salicylate, alkali acetate, succinate, sulfate, sulfosalicylate, sulforaphane, ellagic acid, tartarate, 8-chlorophyllide, toluene sulfonate, triethyl iodide and valerate. A pharmaceutically acceptable salt may have more than one charged atom in its structure. In this embodiment, the pharmaceutically acceptable salt may have a plurality of counterbalance ions. Thus, the pharmaceutically acceptable salt may have one or more charged atoms and/or one or more counterbalancing ions.

The compounds of the present invention may be isotopically labeled in which one or more atoms are replaced by atoms having a different atomic mass or mass number. Examples of isotopes that may be incorporated into a compound of Formula Ib or lib include: isotopes of hydrogen, carbon, nitrogen, oxygen, phosphorus, fluorine, chlorine, or iodine. Examples of such isotopes are respectively: ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ³⁶Cl, ¹²³I and ¹²⁵I. These radiolabeled compounds can be used to detect biodistribution, tissue concentrations, and kinetics of transport and excretion from biological tissues, including the subject to which the labeled compound is administered. The labeled compounds are also used to determine the therapeutic effect, site or mode of action, and binding affinity of the therapeutic candidate for a pharmacologically important target. Thus, certain radiolabeled compounds of formula Ib or IIb can be used for drug and/or tissue distribution studies. The radioisotopes tritium, i.e., ³H, and carbon-14, i.e., ¹⁴C, are particularly useful for this purpose because they are easy to incorporate and detection means are readily available.

Substitution with a heavy isotope such as deuterium, i.e., ²H, can provide certain therapeutic advantages due to higher metabolic stability (e.g., prolonged in vivo half-life of deuterium-containing compounds). Substitution of hydrogen with deuterium reduces the dose required to achieve therapeutic efficacy and can therefore be preferably used in discovery or clinical settings.

Substitution of positron-emitting isotopes (e.g., ¹¹C, ¹⁸F, ¹⁵O and ¹³N) can provide labeled analogs of compounds of the present invention that have been used in positron imaging (PET) studies, for example, for the detection of substance receptor occupancy. For example, for the detection of substance receptor occupancy. Isotopically labeled compounds of Formula Ib or IIb can be prepared generally by conventional techniques known to those skilled in the art or by employing suitable isotopically labeled reagents in a manner similar to those described in the preparation and embodiments section below.

Embodiments of the present invention described herein are also intended to cover in vivo metabolites of compounds of Formula Ib or IIb. These products may be derived, for example, from processes such as oxidation, reduction, hydrolysis, amidation, esterification, etc. that are primarily attributable to the enzymatic activity of the compounds of the present invention after administration. Thus, the present invention includes such compounds which are produced in the form of by-products based on the enzymatic or non-enzymatic activity exerted on the compounds of the present invention after a period of time sufficient to produce metabolites in mammals given the compounds of the present invention. Metabolites, particularly those having pharmaceutical activity, are typically identified by administering a detectable dose of a radiolabeled compound of the present invention to a subject, such as a rat, mouse, guinea pig, monkey or human, for a sufficient period of time during which metabolism occurs, and by isolating said metabolite from a urine, blood or other biological sample obtained from the subject receiving said radiolabeled compound.

The present invention also provides pharmaceutically acceptable salt forms of compounds of Formula Ib or IIb. The scope of the present invention covers acid addition salts and base addition salts, which are formed by bringing a pharmaceutically suitable acid or a pharmaceutically suitable base into contact with a compound of the present invention.

"Pharmacologically acceptable acid addition salts" means those salts that retain biological validity and free base properties, which are not undesirable biologically or otherwise, and which are formed using inorganic acids, such as, but not limited to hydrochloric acid, hydrobromic acid, sulfuric acid, nitric acid, phosphoric acid, etc., and organic acids such as, but not limited to, acetic acid, 2,2-dichloroacetic acid, adipic acid, alginic acid, ascorbic acid, aspartic acid, benzenesulfonic acid, benzoic acid, 4-acetylaminobenzoic acid, camphoric acid, camphor-10-sulfonic acid, capric acid, capric acid, octanoic acid, carbonic acid, cinnamic acid, citric acid, cyclohexane sulfamic acid, dodecyl sulfate, ethane-1, 2-disulfonic acid, ethanesulfonic acid, 2-hydroxyethanesulfonic acid, formic acid, fumaric acid, galactosuccinic acid, gentianic acid, glucuronic acid, glucuronic acid, glucuronic acid, glutamic acid, glutaric acid, 2-oxoglutaric acid, glycerophosphoric acid, glycolic acid, hippuric acid, isobutyric acid, lactic acid, lactobionic acid, lauric acid, maleic acid, malic acid, malonic acid, mandelic acid, methanesulfonic acid, muconic acid, naphthalene-1,5-disulfonic acid, naphthalene 2-sulfonic acid, 1-hydroxy-2-naphthoic acid, nicotinic acid, oleic acid, orotic acid, oxalic acid, palmitic acid, dihydroxynaphthalic acid, propionic acid, pyroglutamic acid, pyruvic acid, salicylic acid, 4-aminosalicylic acid, sebacic acid, stearic acid, succinic acid, tartaric acid, thiocyanic acid, p-toluenesulfonic acid, trifluoroacetic acid, undecylenic acid, etc.

"Pharmacologically acceptable base addition salts" are those salts that retain the biological validity and properties of the free acid and are not biologically or otherwise undesirable. These salts are prepared by adding inorganic or organic bases to the free acid. Salts derived from inorganic bases include, but are not limited to, sodium, potassium, lithium, ammonium, calcium, magnesium, iron, zinc, copper, manganese, aluminum salts, etc. Preferred inorganic salts are ammonium, sodium, potassium, calcium, and magnesium salts. Salts derived from organic bases include, but are not limited to, salts of: primary, secondary and tertiary amines, substituted amines, including naturally occurring substituted amines, cyclic amines and basic ion exchange resins such as ammonia, isopropylamine, trimethylamine, diethylamine, triethylamine, tripropylamine, diethanolamine, ethanolamine, dimethylethanolamine, 2-dimethylaminoethanol, 2-diethylaminoethanol, dicyclohexylamine, lysine, arginine, histidine, caffeine, procaine, hydantoin, choline, betaine, phenethylbenzylamine, benzylpenicillin, ethylenediamine, glucosamine, methylglucosamine, theobromine, triethanolamine, aminobutriol, purine, piperazine, piperidine, N ethyl piperidine, polyamine resin, etc. Particularly preferred organic bases are isopropylamine, diethylamine, ethanolamine, trimethylamine, dicyclohexylamine, choline, and caffeine.

Crystallization typically produces solvent complexes of the compounds of the present invention. The term "solvent complex" as used herein refers to an aggregate comprising one or more molecules of the compound of the invention and one or more molecules of the solvent. The solvent may be water, in which case the solvent complex may be a hydrate. Alternatively, the solvent may be an organic solvent. Thus, the compounds of the present invention may exist in hydrate form, including monohydrate, dihydrate, hemihydrate, sesquihydrate, trihydrate, tetrahydrate, etc., and the corresponding solvent complex forms. The compounds of the invention may be true solvent compounds, while in other cases, the compounds of the invention may retain only indefinite water or a mixture of water plus some indefinite solvent.

"Stereoisomer" means a compound which is not interchangeable by being composed of identical atoms linked by the same bonds, but having different three-dimensional structures. The invention envisages various stereoisomers and mixtures thereof, and includes "enantiomeric", which means: two stereoisomers whose molecules are non-overlapping mirror images of each other.

The compounds of the invention, or pharmaceutically acceptable salts thereof, may contain one or more asymmetric centers, resulting in enantiomers, diastereoisomers, and other stereoisomeric forms that can be identified in terms of absolute stereochemistry, such as (R)- or (S)-, or, in the case of amino acids, such as (D) or (L). The present invention is intended to include all these possible isomers, as well as their racemic and optically pure forms. The optically active (+) and (-), (R)- and (S)- or (D)- and (L)-isomers can be prepared using chiral synthons or chiral reagents, or split using conventional techniques, such as chromatography and graded crystallization. Conventional techniques used to prepare/isolate individual enantiomers include chiral synthesis from suitable optically pure precursors or the use of, for example, chiral high-pressure liquid chromatography (HPLC) to split racemates (or racemates of salts or derivatives). When a compound described herein contains an alkene double bond or other geometric asymmetry center, it is intended to indicate that the compound includes the E and Z geometric isomers unless otherwise stated. Likewise, it is intended to include the full range of tautomerforms.

### III. DRUG COMPOSITIONS

In one embodiment, a compound of Formula Ib or IIb, formulated in the form of a pharmaceutically acceptable composition, said composition comprising an amount of a compound of Formula Ib or IIb that is effective in treating a specific disease or condition of interest after administration of said pharmaceutical composition to a mammal. The pharmaceutical composition of the present invention may comprise a compound of Formula Ib or lib in combination with a pharmaceutically acceptable carrier, diluent or excipient.

For this purpose, "pharmaceutically acceptable carrier, diluent, or excipient" includes, but is not limited to, any adjuvant, carrier, excipient, flow aid, sweetener, diluent, preservative, dye/colorant, flavor enhancer, surfactant, wetting agent, dispersant, suspending agent, stabilizer, isotonic agent, solvent, or emulsifier that is approved by the U.S. Food and Drug Administration as acceptable to humans or domestic animals.

In addition, "mammal" includes human and domestic animals, e.g., laboratory animals and domestic pets (e.g., cats, dogs, pigs, cattle, sheep, goats, horses, rabbits), and non-domestic animals, e.g., wild animals, etc.

Pharmaceutical compositions of the present invention can be prepared by combining compounds of the present invention with suitable pharmaceutically acceptable carriers, diluents, or excipients, and can be formulated in solid, semi-solid, liquid, or gaseous forms, such as, for example, tablets, capsules, powders, granules, ointments, solutions, suppositories, injections, inhalations, gels, microspheres, and aerosols. Typical routes of administration of such pharmaceutical compositions include, but are not limited to, oral, topical, transdermal, inhalation, parenteral, sublingual, buccal, rectal, vaginal, and intranasal. The term parenteral as used herein includes, subcutaneous, intravenous, intramuscular, intrathoracic, or infusion techniques. The pharmaceutical compositions of the present invention are formulated to allow the active ingredient contained therein to be bioavailable after administration of the composition to a patient. The composition to be given to the subject or patient may be in the form of one or more dosage units, for example, the tablet may be a single dosage unit and the container of the compound of the invention in aerosol form may contain a plurality of dosage units. Practical methods for preparing such dosage forms are known or understood by those skilled in the art; see, for example, Remington: The Science and Practice of Pharmacy, 20th edition (Philadelphia College of Pharmacy and Science, 2000). In any case the composition to be given contains a therapeutically effective amount of a compound of the present invention or a pharmaceutically acceptable salt thereof for the treatment of a disease or condition of interest according to the teachings of the present invention.

The pharmaceutical compositions of the present invention can be in solid or liquid form. On the one hand, the carrier is a particle, whereby the composition is, for example, in the form of a tablet or a powder. The carrier may be a liquid, wherein said composition is, for example, an oral type syrup, an injectable liquid, or an aerosol, which may be used, for example, for inhalation administration. When intended for oral administration, said pharmaceutical composition is preferably in solid or liquid form, wherein, semi-solid, semi-liquid, suspension, and gel forms are included in the solid or liquid forms contemplated herein.

As solid compositions for oral administration, the pharmaceutical compositions can be formulated in the form of powders, granules, compressed tablets, pills, capsules, chewable gums, flakes, and the like. Such solid compositions will typically contain one or more inert diluents or edible carriers. In addition, one or more of the following substances may be present: binders, such as carboxymethyl cellulose, ethyl cellulose, microcrystalline cellulose, yarrow gum or gelatin; excipients, such as starch, lactose or dextrin, disintegrants, such as alginate, sodium alginate, Primogel, corn starch, etc.; lubricants, such as magnesium stearate or Sterotex; flow aids, such as colloidal silica; sweeteners, such as sucrose or saccharin; flavoring agents, such as menthol, methyl salicylate, or orange flavoring agents; and coloring agents.

If the pharmaceutical composition is in the form of a capsule (e.g., a gelatin capsule), it may contain a liquid carrier, e.g., a polyethylene glycol or an oil, in addition to the types of substances described above.

The pharmaceutical composition may be in the form of a liquid, such as an elixir, syrup, solution, emulsion or suspension. As two examples, the liquid may be for oral administration or for delivery by injection. If intended for oral administration, the preferred composition may contain one or more sweeteners, preservatives, dyes/colors, and flavor enhancers in addition to the compounds of the present invention. For compositions intended to be administered by injection, one or more of surfactants, preservatives, wetting agents, dispersants, suspension aids, buffers, stabilizers, and isotonic agents may be included.

The liquid pharmaceutical compositions of the present invention, whether they are in solution, suspension or other similar form, may comprise one or more of the following adjuvants: sterile diluents such as water for injection, saline solutions, preferably saline, Ringer's solution, isotonic sodium chloride; Non-volatile oils, such as synthetic glycerol mono- or diglycerides, polyethylene glycol, glycerol, propylene glycol or other solvents that can be used as solvents or suspension media, Antimicrobial agents, such as benzyl alcohol or methyl paraben; Antioxidants, such as ascorbic acid or sodium bisulfite; Chelating agents, such as ethylenediaminetetraacetic acid; buffers, such as acetate, citrate, or phosphate, and reagents for tension regulation, such as sodium chloride or glucose. Extragastric preparations may be encapsulated in ampoules, disposable syringes, or multi-dose vials made of glass or plastic. Physiological saline is the preferred adjuvant. Injectable pharmaceutical compositions are preferably sterile.

The pharmaceutical compositions of the present invention may be prepared by any method known to those skilled in the field of pharmacy. For example, pharmaceutical compositions intended to be given by injection may be prepared by combining the compounds of the present invention with sterile, distilled water to form a solution. A surfactant may be added to facilitate the formation of a homogeneous solution or suspension. Surfactants are compounds that interact non-covalently with the compounds of the present invention, thereby facilitating the dissolution or homogeneous suspension of said compounds in an aqueous delivery system.

### IV. THERAPEUTIC APPLICATIONS

Administration of a therapeutically effective amount of a compound of the present invention or a pharmaceutically acceptable salt thereof, the therapeutically effective amount varying according to various factors including the activity of the particular compound used, the metabolic stability and duration of action of the compound, the age, weight, general health status, gender, diet, mode and timing of administration, excretion rate, co-administration, severity of the specific disease or condition, and the recipient of the treatment.

"Effective amount" or "therapeutically effective amount" means an amount of a compound of the present invention which (when administered to a mammal, preferably a human) is sufficient to provide effective treatment of an Mnk-related condition or disease in that mammal (preferably a human), as described below. The amount of compound of the present invention that constitutes a "therapeutically effective amount" will vary depending on the compound, the condition and its severity, the manner of administration, and the age of the mammal to be treated, but can be routinely determined by those skilled in the art based on their knowledge and the content of the present invention.

The compounds of the present invention, or pharmaceutically acceptable salts thereof, may also be given prior to, concurrently with, or after the administration of one or more other therapeutic agents. Such combination therapy includes the administration of a single drug-dose formulation comprising the compound of the present invention and one or more other active substances, and the administration of the compound of the present invention and each active substance in separate drug-dose formulations. For example, the compounds of the present invention and other active substances may be given to the patient together in a single oral dose combination (e.g., tablets or capsules), or each substance may be given in separate oral dose formulations. When using separate dose formulations, the compounds of the present invention and one or more other active agents may be given at substantially the same time (i.e., simultaneously) or at separate staggered times (i.e., sequentially); it should be understood that combination therapy includes all of these regimens.

The present invention also optimizes the structure of GPR40 target agonist compound formula **Ib-IIb** by further study of structure activity relationship (SAR) and can effectively reduce the risk of hypoglycemia production for safer and more effective treatment of type II diabetes.

The benzo oxygen-containing heterocyclic compounds of the present invention can lower blood glucose levels by activating GPR40 targets to stimulate insulin release from pancreatic beta cells, and such compounds of formula Ib-IIb are characterized by promoting insulin secretion only when blood glucose concentrations are high in patients with type II diabetes, thus effectively reducing the risk of hypoglycemia in patients and providing better GPR40 target selectivity and safety.

### DETAILED DESCRIPTION OF THE INVENTION

Details of the present invention are further illustrated below by way of embodiments, but the invention is not thereby limited to the scope of the described embodiments. Experimental methods for which specific conditions are not indicated in the following embodiments are completed according to conventionally known methods and conditions, or according to the commodity specification of choice for implementation.

The abbreviated notes of the chemical reagents and solvents used in the synthesis of the novel benzo oxygen-containing heterocyclic compounds of the present invention are all summarized in the description of the apparatus and raw materials section of the Examples.

The key innovation of the present invention is to follow synthetic reaction routes 1 or 2 as shown in the following schemes 1 and 2, first preparation of the target products Ib-IIb or intermediate RM-2b by reaction of raw material RM-1b with raw material SM-1 (e.g. reagents of the following SM-1a or RM-1b structures), respectively; when R⁷ is an alkoxy group (e.g., R⁷ = OCH3 or OEt), the individual target products of the present invention in which R⁷ is a hydroxyl group in Formula **IIb** can then be synthesized by the LiOH hydrolysis reaction.

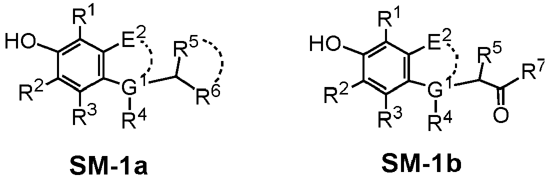

The synthetic reaction route 1b described above is as follows:

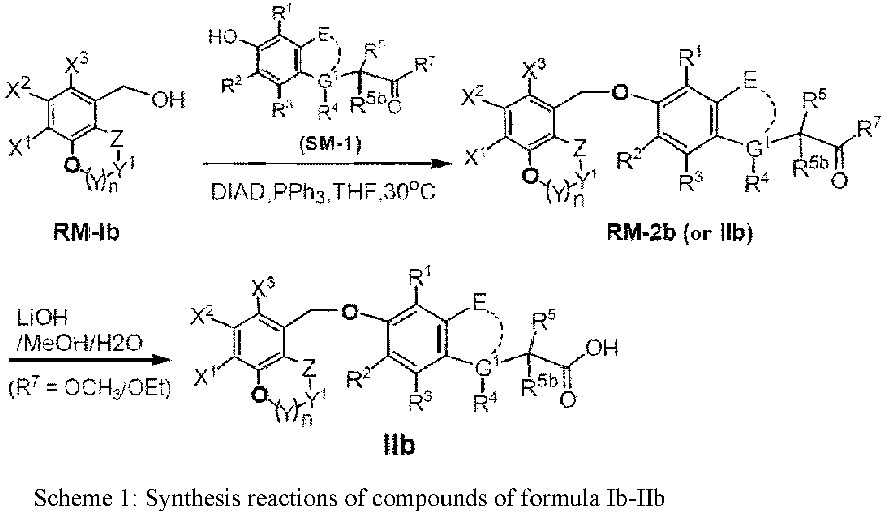

In the above synthesis reaction route 1b was operated as follows:
1. Synthesis of RM-2b: RM-Ib (1.0eq), SM-1b (1.0eq), and DIAD and PPh3 (1.2eq) were added to THF (5x) in a round bottom reaction flask, nitrogen was replaced and protected, and the reaction was followed by TLC and/or HPLC until the end of the reaction, and RM-2b (or RM-IIb) was obtained after post-treatment and other routine operations.
2. Synthesis of target products Ib-IIb: RM-Ib (or RM-IIb, 1 eq) was hydrolyzed in a mixture of MeOH-H2O with LiOH (1:1, 10X) in a round-bottom reaction flask, detected by TLC and/or HPLC until the end of the reaction, and then the target products Ib-IIb were obtained after post-treatment and purification and other routine operations.

The synthetic reaction route 2b is as follows:

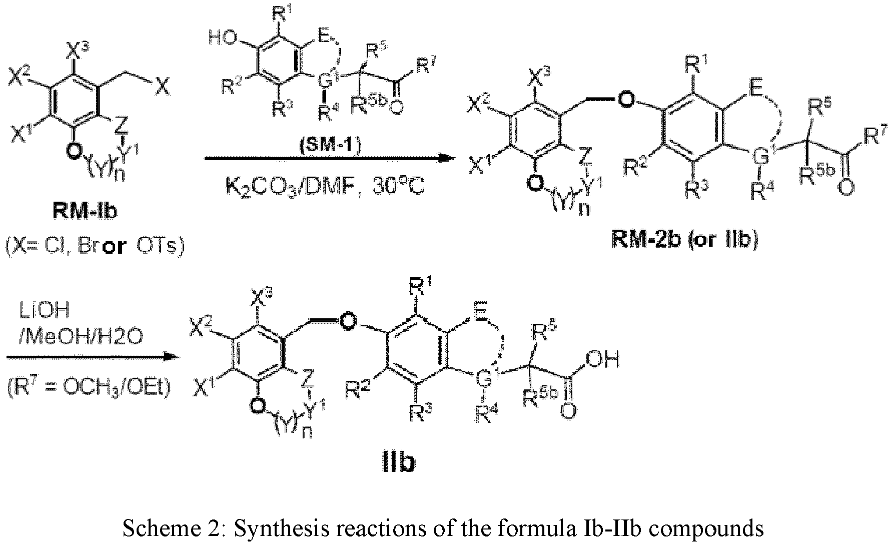

In the above synthesis reaction route 2 was operated as follows:
1. Synthesis of RM-2b: RM-Ib (1.0eq), SM-1b (1.0eq), and basic reagents (e.g. potassium phosphate or potassium carbonate, 2-3eq) were added to DMF (5x) in a round bottom reaction flask, nitrogen was replaced and the reaction was protected. The reaction solution was slowly poured into 40X ice water with stirring, and RM-Ib (or RM-IIb) was obtained after post-processing and purification as usual.
2. Synthesis of target product lib: RM-Ib (or RM-IIb) was hydrolyzed in a mixture of MeOH-H2O with LiOH (1:1, 10X) in a round-bottom reaction flask, respectively, and detected by TLC and/or HPLC until the end of the reaction, and then the target products Ib-IIb were obtained after post-treatment and purification and other routine operations.

The above synthesized compounds of formula Ib-IIb, where n, n, L¹, R¹, R², R³, R⁵, R^{5b}, R⁷, Ra, Rb, Rc, Rd, Re, X⁵, X⁶, Y, Y¹ and Z are defined the same as n, L¹, R¹, R², R³, R⁵, R^{5b}, R⁷, Ra, Rb, Rc, Rd, Re, X⁵, X⁶, Y, Y¹, and Z of claims 1-4 of the present invention, respectively. Examples of synthetic reactions for the compounds in Formulas Ib-IIb of the present invention are shown in Table 3 below, respectively, for the preparation of each specific compound of different structures, which were successfully synthesized using the benzo-oxanthene raw material RM-1b in Table 1 and the raw material SM-1 in Table 2 below, respectively:

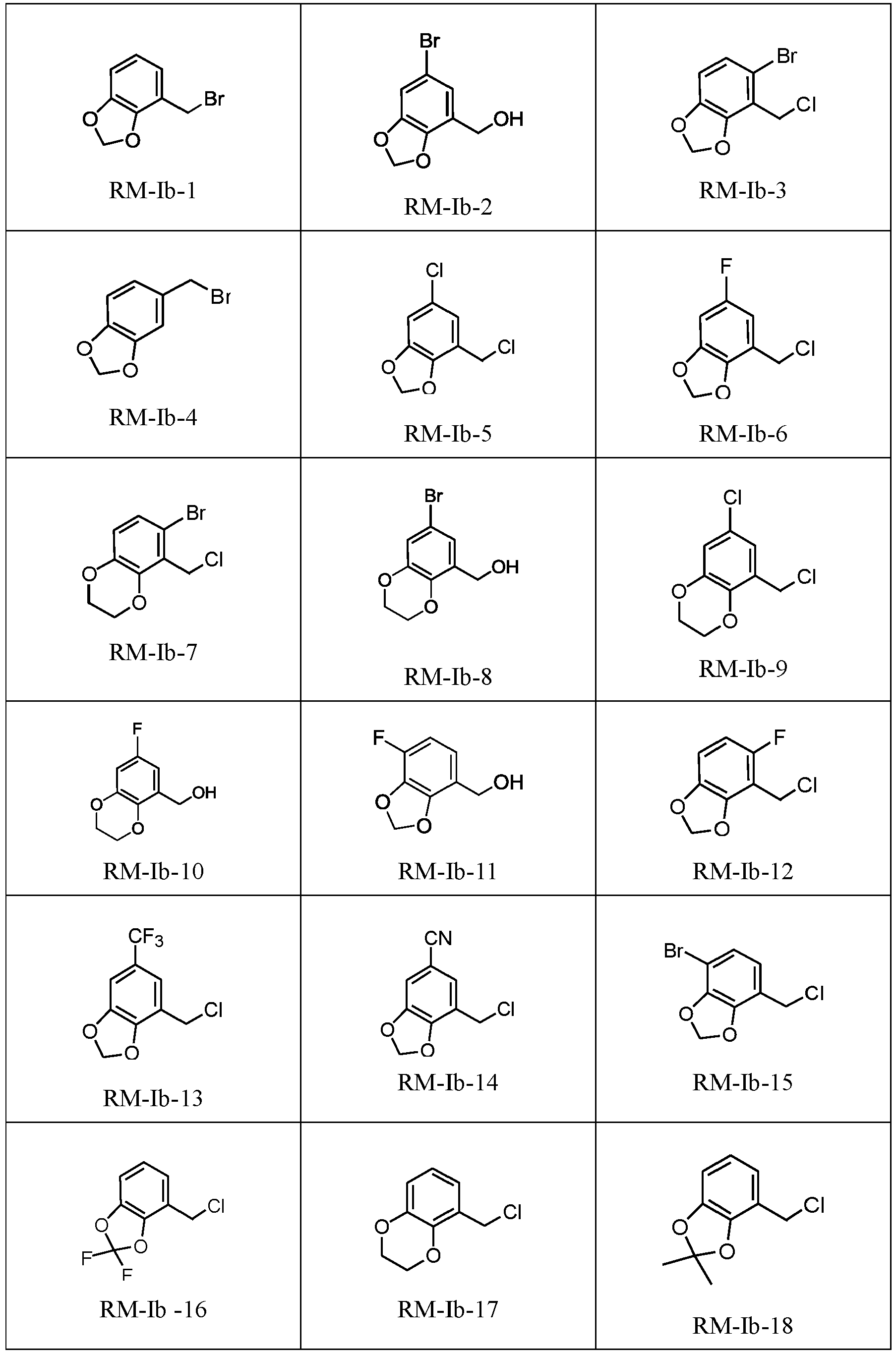

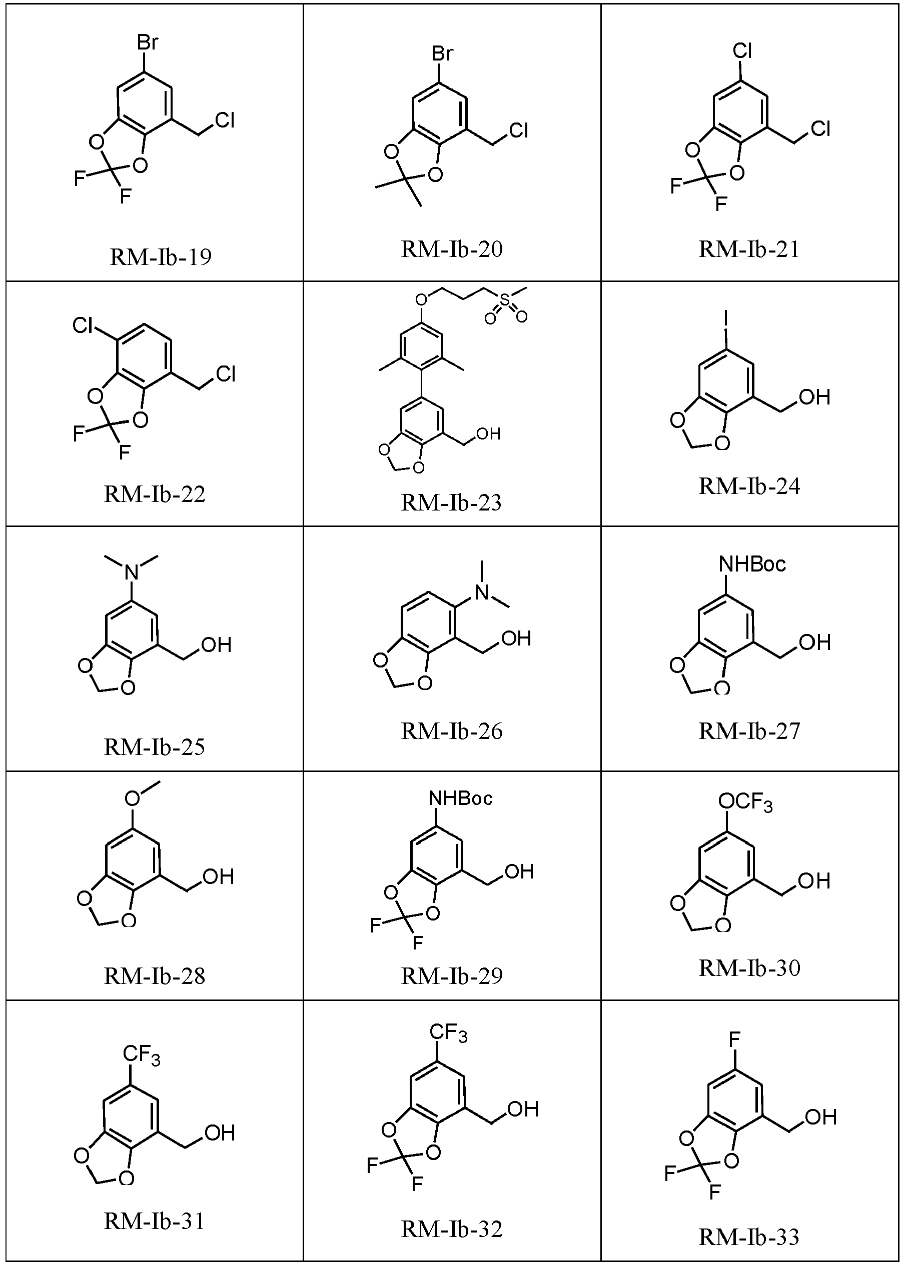

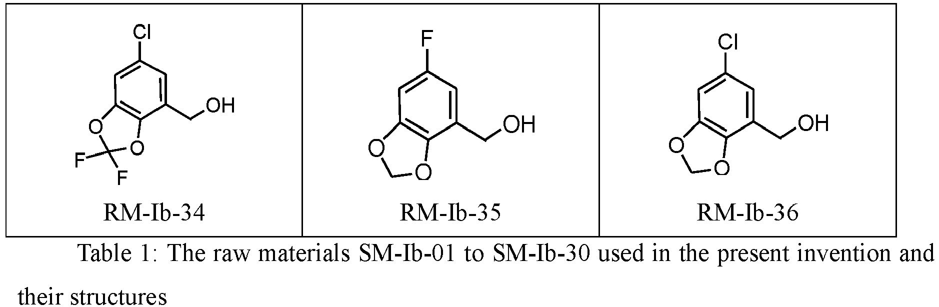

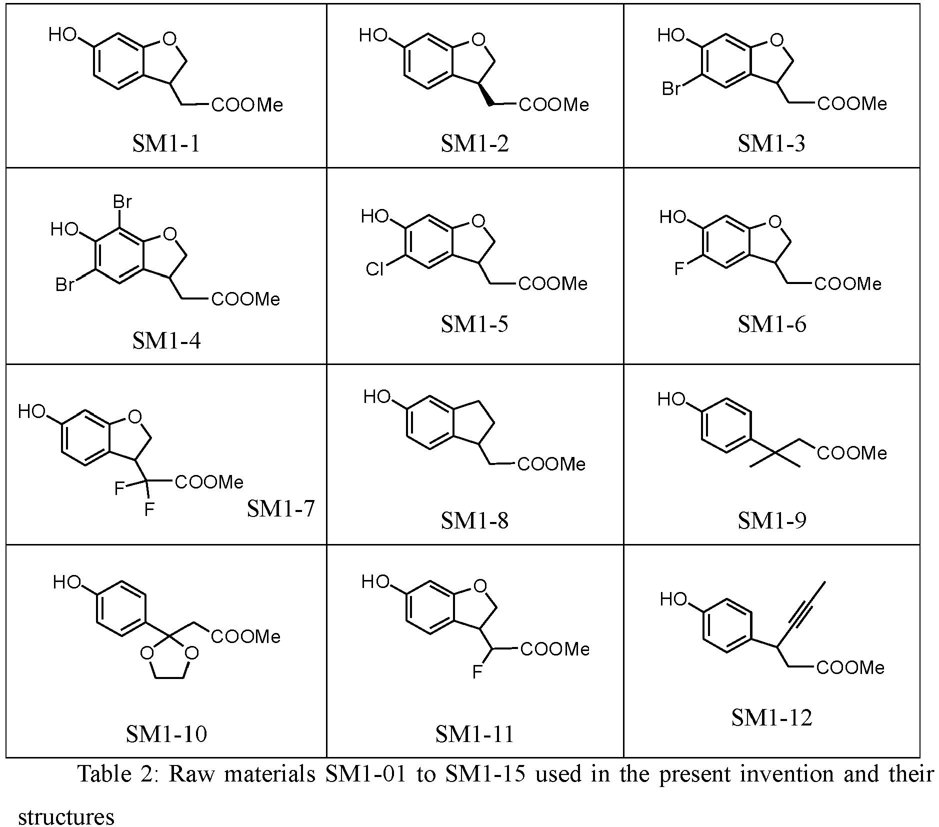

The above-mentioned raw materials RM-1b and SM1 were used, and then intermediate RM-2b was prepared by the synthetic reaction route in Scheme 3 below, and finally each specific compound of formula Ib-IIb was synthesized by hydrolysis reaction, respectively. Examples of specific reactions are as follows, respectively:

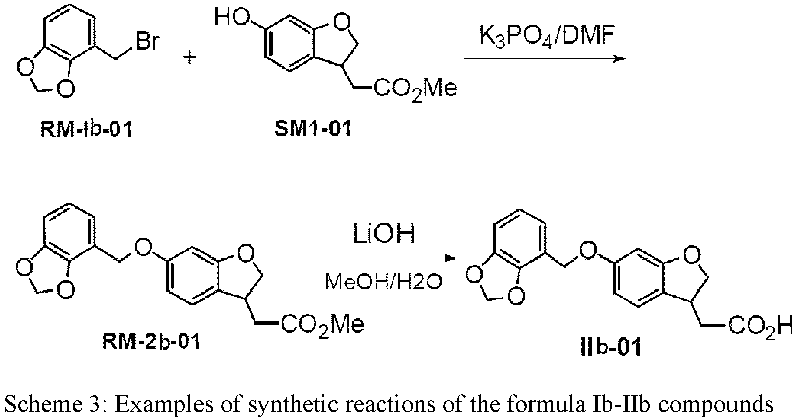

In the above example of synthesis reaction of Scheme 3:
Step 1 reaction: The key intermediate compound (RM-Ib-01) is first obtained by first passing raw material RM-Ib-01 with another raw material SM1-01 in the presence of an inorganic base (e.g., K3PO4) in a solvent (e.g., DMSO or DMF);

The second reaction step: the intermediate (RM-2b-01) is then hydrolyzed in the presence of an inorganic base (e.g., LiOH) in a solvent (e.g., MeOH or a mixture of methanol and water) to obtain the target product Ib-01.

The intermediate structures of the intermediate compound RM-2b obtained by the first step reactions of the synthetic reaction routes in options 1, 2 and 3 above, respectively, are shown in the following structural formula series of RM-2b; and the target products of formula IIb obtained by the second step hydrolysis reactions, respectively, are shown in the following structural formula series of formula IIb:
RM-2b structural series:

| | | | |
|---|---|---|---|
| 1 | 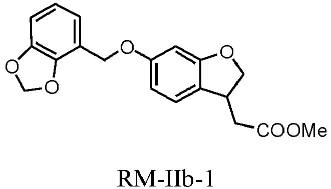 | 2 | 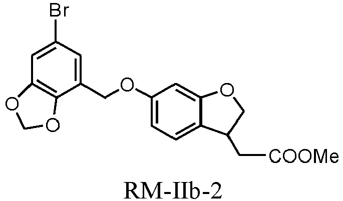 |
| 3 | 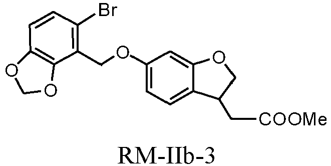 | 4 | 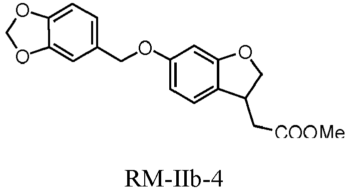 |
| 5 | 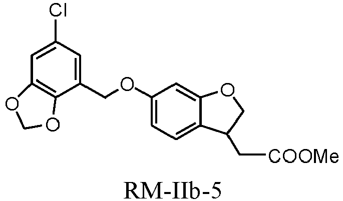 | 6 | 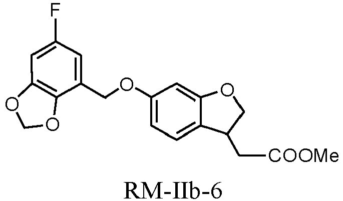 |
| 7 | 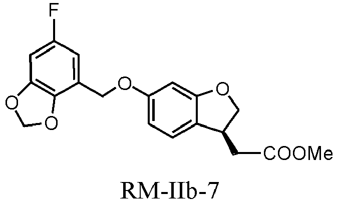 | 8 | 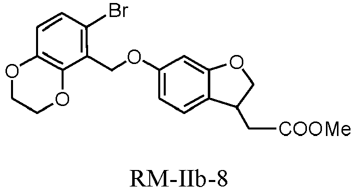 |
| 9 | 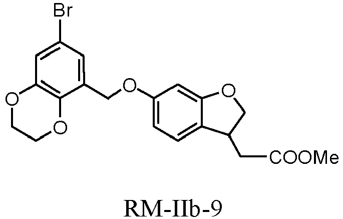 | 10 | 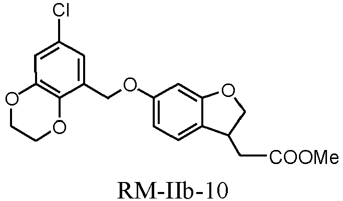 |
| 11 | 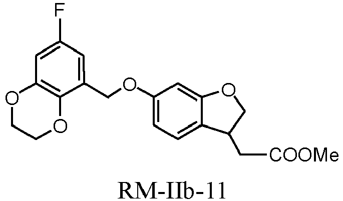 | 12 | 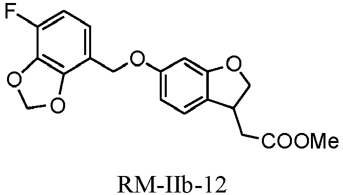 |
| 13 | 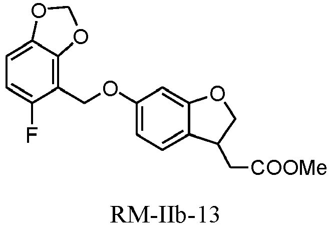 | 14 | 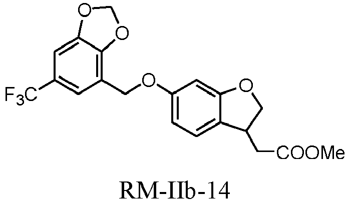 |
| 15 | 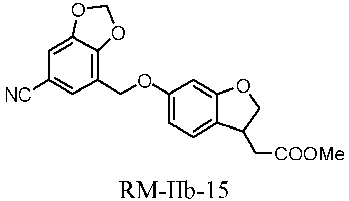 | 16 | 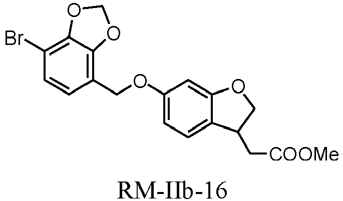 |
| 17 | 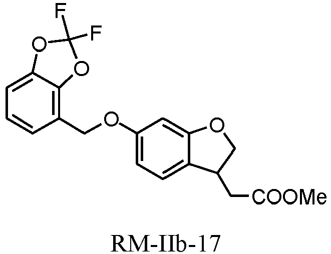 | 18 | 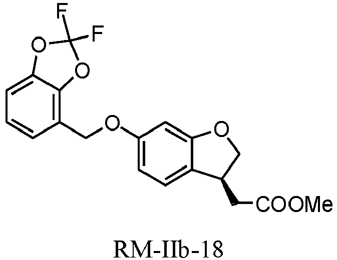 |
| 19 | 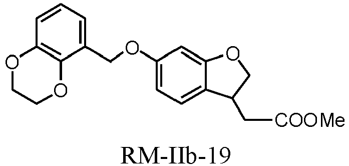 | 20 | 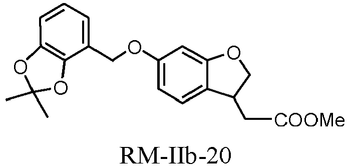 |
| 21 | 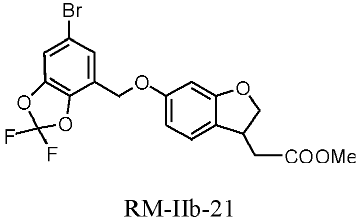 | 22 | 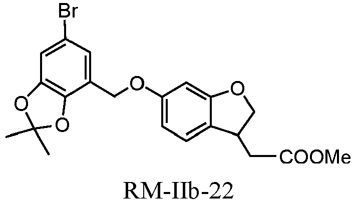 |
| 23 | 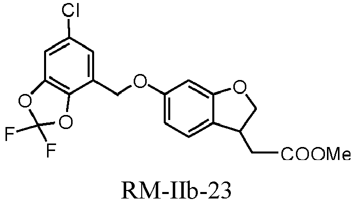 | 24 | 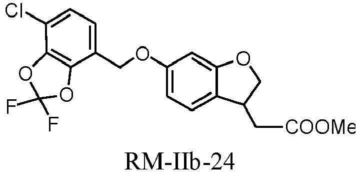 |
| 25 | 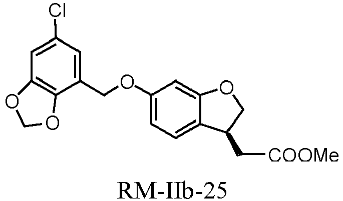 | 26 | 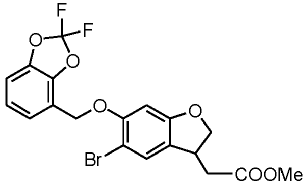 |
| | | | 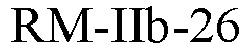 |
| 27 | 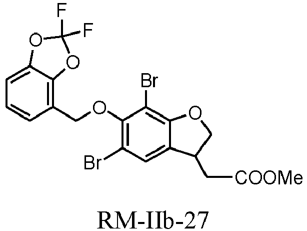 | 28 | 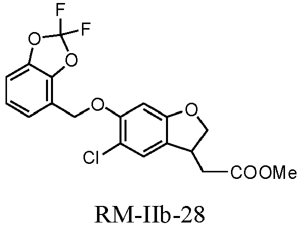 |
| 29 | 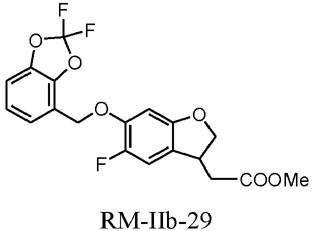 | 30 | 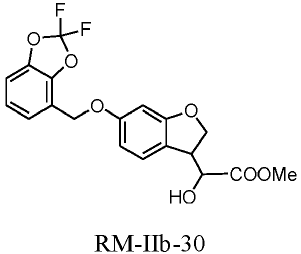 |
| 31 | 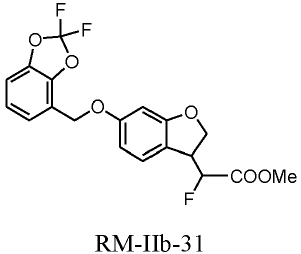 | 32 | 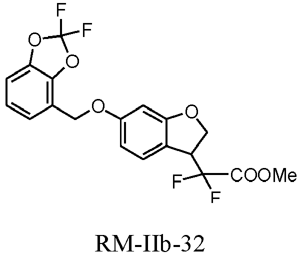 |
| 33 | 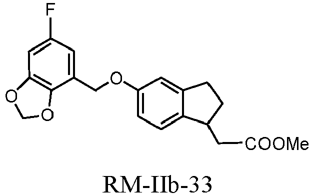 | 34 | 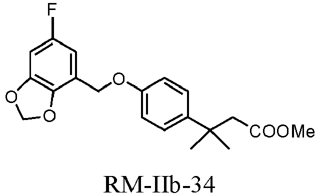 |
| 35 | 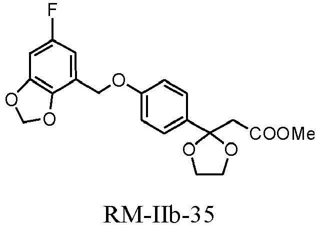 | 36 | 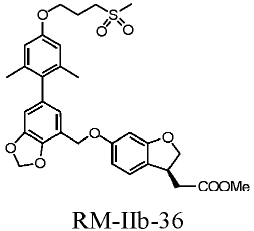 |
| 37 | 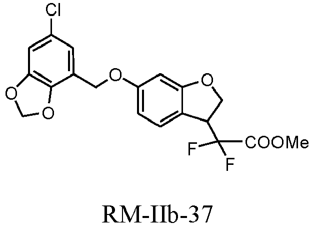 | 38 | 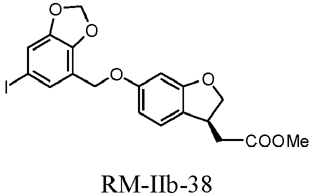 |
| 39 | 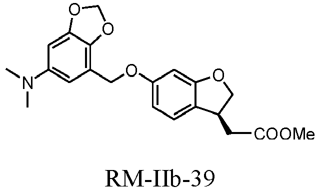 | 40 | 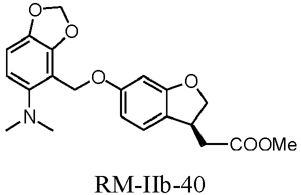 |
| 41 | 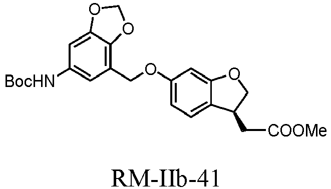 | 42 | 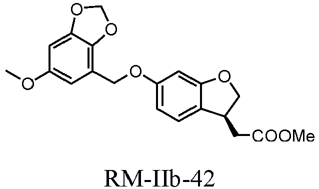 |
| 43 | 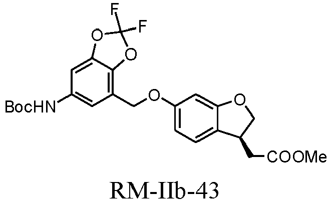 | 44 | 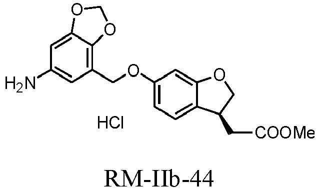 |
| 45 | 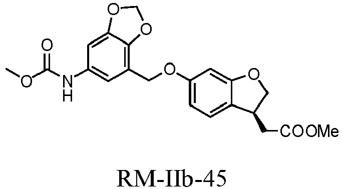 | 46 | 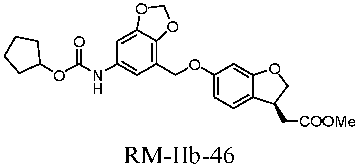 |
| 47 | 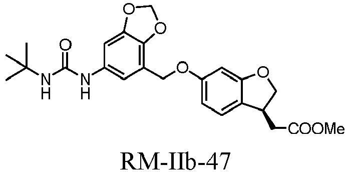 | 48 | 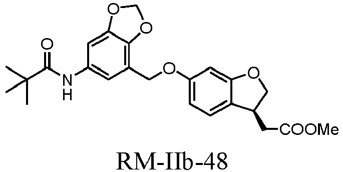 |
| 49 | 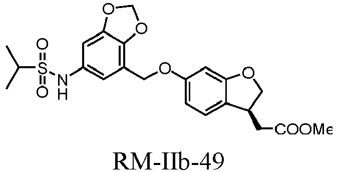 | 50 | 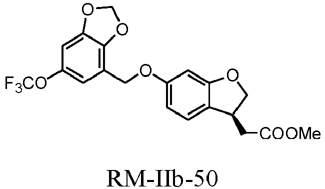 |
| 51 | 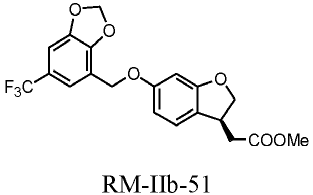 | 52 | 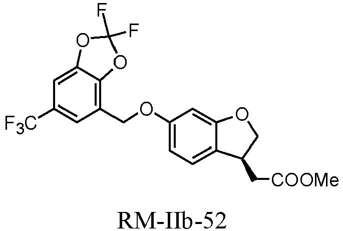 |
| 53 | 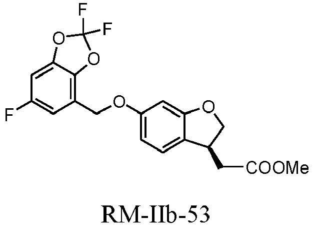 | 54 | 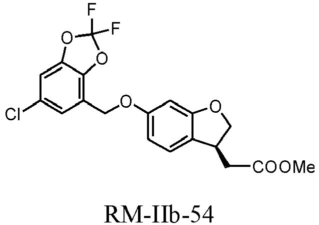 |
| 55 | 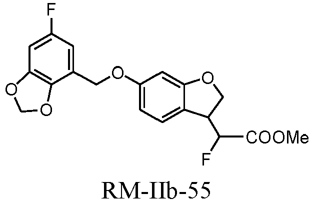 | 56 | 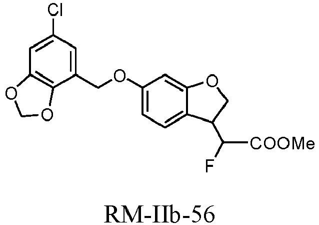 |
| 57 | 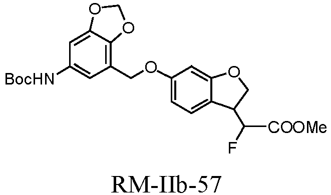 | 58 | 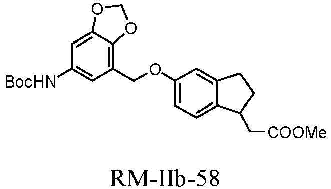 |
| 59 | 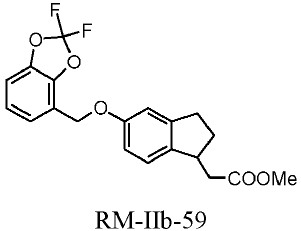 | 60 | 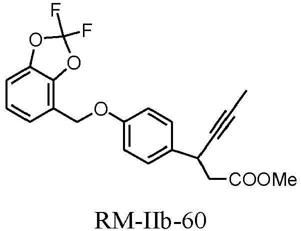 |
| 61 | 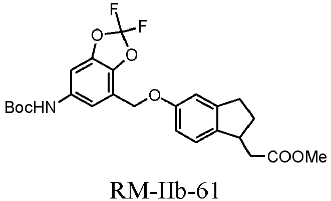 | 62 | 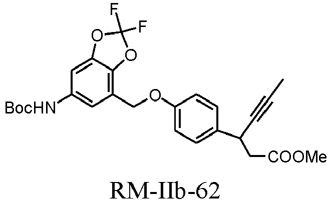 |

Formula IIb structural series

| | | | |
|---|---|---|---|
| 1 | 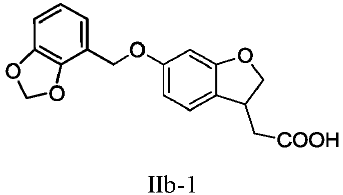 | 2 | 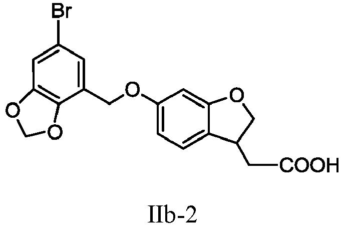 |
| 3 | 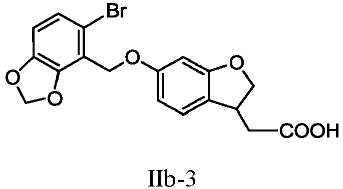 | 4 | 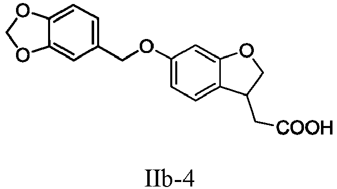 |
| 5 | 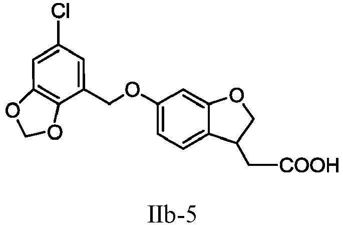 | 6 | 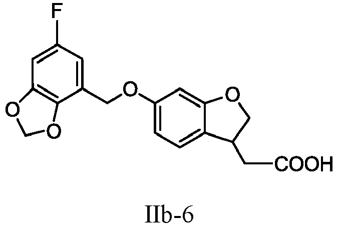 |
| 7 | 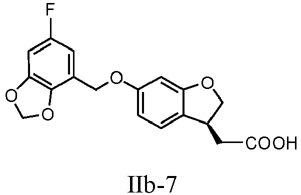 | 8 | 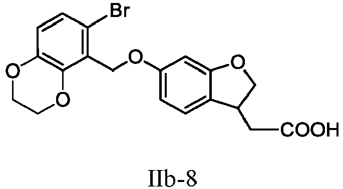 |
| 9 | 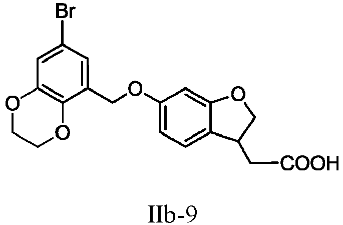 | 10 | 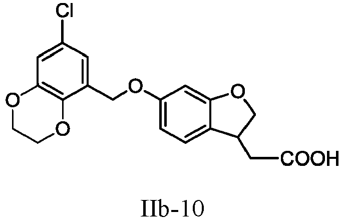 |
| 11 | 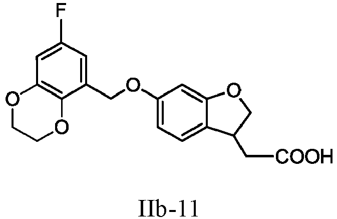 | 12 | 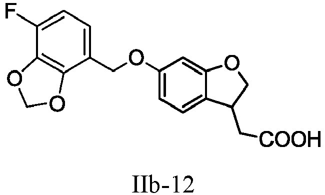 |
| 13 | 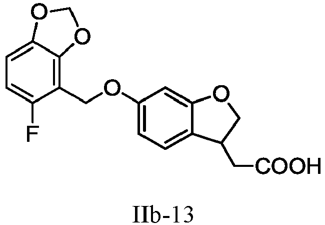 | 14 | 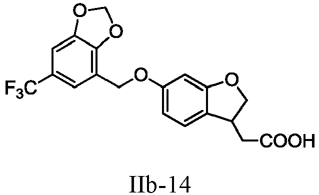 |
| 15 | 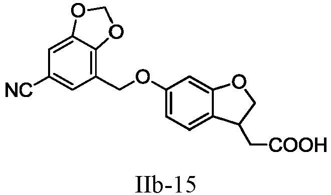 | 16 | 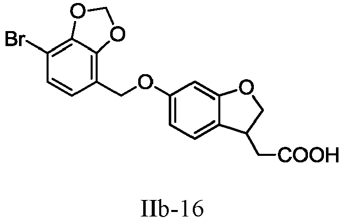 |
| 17 | 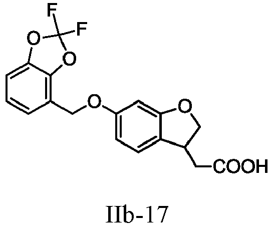 | 18 | 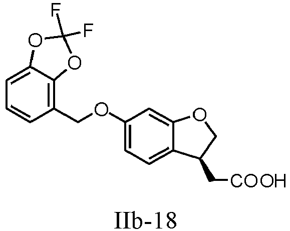 |
| 19 | 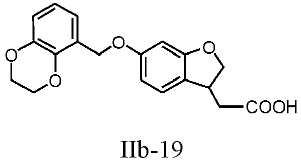 | 20 | 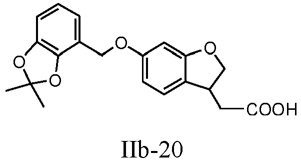 |
| 21 | 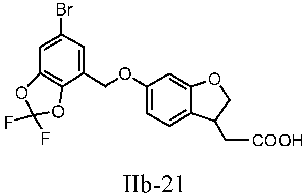 | 22 | 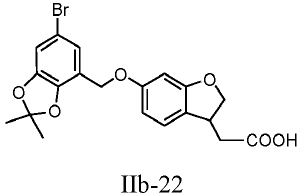 |
| 23 | 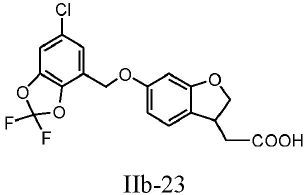 | 24 | 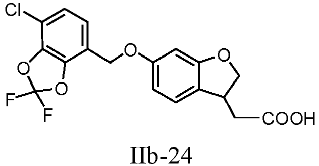 |
| 25 | 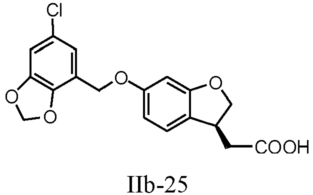 | 26 | 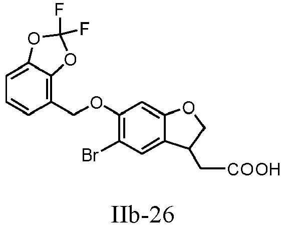 |
| 27 | 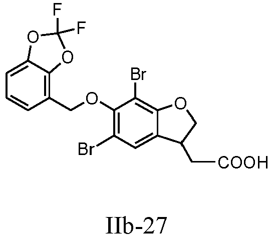 | 28 | 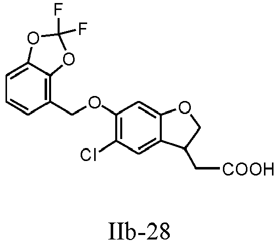 |
| 29 | 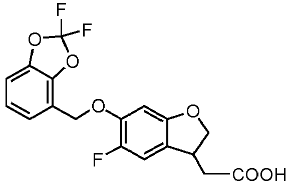 | 30 | 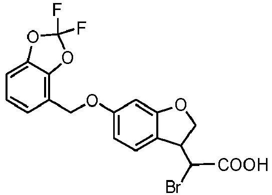 |
| | 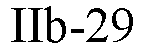 | | 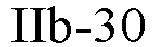 |
| 31 | 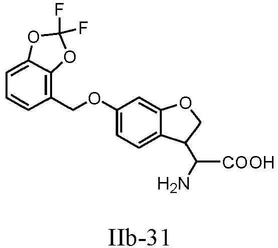 | 32 | 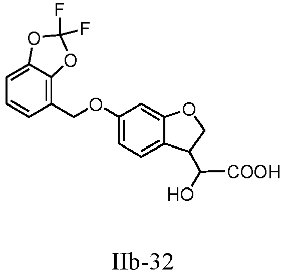 |
| 33 | 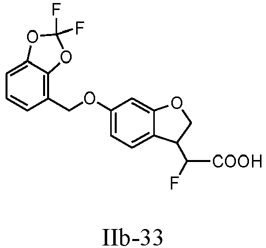 | 34 | 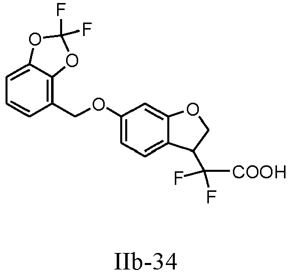 |
| 35 | 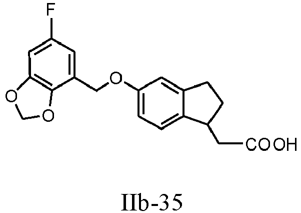 | 36 | 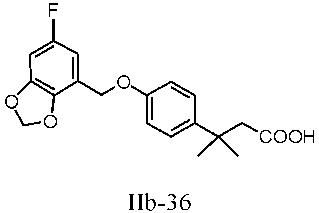 |
| 37 | 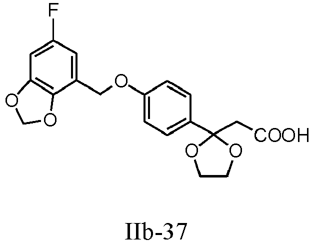 | 38 | 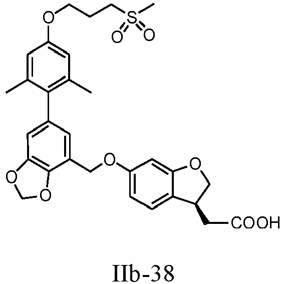 |
| 39 | 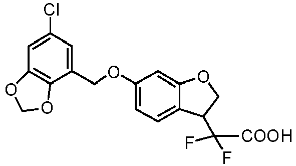 | 40 | 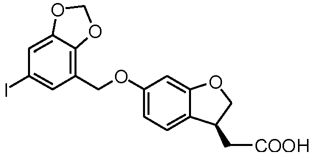 |
| | 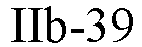 | | 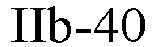 |
| 41 | 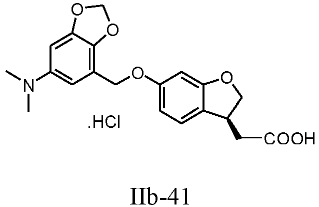 | 42 | 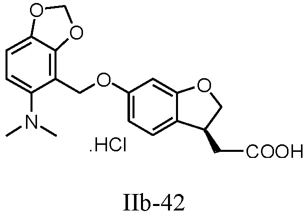 |
| 43 | 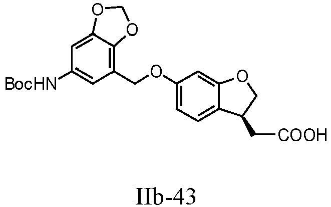 | 44 | 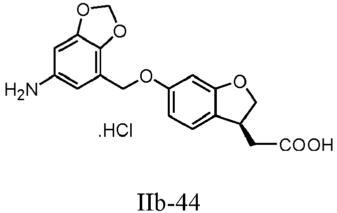 |
| 45 | 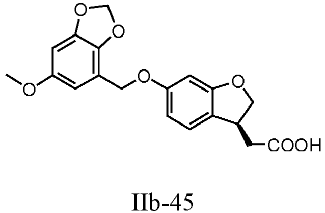 | 46 | 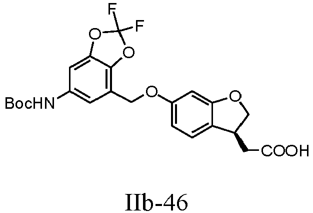 |
| 47 | 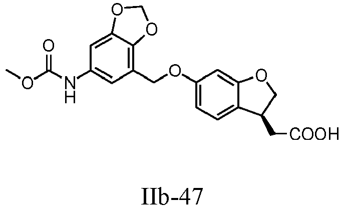 | 48 | 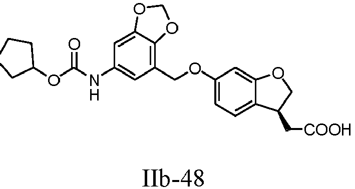 < |
| 49 | 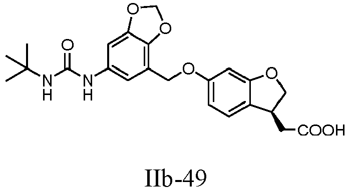 | 50 | 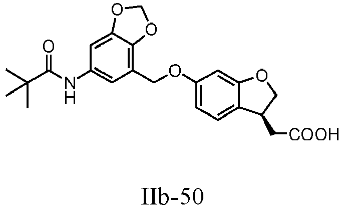 |
| 51 | 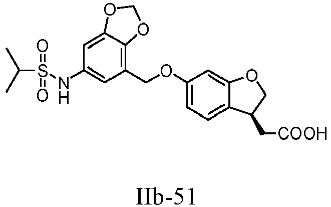 | 52 | 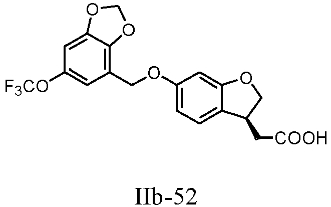 |
| 53 | 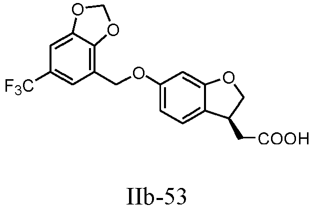 | 54 | 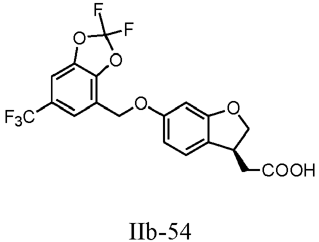 |
| 55 | 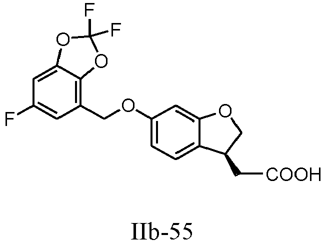 | 56 | 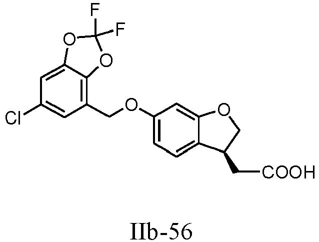 |
| 57 | 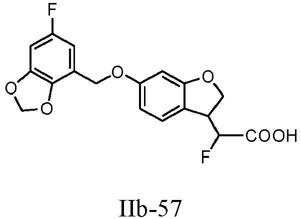 | 58 | 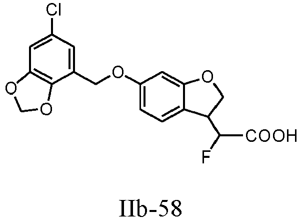 |
| 59 | 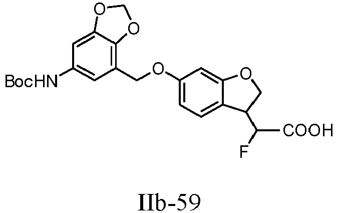 | 60 | 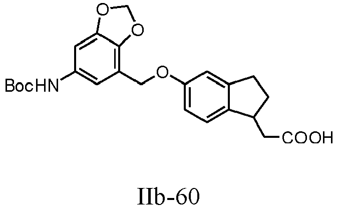 |
| 61 | 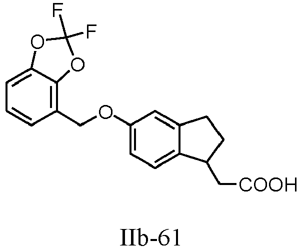 | 62 | 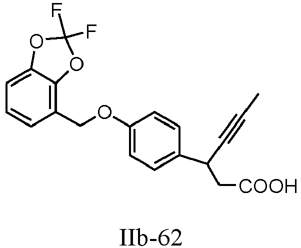 |
| 63 | 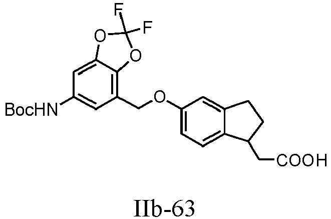 | 64 | 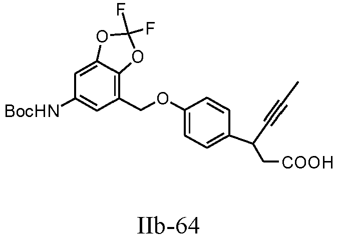 |

The experimental conditions and product analysis results for each specific reaction step are listed separately in the Examples.

Specific results concerning the synthesis and analysis of each of the novel structural compounds of formula **Ib-IIb** described above are detailed in the final embodiment of the invention, with the chemistry of each compound and its chiral asymmetric purity determined by HPLC on a chiral column and the corresponding chemical structure characterization determined by LC-MS and/or hydrogen spectrum nuclear magnetic resonance (1H-NMR) analysis, respectively.

The synthesis and effects of the various types of intermediates and compounds of the present invention are illustrated below by means of embodiments.

**The apparatus and raw materials involved in the embodiment are described as follows:**
The infrared spectral data were analyzed using a Thermo Nicolet Fourier Transform AVATAR^{™} 360 E.S.P^{™} infrared instrument and are expressed in cm⁻¹ units.

The NMR hydrogen spectra were obtained from the analysis of a Varian Mercury Plus 400 (400 MHz) NMR instrument. The chemical shifts were recorded using tetramethylsilane as an internal standard and expressed in ppm (CHCl3: δ= 7.26 ppm). The following data information was recorded: chemical shifts and their cleavage and coupling constants (s: single peak; d : double peak; t: triple peak; q: quadruple peak; br: broad peak; m: multiple peak).

The mass spectrometry data were analyzed using a liquid mass spectrometer from Finnigan Advanced LCQ (Finnigan LCQ Advantage), among other needs. The molecular weights of the organic carboxylic acid (-COOH) compounds of formulas Ib-IIb in this invention are mainly in the anionic mode ESI-MS [(M-H)+], but the ester intermediate compounds of formulas RM-IIb and some compounds containing amine groups in **Ib-IIb** have molecular weights in the cationic mode ESI-MS [(M+H)+].

The special raw materials and intermediates involved in this invention were provided by Zannan Technology Co., Ltd. and other custom processing, and all other chemical reagents were purchased from reagent suppliers such as Shanghai Reagent Company, Aldrich Company (Aldrich), Acros Company (Acros), etc. If the intermediates or products required for the reaction during the synthesis were not enough for the next step and other tests, the synthesis was repeated several times until sufficient quantities were available.The GPR40 activity tests of the compounds prepared by the invention as well as pharmacological and toxicological tests were done by CRO service units in Shanghai and Beijing.

The abbreviations of the relevant chemical raw materials, reagents and solvents involved in the present invention and its embodiments are annotated as follows:
AIBN: Azo diisobutyronitrile
Boc: tert-Butoxycarbonyl
(Boc)₂O: Di-tert-butyl dicarbonate
CDI: N,N'-carbonyl diimidazole
DBU: 1,8-Diazabicyclo[5.4.0]undec-7-ene
Et: Ethyl
EDCI: N-ethyl-N'-(3-dimethylaminopropyl)carbodiimide hydrochloride
HATU: 2-(7-azobenzotriazole)-N,N,N',N'-tetramethyluronium hexafluorophosphate
NBS: N-bromosuccinimide
SOCl₂: Sulfoxide chloride
Pd/C: Palladium carbon
DMAP: 4-Dimethylaminopyridine
HMTA: Hexamethylenetetramine
DIEA: N,N-diisopropylethylamine
Py: Pyridine
HBr: Hydrobromic acid
HCl: Hydrochloric acid
HOAc: Glacial acetic acid
TFA: Trifluoroacetic acid
TsOH: p-Toluenesulfonic acid
NaOH: Sodium hydroxide
LiOH: Lithium hydroxide
ACN: Acetonitrile
DCM: Dichloromethane
DCE: Dichloroethane
DMF: N,N-dimethylformamide
DMSO: Dimethyl sulfoxide
Et₂O: Diethyl ether
EA: Ethyl acetate
PE: Petroleum ether
THF: Tetrahydrofuran
TBME: Methyl tert-butyl ether

### Example 1

### Synthesis of compound IIb-1

The synthesis reaction is shown in the following general formula **I:**

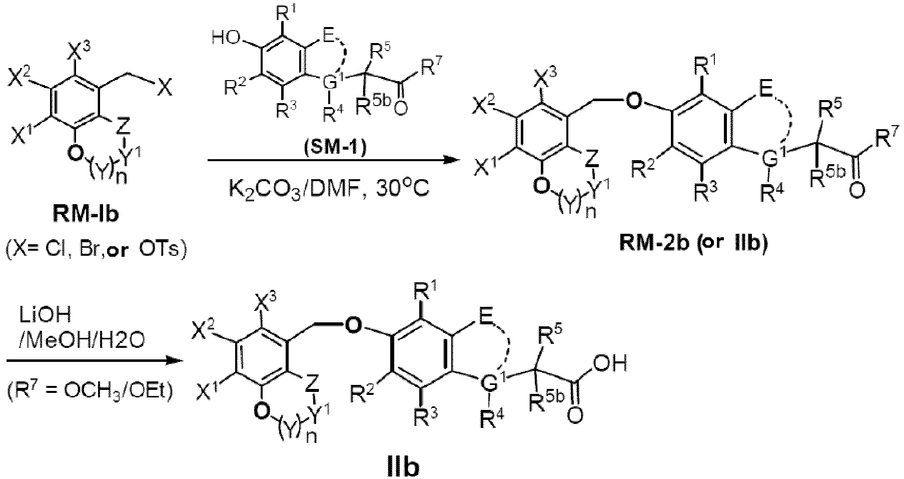

### Step 1:

The raw materials **RM-Ib-1** (0.051g, 0.24mmol) and SM1-1 (0.050g, 0.24mmol, 1.0eq.) were dissolved in 2mL of DMF, and potassium carbonate (0.050g, 0.36mmol, 1.5eq.) was added with stirring, and the reaction was completed by heating to 90°C under nitrogen protection overnight.HPLC showed that the reaction solution was cooled to room temperature after the reaction was finished, water was added, ethyl acetate was added for extraction, and then the organic phases were combined, washed with saline and dried, and the intermediate RM-IIb-1 (0.1g) was concentrated.

ESI-MS [(M+H)⁺]: m/z of RM-IIb-1 calculated: 343.1, founded: 343.0.

### Step 2:

AddedMeOH (2 mL), THF (1 mL) and aqueous LiOH (1N, 1 mL) were added sequentially to intermediate RM-IIb-1, stirred at room temperature for 1 hr. After the reaction was completed, the reaction solution was neutralized with hydrochloric acid (1N) to pH about 4,addedwater, added ethyl acetate for extraction, and then the organic phases were combined, washed with saline and dried, and finally purified by column chromatography, obtained the light yellow solid product IIb-1 (0.040 g) in two-step yield: 50.7%.

Upon examination,¹H NMR (400 MHz, CDCl₃) of product IIb-1: δ 7.07-7.05 (m, 1H), 6.95-6.93 (m, 1H), 6.86-6.79 (m, 2H), 6.52-6.49 (m, 2H), 5.99 (s, 2H), 5.01 (s, 2H), 4.78-4.74 (m, 1H) , 4.31-4.27 (m, 1H), 3.83-3.80 (m, 1H), 2.83-2.78 (m, 1H), 2.65-2.59 (m, 1H).

ESI-MS [(M-H)⁺]: m/z of IIb-1 calculated: 327.1, founded: 327.0.

### Example 2

### Synthesis of compound IIb-2

The synthesis reaction is shown in the following general formula II:

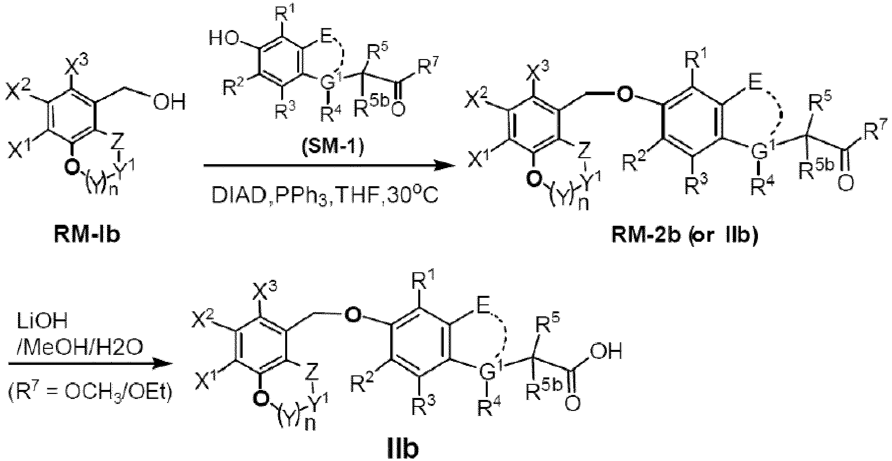

### Step 1:

The raw materials RM-Ib-2 (0.043 g, 0.19 mmol), SM1-1 (0.039 g, 0.19 mmol, 1.0 eq.) and PPh3 (0.146 g, 0.56 mmol, 3 eq.) were dissolved in 4 mL of THF, protected by nitrogen, under cooling in an ice-water bath, and DIAD (0.113 g, 0.56 mmol, 3eq.), stirred for 1h in an ice water bath after addition, and the reaction was completed at 20-30 degrees C overnight.HPLC showed that the reaction solution was cooled to room temperature after the reaction was finished, water was added, DCM was added for extraction, and then the organic phases were combined, washed with saline and dried, and the crude product was concentrated; the crude product was separated by TLC scraper to obtain intermediate **RM-IIb-2**

ESI-MS [(M+H)⁺]: m/z of RM-IIb-2 calculated: 421.0, founded: 421.1.

### Step 2:

MeOH (2 mL), THF (1 mL) and aqueous LiOH (1N, 1 mL) were added to intermediate RM-IIb-2 and stirred at room temperature for 1h. After the reaction was completed as indicated by HPLC, the reaction solution was neutralized with hydrochloric acid (1N) to pH about 4,water was added, ethyl acetate was added for extraction, and then the organic phases were combined, washed with saline and dried, and finally purified by column chromatography to give The pale yellow solid product IIb-2 (0.025 g) was obtained in two-step yield: 33%.

¹H-NMR for the**IIb-2** hydrochloride (400 MHz, CDCl₃) δ: 7.09-7.05 (m, 2H), 6.92 (m, 1H), 6.50-6.46 (m, 2H), 6.01 (s, 2H), 4.95 (s, 2H), 4.79-4.75 (m, 1H), 4.31-4.28 (m, 1H), 3.84 -3.79 (m, 1H), 2.84-2.79 (m, 1H), 2.66-2.59 (m, 1H).

ESI-MS [(M-H)⁺]: m/z of IIb-2 calculated: 405.0, founded: 405.2.

### Example 3

### Synthesis of compound IIb-3

The synthesis of compound IIb-3 was prepared in the same way as in Example 1, and the product IIb-3 was obtained by etherification and hydrolysis reaction, wherein compound RM-Ib-3 (0.48 mmol) was used in the reaction instead of compound RM-Ib-1 to obtain intermediate RM-IIb-3, and the white solid product IIb-3 (0.039 g) was obtained from the intermediate by hydrolysis in two-step yield : 20%.

ESI-MS [(M-H)⁺]: m/z of IIb-3 calculated: 405.0, founded: 404.8.

### Example 4

### Synthesis of compound IIb-4

The synthesis method for the preparation of compound IIb-4 was the same as that of Example 1, and the product IIb-4 was obtained by etherification and hydrolysis reaction, wherein compound RM-Ib-4 (0.24 mmol) was used in the reaction instead of compound RM-Ib-1 to obtain intermediate RM-IIb-4, and the light yellow solid product IIb-4 (0.060 g) was obtained from the intermediate by hydrolysis in two-step yield: 76%.

¹H-NMR for the**IIb-4**hydrochloride (400 MHz, CDCl₃)δ: 7.07-7.05 (m, 1H), 6.91-6.86 (m, 2H), 6.81-6.79 (m, 1H), 6.49-6.45 (m, 2H), 5.96 (s, 2H), 4.91 (s, 2H), 4.78-4.74 (m, 1H) , 4.31-4.27 (m, 1H), 3.83-3.79 (m, 1H), 2.84-2.78 (m, 1H), 2.65-2.59 (m, 1H).

ESI-MS [(M-H)⁺]: m/z of IIb-4 calculated: 327.1, founded: 327.0.

### Example 5

### Synthesis of compound IIb-5

The synthesis of compound IIb-5 was prepared in the same way as in Example 1, and the product IIb-5 was obtained by etherification and hydrolysis reaction, wherein compound RM-Ib-5 (0.54 mmol) was used in the reaction instead of compound RM-Ib-1 to obtain intermediate RM-IIb-5, and the white solid product IIb-5 (0.068 g) was obtained from the intermediate by hydrolysis, in two-step yield : 34.8%.

¹H-NMR for the **IIb-5**(400 MHz, CDCl3) δ: 7.08-7.06 (m, 1H), 6.95 (s, 1H), 6.78 (s, 1H), 6.50-6.46 (m, 2H), 6.02 (s, 2H), 4.95 (s, 2H), 4.79-4.75 (m, 1H), 4.32-4.28 (m, 1H), 3.83-3.80 (m, 1H), 2.84-2.78 (m, 1H), 2.66-2.59 (m, 1H).

ESI-MS [(M-H)⁺]: m/z of IIb-5 calculated:361.1,founded:360.9.

### Example 6

### Synthesis of compound IIb-6

The synthesis of compound **IIb-6** was prepared in the same way as in Example 1, and the product **IIb-6** was obtained by etherification and hydrolysis reaction, wherein compound **RM-Ib-6** (0.41 mmol) was used in the reaction instead of compound **RM-Ib-1** to obtain intermediate **RM-IIb-6,** and the white solid product **IIb-6** (0.090 g) was obtained from the intermediate by hydrolysis in two-step yield: 63.6%.

¹H-NMR for theIIb-6 hydrochloride (400 MHz, DMSO) δ: 7.10-7.09 (m, 1H), 6.92-6.89 (m, 1H), 6.74-6.71 (m, 1H), 6.46-6.44 (m, 2H), 6.08 (s, 2H), 4.93 (s, 2H), 4.69-4.64 (m, 1H), 4.19-4.15 (m, 1H), 3.69-3.64 (m, 1H), 2.71-2.65 (m, 1H),2.48-2.43 (m, 1H). ¹⁹F NMR (376 MHz, DMSO) δ -120.47.

ESI-MS [(M-H)⁺]: m/z of IIb-6 calculated: 345.1, founded: 345.0.

### Example 7

### Synthesis of compound IIb-7

The synthesis method for preparing compound IIb-7 was the same as that of Example 1, and the product IIb-7 was obtained by etherification and hydrolysis reaction, in which compound RM-Ib-6 (0.35 mmol) was used in the reaction instead of compound RM-Ib-1 and compound SM1-2 (0.35 mmol) was used instead of compound SM1-1 to obtain intermediate RM-IIb-7, and the intermediate was hydrolyzed to give the white solid product IIb-7 (0.085 g) in two-step yield: 70.8%. The white solid product IIb-7 (0.085 g) was obtained in two-step yield: 70.8%.

¹H-NMR for the **IIb-7**hydrochloride (400 MHz, CDCl₃) δ: 7.07-7.05 (m, 1H), 6.66-6.63 (m, 1H), 6.56-6.54 (m, 1H), 6.49-6.46 (m, 2H), 6.01 (s, 2H), 4.96 (s, 2H), 4.79-4.74 (m, 1H) , 4.31-4.27 (m, 1H), 3.84-3.77 (m, 1H), 2.84-2.79 (m, 1H), 2.66-2.59 (m, 1H); ¹⁹F NMR (376 MHz, CDCl₃): δ -119.95.

The ESI-MS [(M-H)⁺]: m/z of IIb-7 calculated: 345.1, founded: 345.2.

### Example 8

### Synthesis of Compound IIb-8

The synthesis of compound IIb-8 was prepared in the same way as in Example 1, and the product IIb-8 was obtained by etherification and hydrolysis reaction, wherein compound RM-Ib-7 (0.24 mmol) was used in the reaction instead of compound RM-Ib-1 to obtain intermediate RM-IIb-8, and the white solid product IIb-8 (0.059 g) was obtained from the intermediate by hydrolysis, in two-step yield : 65.5%.

¹H-NMR for the**IIb-8** hydrochloride (400 MHz, CDCl3)δ :7.09-7.07 (m, 2H), 6.79-6.76 (m, 1H), 6.57-6.54 (m, 2H), 5.35 (s, 2H), 4.79-4.75 (m, 1H), 4.32-4.25 (m, 5H), 3.84-3.80 (m, 1H), 2.84-2.79 (m, 1H), 2.65-2.59 (m, 1H).

ESI-MS [(M-H)⁺]: m/z of IIb-8 calculated: 419.0, founded: 418.9.

### Example 9

### Synthesis of Compound IIb-9

The synthesis of compound **IIb-9** was prepared in the same way as in Example 2, and the product **IIb-9** was obtained by etherification and hydrolysis reaction, wherein compound **RM-Ib-8** (0.24 mmol) was used in the reaction instead of compound **RM-Ib-2** to obtain intermediate **RM-IIb-9,** and the white solid product IIb-9 (0.035 g) was obtained from the intermediate by hydrolysis in two-step yield : 45%.

ESI-MS [(M-H)⁺]: m/z of IIb-9 calculated: 419.0, founded: 418.8.

### Example 10

### Synthesis of compound IIb-10

The synthesis of compound **IIb-10** was carried out in the same way as in Example 1, and the product **IIb-10** was obtained by etherification and hydrolysis reaction, in which compound **RM-Ib-9** (0.65 mmol) was used in the reaction instead of compound **RM-Ib-1** to obtain intermediate **RM-IIb-10,** and the white solid product IIb-10 (0.196 g) was obtained from the intermediate by hydrolysis in two steps. Yield: 53.2%.

¹H-NMR for the **IIIb-10**hydrochloride (400 MHz, CDCl3) δ: 7.34 (s, 1H), 7.07-7.05 (m, 1H), 7.00 (m, 1H), 6.48 (m, 1H), 6.51-6.46 (m, 2H), 4.98 (s, 2H), 4.79-4.74 (m, 1H), 4.31- 4.27 (m, 5H), 3.85-3.78 (m, 1H), 2.84-2.79 (m, 1H), 2.66-2.60 (m, 1H).

ESI-MS [(M-H)⁺]: m/z of IIb-10 calculated: 375.1, founded: 374.9.

### Example 11

### Synthesis of compound IIb-11

The synthesis method for the preparation of compound IIb-11 was the same as that of Example 2, and the product **IIb-11** was obtained by etherification and hydrolysis reaction, wherein compound **RM-Ib-10** (0.24 mmol) was used in the reaction instead of compound **RM-Ib-2** to obtain intermediate RM-IIb-11, and the white solid product **IIb-11** (0.033 g) was obtained from the intermediate by hydrolysis in two steps Yield:37%.

ESI-MS [(M-H)⁺]: m/z ofIIb-11calculated: 359.1, founded: 359.0.

### Example 12

### Synthesis of compound IIb-12

The synthesis of compound **IIb-12** was carried out in the same way as in Example 2, and the product **IIb-12** was obtained by etherification and hydrolysis, in which compound **RM-Ib-11** (0.63 mmol) was used in the reaction instead of **RM-Ib-2** to obtain intermediate **RM-IIb-12,** and the light yellow solid product **IIb-12** (0.028 g) was obtained from the intermediate by hydrolysis. Two-step yield: 12.8%.

ESI-MS [(M-H)⁺]: m/z of IIb-12calculated: 345.1, founded: 344.9.

### Example 13

### Synthesis of Compound IIb-13

The synthesis of compound **IIb-13** was carried out in the same way as in Example 1, and the product **IIb-13** was obtained by etherification and hydrolysis, in which compound **RM-Ib-12** (0.43 mmol) was used in the reaction instead of RM-Ib-1 to obtain intermediate **RM-IIb-13,** and the light yellow solid product **IIb-13** (0.060 g) was obtained from the intermediate by hydrolysis. Two-step yield: 40%.

¹H-NMR for the **IIb-13**hydrochloride (400 MHz, CDCl3)δ: 6.92-6.90 (m, 1H), 6.65-6.62 (m, 1H), 6.49-6.39 (m, 3H), 5.90 (s, 2H), 4.90 (s, 2H), 4.62-4.58 (m, 1H), 4.20-4.16 (m, 1H), 3.64-3.61 (m, 1H), 2.87-2.57 (m, 1H), 2.41-2.19 (m, 1H).

ESI-MS [(M-H)⁺]: m/z of IIb-13 calculated:345.0, founded: 345.0.

### Example 14

### Synthesis of Compound IIb-14

The synthesis method for the preparation of compound **IIb-14** was the same as that of Example 1, and the product **IIb-14** was obtained by etherification and hydrolysis reaction, in which compound **RM-Ib-13** (0.22 mmol) was used in the reaction instead of compound **RM-Ib-1** to obtain intermediate **RM-IIb-14,** and the white solid product **IIb-14** (0.045 g) was obtained from the intermediate by hydrolysis in two steps. Yield: 51.3%.

¹H-NMR for the **IIb-14**hydrochloride (400 MHz, CDCl3)δ: 7.28 (s, 1H), 7.08-7.06 (m, 1H), 7.00 (s, 1H), 6.51-6.47 (m, 2H), 6.09 (s, 2H), 5.00 (s, 2H), 4.79-4.75 (m, 1H), 4.32- 4.28 (m, 1H), 3.84-3.79 (m, 1H), 2.84-2.78 (m, 1H), 2.66-2.59 (m, 1H).

ESI-MS [(M-H)⁺]: m/z of IIb-14 calculated: 395.1, founded: 395.0.

### Example 15

### Synthesis of compound IIb-15

The synthesis of compound **IIb-15** was carried out in the same way as in Example 1, and the product **IIb-15** was obtained by etherification and hydrolysis reaction, in which compound **RM-Ib-14** (0.42 mmol) was used in the reaction instead of compound **RM-Ib-1** to obtain intermediate RM-IIb-15, and the white solid product **IIb-15** (0.072 g) was obtained from the intermediate by hydrolysis in two steps. Yield: 54%.

¹H-NMR for the **IIb-15**hydrochloride (400 MHz, CDCl3) δ:7.34 (s, 1H), 7.08-7.06 (m, 1H), 7.00 (s, 1H), 6.48-6.43 (m, 2H), 6.11 (s, 2H), 4.98 (s, 2H), 4.79-4.75 (m, 1H), 4.32-4.28 (m, 1H), 3.83-3.80 (m, 1H), 2.83-2.78 (m, 1H), 2.65-2.59 (m, 1H).

ESI-MS [(M-H)⁺]: m/z of IIb-15 calculated: 352.1, founded: 352.0.

### Example 16

### Synthesis of Compound IIb-16

The synthesis method for the preparation of compound **IIb-16** was the same as that of Example 1, and the product **IIb-16** was obtained by etherification and hydrolysis reaction, wherein compound **RM-Ib-15** (0.39 mmol) was used in the reaction instead of compound **RM-Ib-1** to obtain intermediate RM-IIb-16, and the white solid product **IIb-16** (0.032 g) was obtained from the intermediate by hydrolysis in two steps. Yield: 19.7%.

¹H-NMR for the**IIb-16** hydrochloride (400 MHz, CDCl3)δ: 7.51-7.48 (m, 2H), 7.29-7.27 (m, 2H), 7.06-7.04 (m, 1H), 6.47-6.43 (m, 2H), 4.97 (s, 2H), 4.78-4.74 (m, 1H), 4.30-4.27 (m, 1H), 3.83-3.78 (m, 1H), 2.83-2.77 (m, 1H), 2.65-2.58 (m, 1H).

ESI-MS [(M-H)⁺]: m/z of IIb-16 calculated: 405.0, founded: 404.8.

### Example 17

### Synthesis of Compound IIb-17

The synthesis of compound **IIb-17** was carried out in the same way as in Example 1, and the product **IIb-17** was obtained by etherification and hydrolysis reaction, in which compound **RM-Ib-16** (0.28 mmol) was used in the reaction instead of compound **RM-Ib-1** to obtain intermediate **RM-IIb-17,** and the white solid product **IIb-17** (0.020 g) was obtained from the intermediate by hydrolysis in two steps. Yield: 15.8%.

¹H-NMR for the**IIb-17**hydrochloride (400 MHz, CDCl₃)δ:7.30-7.18 (m, 1H), 7.10-7.06 (m, 1H), 7.03-7.01 (m, 1H), 6.50-6.46 (m, 2H), 5.07 (s, 2H), 4.79-4.75 (m, 1H), 4.31-4.27 (m, 1H), 3.84-3.79 (m, 1H), 2.83-2.78 (m, 1H), 2.65-2.58 (m, 1H). ¹⁹F NMR (376 MHz, CDCl₃): δ -49.74.

ESI-MS [(M-H)⁺]: m/z of IIb-17 calculated: 363.1, founded: 362.9.

### Example 18

### Synthesis of Compound IIb-18

The synthesis of compound **IIb-18** was carried out in the same way as in Example 1, and the product IIb-18 was obtained by etherification and hydrolysis, wherein compound **RM-Ib-16** (0.22 mmol) was used instead of compound RM-Ib-1 and compound **SM1-2** (0.22 mmol) was used instead of **SM1-1** to obtain intermediate **RM-IIb-18.** The intermediate was hydrolyzed to give the white solid product **IIb-18** (0.009 g) in two-step yield: 11.4%.

¹H-NMR for the**IIb-18** hydrochloride (400 MHz, CDCl₃) δ: 7.20-7.18 (m, 1H), 7.10-7.03 (m, 2H), 7.01 (m, 1H), 6.51-6.46 (m, 2H), 5.07 (s, 2H), 4.79-4.75 (m, 1H), 4.32-4.28 (m, 1H), 3.84-3.79 (m, 1H), 2.85-2.79 (m, 1H), 2.66-2.60 (m, 1H); ¹⁹F NMR (376 MHz, CDCl₃): δ -49.63.

ESI-MS [(M-H)⁺]: m/z of **IIb-18** calculated: 363.1, founded: 363.1.

### Example 19

### Synthesis of Compound IIb-19

The synthesis of compound **IIb-19** was carried out in the same way as in Example 1, and the product **IIb-19** was obtained by etherification and hydrolysis, in which compound **RM-Ib-17** (0.91 mmol) was used in the reaction instead of compound **RM-Ib-1** to obtain intermediate **RM-IIb-19,** and the light yellow solid product **IIb-19** (0.231 g) was obtained from the intermediate by hydrolysis. Two-step yield: 72.8%.

¹H-NMR for the **IIb-19**hydrochloride (400 MHz, CDCl3) δ: 7.06-7.04 (m, 1H), 7.00-6.98 (m, 1H), 6.84-6.83 (m, 2H), 6.53-6.48 (m, 2H), 5.02 (s, 2H), 4.78-4.74 (m, 1H), 4.30-4.23 (m, 5H), 3.83-3.77 (m, 1H), 2.82-2.77 (m, 1H), 2.63-2.57 (m, 1H).

ESI-MS [(M-H)⁺]: m/z of IIb-19 calculated: 341.1, founded: 341.0.

### Example 20

### Synthesis of compound IIb-20

The synthesis of compound **IIb-20** was carried out in the same way as in Example 1, and the product **IIb-20** was obtained by etherification and hydrolysis reaction, in which compound **RM-Ib-18** (0.64 mmol) was used in the reaction instead of compound **RM-Ib-1** to obtain intermediate **RM-IIb-20,** and the light yellow solid product **IIb-20** (0.166 g) was obtained from the intermediate by hydrolysis. Two-step yield: 71.2%.

¹H-NMR for the **IIb-20**hydrochloride (400 MHz, CDCl3)δ: 7.05-7.03 (m, 1H), 6.91-6.89 (m, 1H), 6.80-6.76 (m, 1H), 6.70-6.68 (m, 1H), 6.52-6.49 (m, 2H), 4.98 (s, 2H), 4.77-4.73 (m, 1H), 4.30-4.26 (m, 1H), 3.82-3.77 (m, 1H), 2.81-2.76 (m, 1H), 2.62-2.55 (m, 1H), 1.69 (s, 6H).

ESI-MS [(M-H)⁺]: m/z of IIb-20 calculated: 355.1, founded: 355.0.

### Example 21

### Synthesis of Compound IIb-21

The synthesis of compound **IIb-21** was carried out in the same way as in Example 1, and the product **IIb-21** was obtained by etherification and hydrolysis, in which compound **RM-Ib-19** (0.57 mmol) was used in the reaction instead of RM-Ib-1 to obtain intermediate **RM-IIb-21,** and the light yellow solid product IIb-21 (0.072 g) was obtained from the intermediate by hydrolysis. Two-step yield: 28.4%.

ESI-MS [(M-H)⁺]: m/z of IIb-21calculated: 441.0, founded: 440.8.

### Example 22

### Synthesis of Compound IIb-22

The synthesis method for the preparation of compound IIb-22 was the same as that of Example 1, and the product **IIb-22** was obtained by etherification and hydrolysis reaction, in which compound **RM-Ib-20** (0.43 mmol) was used in the reaction instead of compound **RM-Ib-1** to obtain intermediate **RM-IIb-22,** and light yellow oil **IIb-22** (0.150 g) was obtained from the intermediate by hydrolysis in two steps. Yield: 80%.

ESI-MS [(M-H)⁺]: m/z oflIb-22calculated: 435.0, founded: 434.9.

### Example 23

### Synthesis of Compound IIb-23

The synthesis of compound **IIb-23** was carried out in the same way as in Example 1, and the product **IIb-23** was obtained by etherification and hydrolysis, wherein compound **RM-Ib-21** (0.13 mmol) was used in the reaction instead of compound **RM-Ib-1** to obtain intermediate **RM-IIb-23,** and the light yellow solid product **IIb-23** (0.0033 g) was obtained from the intermediate by hydrolysis. Two-step yield: 6.2%.

ESI-MS [(M-H)⁺]: m/z of IIb-23 calculated: 397.0, founded: 397.3.

### Example 24

### Synthesis of compound IIb-24

The synthesis of compound **IIb-24** was carried out in the same way as in Example 1, and the product **IIb-24** was obtained by etherification and hydrolysis, in which compound **RM-Ib-22** (0.38 mmol) was used in the reaction instead of RM-Ib-1 to obtain intermediate **RM-IIb-24,** and the light yellow solid product **IIb-24** (0.028 g) was obtained from the intermediate by hydrolysis. Two-step yield: 18.8%.

ESI-MS [(M-H)⁺]: m/z oflIb-24calculated: 397.0, founded: 397.4.

### Example 25

### Synthesis of compound IIb-25

The synthesis of compound **IIb-25** was prepared in the same way as in Example 1, and the product **IIb-25** was obtained by etherification and hydrolysis reaction, where compound **RM-Ib-5** (0.54 mmol) was used in the reaction instead of compound **RM-Ib-1,** and compound **SM1-2** (0.54 mmol) was used instead of compound **SM1-1** to obtain intermediate **RM-IIb-25,** which was obtained from intermediate **IIb-25** (0.085 g) was obtained as a white solid product by hydrolysis in two-step yield: 44%.

¹H-NMR for the IIb-25hydrochloride (400 MHz, CDCl3)δ: 7.07-7.05 (m, 1H), 6.94 (m, 1H), 6.78-6.77 (m, 1H), 6.50-6.45 (m, 2H), 6.02 (s, 2H), 4.95 (s, 2H), 4.79-4.74 (m, 1H), 4.31-4.27 (m, 1H), 3.84-3.79 (m, 1H), 2.84-2.79 (m, 1H), 2.66-2.59 (m, 1H).

ESI-MS [(M-H)⁺]: m/z of IIb-25calculated: 361.0, founded: 361.0.

### Example 26

### Synthesis of Compound IIb-26

The synthesis of compound **IIb-26** was prepared as in Example 1, and the product **IIb-26** was obtained by etherification and hydrolysis reaction, where compound **RM-Ib-16** (0.22 mmol) was used in the reaction instead of compound **RM-Ib-1**, and compound **SM1-3** (0.22 mmol) was used instead of compound **SM1-1** to obtain intermediate **RM-IIb-26**, which was obtained from The intermediate was hydrolyzed to give the white solid product **IIb-26** (0.045 g) in two-step yield: 21%.

¹H-NMR for the**IIb-26** hydrochloride(400 MHz, CDCl3) δ: 7.33 (s, 1H), 7.32-7.30 (m, 1H), 7.13-7.09 (m, 1H), 7.06-7.02 (m, 1H), 6.50 (s, 1H), 5.13 (s, 2H), 4.80-4.76 (m, 1H), 4.32-4.29 (m, 1H), 3.845-3.81 (m, 1H), 2.83-2.78 (m, 1H), 2.67-2.60 (m, 1H).

ESI-MS [(M-H)⁺]: m/z of IIb-26 calculated: 441.0, founded: 441.0.

### Sample 27

### Synthesis of Compound IIb-27

The synthesis of compound **IIb-27** was prepared as in Example 1, and the product **IIb-27** was obtained by etherification and hydrolysis reaction, where compound **RM-Ib-16** (0.22 mmol) was used in the reaction instead of compound **RM-Ib-1**, and compound **SM1-4** (0.22 mmol) was used instead of compound **SM1-1** to obtain intermediate **RM-IIb-27,** which was obtained from The intermediate was hydrolyzed to give the white solid product IIb-27 (0.070 g) in two-step yield: 28%.

¹H-NMR for the **IIb-27**hydrochloride (400 MHz, CDCl3) δ: 7.43-7.41 (m, 1H), 7.32 (s, 1H), 7.14-7.10 (m, 1H), 7.06-7.04 (m, 1H), 5.08 (s, 2H), 4.93-4.88 (m, 1H), 4.45-4.41 (m, 1H), 4.00-3.96 (m, 1H), 2.88-2.82 (m, 2H), 2.74-2.67 (m, 1H); ¹⁹F NMR (376 MHz, CDCl₃): δ -49.68.

ESI-MS [(M-H)⁺]: m/z of IIb-27 calculated: 520.9, founded: 520.9.

### Example 28

### Synthesis of Compound IIb-28

The synthesis of compound **IIb-28** was prepared in the same way as in Example 1, and the product **IIb-28** was obtained by etherification and hydrolysis reaction, where compound **RM-Ib-16** (0.48 mmol) was used in the reaction instead of compound **RM-Ib-1,** and compound **SM1-5** (0.48 mmol) was used instead of compound **SM1-1** to obtain intermediate **RM-IIb-28,** which was obtained from The intermediate was hydrolyzed to give the white solid product **IIb-28** (0.070 g) in two-step yield: 36%.

¹H-NMR for the**IIb-28**hydrochloride (400 MHz, CDCl3) δ: 7.29-7.26 (m, 1H), 7.18 (s, 1H), 7.12-7.08 (m, 1H), 7.04-7.02 (m, 1H), 6.50 (s, 2H), 5.14 (s, 2H), 4.80-4.75 (m, 1H), 4.32-4.28 (m, 1H), 3.84-3.80 (m, 1H), 2.83-2.77 (m, 1H), 2.66-2.60 (m, 1H).

ESI-MS [(M-H)⁺]:m/z of IIb-28 calculated: 397.0, founded: 397.0.

### Example 29

### Synthesis of Compound IIb-29

The synthesis method for the preparation of compoun**d IIb-29** was the same as that of Example 1, and the product **IIb-29** was obtained by etherification and hydrolysis reaction, where compound **RM-Ib-16** (1.0 mmol) was used in the reaction instead of compound **RM-Ib-1** and compound **SM1-6** (1.0 mmol) was used instead of compound **SM1-1** to obtain intermediate **RM-IIb-29,** which was obtained from the intermediate by The white solid product **IIb-29** (0.070 g) was obtained by hydrolysis, two-step yield: 18%.

ESI-MS [(M-H)⁺]:m/z of IIb-29 calculated: 381.1, founded: 381.0.

### Example 30

### Synthesis of compound IIb-30

The reaction was completed by adding LiHMDS (1M, 1.4mL, 1.4mmol) dropwise and stirring for 0.5h. TMSCl (0.14g, 1.3mmol) was added dropwise and stirred for 0.5h, Add NBS (0.11g, 0,62mmol), slowly raise to room temperature, and react for 5h, the reaction is completed.HPLC showed that after the reaction, the reaction solution was added with water, neutralized with hydrochloric acid (1N) to a pH of about 2, extracted with ethyl acetate, and then the organic phases were combined, washed with saline and dried, and finally purified by column chromatography to give the light yellow solid product IIb-30 (0.055 g) in one-step yield: 24%.

ESI-MS [(M-H)⁺]: m/z of IIb-30calculated: 441.0, founded: 440.9.

### Example 31

### Synthesis of compound IIb-31

The reaction was completed by adding the raw material **IIb-30** (0.035 g, 0.08 mmol) to concentrated ammonia (3 mL) and heating in an oil bath at 100 degrees C for 2.5h.HPLC showed that the reaction solution was concentrated and dried under reduced pressure to obtain the light yellow solid product **IIb-31** (0.025g) with one-step yield: 83%.

ESI-MS [(M+H)⁺]: m/z of IIb-31calculated: 380.1, founded: 380.0.

### Example 32

### Synthesis of Compound IIb-32

### Step 1:

RM-IIb-17 (0.29 g, 0.78 mmol) was dissolved in 2 mL of THF, protected by nitrogen and cooled to -70C in an acetone bath on dry ice, LiHMDS (1 M, 0.8 mL, 0.8 mmol) was added dropwise and stirred for 0.5 h. (PhSO2)NF/THF (0.29 g, 0.93 mmol) solution was added dropwise, and the reaction was slowly increased to room temperature. The reaction was completed at room temperature for 2h.HPLC showed that after the reaction was finished, the reaction solution was added with water, neutralized with hydrochloric acid (1N) to a pH of about 2, extracted with ethyl acetate, then the organic phases were combined, washed with saline and dried, and finally purified by column chromatography to obtain intermediate RM-IIb-30 (0.043g).

### Step 2:

MeOH (2 mL), THF (1 mL) and aqueous LiOH (1N, 1 mL) were added sequentially to intermediate **RM-IIb-30** and stirred at room temperature for 1 h. After the reaction was completed, the reaction solution was neutralized with hydrochloric acid (1N) to pH about 4, water was added, ethyl acetate was added for extraction, and then the organic phases were combined, washed with saline and dried, and finally purified by column chromatography. A white solid product **IIb-32** (0.018 g) was obtained in two-step yield:6%.

ESI-MS [(M-H)⁺]: m/z of**IIb-32**calculated: 379.1, founded: 379.0.

### Example 33

### Synthesis of Compound IIb-33

### Step 1:

**RM-IIb-17** (0.24 g, 0.65 mmol) was dissolved in 2 mL of THF, protected by nitrogen and cooled to -70C in an acetone bath on dry ice, LiHMDS (1 M, 0.8 mL, 0.8 mmol) was added dropwise, stirred for 0.5 h, and (PhSO₂)NF/THF (0.24 g, 0.78 mmol) solution was added dropwise, and the reaction was slowly increased to room temperature. The reaction was completed at room temperature for 2h.HPLC showed that after the reaction was finished, the reaction solution was added with water, neutralized with hydrochloric acid (1N) to pH about 2, extracted by adding ethyl acetate, and then the organic phases were combined, washed with saline and dried, and finally purified by column chromatography to obtain intermediates **RM-IIb-31 and RM-IIb-32** (0.045g).

### Step 2:

MeOH (2 mL), THF (1 mL) and aqueous LiOH (1N, 1 mL) were added to intermediates RM-IIb-31 and RM-IIb-32 in turn and stirred at room temperature for 1 h. After the reaction was completed, the reaction solution was neutralized with hydrochloric acid (1N) to a pH of about 4, water was added, ethyl acetate was added for extraction, and then the organic phases were combined, washed with saline and dried. Finally, the reaction was purified by column chromatography, and the light yellow solid products **IIb-33** and **IIb-34** (0.030 g) were obtained in two-step yield:12%.

ESI-MS [(M-H)⁺]: m/z of**IIb-33**calculated: 381.1, found: 381.0.

### Example 34

### Synthesis of compound IIb-34

The synthesis of compound **IIb-34** was prepared as in Example 1, and the product IIb-34 was obtained by etherification and hydrolysis reaction, where compound **RM-Ib-16** (0.36 mmol) was used in the reaction instead of compound **RM-Ib-1,** and compound **SM1-7** (0.36 mmol) was used instead of compound SM1-1 to obtain intermediate **RM-IIb-32,** which was obtained from intermediate was hydrolyzed to give the white solid product **IIb-34** (0.021 g) in two-step yield: 14%.

ESI-MS [(M-H)⁺]: m/z of **IIb**-34calculated: 399.0, founded: 399.0.

### Example 35

### Synthesis of compound IIb-35

The synthesis method for the preparation of compound **IIb-35** was the same as that of Example 1, and the product **IIb-35** was obtained by etherification and hydrolysis reaction, where compound **RM-Ib-6** (0.27 mmol) was used in the reaction instead of compound **RM-Ib-1** and compound **SM1-8** (0.27 mmol) was used instead of compound **SM1-1** to obtain intermediate **RM-IIb-33,** and from intermediate **IIb-35** (0.056 g) was obtained as a light yellow solid product by hydrolysis in two-step yield: 60%.

¹H-NMR for the**IIb-35**hydrochloride (400 MHz, CDCl3) δ: 7.12-7.10 (m, 1H), 6.85 (m, 1H), 6.80-6.78 (m, 1H), 6.69-6.66 (m, 1H), 6.57-6. 54 (m, 1H), 6.01 (S, 2H), 4.99 (S, 2H) 3.56-3.52 (m, 1H), 2.92-2.77 (m, 3H), 2.51-2.41 (m, 2H), 1.81-1.74 (m, 1H).

ESI-MS [(M-H)⁺]: m/z of **IIb-35** calculated: 343.1, founded: 343.0.

### Example 36

### Synthesis of compound IIb-36

The synthesis method for the preparation of compound **IIb-36** was the same as that of Example 1, and the product **IIb-36** was obtained by etherification and hydrolysis reaction, where compound **RM-Ib-6** (0.27 mmol) was used in the reaction instead of compound **RM-Ib-1,** and compound **SM1-9** (0.27 mmol) was used instead of compound **SM1-1** to obtain intermediate **RM-IIb-34,** and from intermediate IIb-36 (0.072 g) was obtained as a light yellow solid product by hydrolysis in two-step yield: 75%.

¹H-NMR for the**IIb-36**hydrochloride (400 MHz, CDCl3)δ: 7.31-7.26 (m, 2H), 6.93-6.91 (m, 2H), 6.69-6.68 (m, 1H), 6.66 (m, 1H), 6.01 (s, 2H), 4.99 (s, 2H), 2.64 (s, 2H), 1.45 (s, 6H).

ESI-MS [(M-H)⁺]: m/z of **IIb**-36calculated: 345.1, founded: 345.0.

### Example 37

### Synthesis of compound IIb-37

The synthesis of compound **IIb-37** was prepared in the same way as in Example 1, and the product **IIb-37** was obtained by etherification and hydrolysis reaction, where compound **RM-Ib-6** (0.27 mmol) was used in the reaction instead of compound **RM-Ib-1,** and compound **SM1-10** (0.27 mmol) was used instead of compound **SM1-1** to obtain intermediate **RM-IIb-35,** which was obtained from The intermediate was hydrolyzed to give the light yellow solid product **IIb-37** (0.055 g) in two-step yield: 53%.

¹H-NMR for the IIb-37hydrochloride (400 MHz, CDCl3) δ: 7.41-7.39 (m, 2H), 6.95-6.92 (m, 2H), 6.67-6.63 (m, 1H), 6.57-6.55 (m, 1H), 6.03-6.01 (m, 2H), 4.99 (s, 2H), 4.13-4.10 (m, 2H), 3.83-3.82 (m, 2H), 2.98 (s, 2H).

ESI-MS [(M-H)⁺]: m/z of **IIb-37**calculated: 375.1, founded: 374.9.

### Example 38

### Synthesis of compound IIb-38

The synthesis of compound **IIb-38** was prepared as in Example 2, and the product **IIb-38** was obtained by etherification and hydrolysis reaction, where compound **RM-Ib-23** (0.16 mmol) was used in the reaction instead of compound **RM-Ib-2,** and compound **SM1-2** (0.16 mmol) was used instead of compound **SM1-1** to obtain intermediate **RM-IIb-36,** which was obtained from intermediate was hydrolyzed to give the white solid product I**Ib-38** (0.040 g) in two-step yield:44%.

ESI-MS [(M-H)⁺]: m/z of**IIb-38** calculated: 567.2, founded: 567.0.

### Example 39

### Synthesis of Compound IIb-39

The synthesis of compound **IIb-39** was prepared as in Example 1, and the product **IIb-39** was obtained by etherification and hydrolysis reaction, where compound **RM-Ib-5** (0.35 mmol) was used in the reaction instead of compound **RM-Ib-1,** and compound **SM1-7** (0.35 mmol) was used instead of compound **SM1-1** to obtain intermediate **RM-IIb-37,** which was obtained from intermediate IIb-39 (0.021 g) was obtained as a white solid product by hydrolysis, two-step yield:15%.

ESI-MS [(M-H)⁺]: m/z of IIb-39 calculated: 397.0, founded: 397.0.

### Example 40

### Synthesis of compound IIb-40

The synthesis of compound **IIb-40** was prepared as in Example 2, and the product **IIb-40** was obtained by etherification and hydrolysis reaction, where compound **RM-Ib-24** (0.31 mmol) was used in the reaction instead of compound **RM-Ib-2,** and compound **SM1-2** (0.31 mmol) was used instead of compound **SM1-1** to obtain intermediate **RM-IIb-38,** which was obtained from intermediate was hydrolyzed to give the white solid product **IIb-40** (0.067 g) in two-step yield:47%.

ESI-MS [(M-H)⁺]: m/z of IIb-40 calculated: 453.0, founded: 453.0.

### Example 41

### Synthesis of compound IIb-41

The synthesis of compound **IIb-41** was prepared as in Example 2, and the product **IIb-41** was obtained by etherification and hydrolysis reaction, where compound **RM-Ib-25** (0.10 mmol) was used in the reaction instead of compound **RM-Ib-2,** and compound **SM1-2** (0.10 mmol) was used instead of compound SM1-1 to obtain intermediate **RM-IIb-39,** which was obtained from intermediate was hydrolyzed to give the white solid product **IIb-41** (0.014 g) in two-step yield:36%.

ESI-MS [(M+H)⁺]: m/z of **IIb-41**calculated: 372.1, founded: 372.1.

### Example 42

### Synthesis of compound IIb-42

The synthesis of compound **IIb-42** was prepared as in Example 2, and the product **IIb-42** was obtained by etherification and hydrolysis reaction, where compound **RM-Ib-26** (0.13 mmol) was used in the reaction instead of compound **RM-Ib-2,** and compound **SM1-2** (0.13 mmol) was used instead of compound **SM1-1** to obtain intermediate **RM-IIb-40,** which was obtained from intermediate was hydrolyzed to give the white solid product **IIb-42** (0.029 g) in two-step yield:58%.

ESI-MS [(M+H)⁺]: m/z of IIb-42 calculated: 372.1, founded: 372.1

### Example 43

### Synthesis of compound IIb-43

The synthesis of compound **IIb-43** was prepared in the same way as in Example 2, and the product **IIb-43** was obtained by etherification and hydrolysis reaction, where compound **RM-Ib-27** (0.09 mmol) was used in the reaction instead of compound **RM-Ib-2,** and compound **SM1-2** (0.09 mmol) was used instead of compound **SM1-1** to obtain intermediate **RM-IIb-41,** which was obtained from intermediate was hydrolyzed to give the white solid product **IIb-43** (0.012 g) in two-step yield: 31%.

¹H-NMR for the IIb-43 hydrochloride (400 MHz, CDCl3) δ: 7.06-7.03 (m, 2H), 6.73 (s, 1H), 6.49-6.46 (m, 2H), 5.96 (s, 2H), 4.95 (s, 2H), 4.78-4.73 (m, 1H), 4.30-4.26 (m, 1H), 3.82-3.78 (m, 1H), 2.83-2.77 (m, 1H), 2.65-2.58 (m, 1H) , 1.49 (s, 9H).

ESI-MS [(M-H)⁺]: m/z of **IIb-43**calculated: 442.1, founded: 442.2.

### Example 44

### Synthesis of Compound IIb-44

Compound **IIb-43** (0.017g,0.04mmol) was added to HCl/MeOH (6N,2mL) and stirred at room temperature for 1h, and the reaction was detected to be complete, and the white solid product **IIb-44** (0.015g) was obtained by concentration in one-step yield:91%.

ESI-MS [(M+H)⁺]: m/z of **IIb-44**calculated: 344.1, founded: 344.1.

### Example 45

### Synthesis of compound IIb-45

The synthesis of compound **IIb-45** was prepared as in Example 2, and the product **IIb-45** was obtained by etherification and hydrolysis reaction, where compound **RM-Ib-28** (0.14 mmol) was used in the reaction instead of compound **RM-Ib-2,** and compound **SM1-2** (0.14 mmol) was used instead of compound **SM1-1** to obtain intermediate **RM-IIb-42,** which was obtained from intermediate was hydrolyzed to give the white solid product **IIb-45** (0.035 g) in two-step yield:66%.

ESI-MS [(M-H)⁺]: m/z of IIb-45 calculated: 357.3, founded: 357.2.

### Example 46

### Synthesis of compound IIb-46

The synthesis of compound **IIb-46** was prepared as in Example 2, and the product **IIb-46** was obtained by etherification and hydrolysis reaction, where compound **RM-Ib-29** (0.12 mmol) was used in the reaction instead of compound **RM-Ib-2,** and compound **SM1-2** (0.12 mmol) was used instead of compound **SM1-1** to obtain intermediate **RM-IIb-43,** which was obtained from intermediate was hydrolyzed to give the white solid product **IIb-46** (0.030 g) in two-step yield:52%.

ESI-MS [(M-H)⁺]: m/z of **IIb-46**calculated: 478.4, founded: 478.3.

### Example 47

### Synthesis of Compound IIb-47

### Step 1:

The compound **RM-IIb-41** (0.6 g,1.31 mmol) was added to HCl/MeOH (6N,12 mL) and stirred overnight at room temperature, and the reaction was assayed to completion and concentrated to give the white solid intermediate **RM-IIb-44** (0.5 g).

### Step 2:

Dissolve the raw material **RM-IIb-44** (0.050g, 0.13mmol) in 2mL of DCM, add DMAP (0.039g, 0.32mmol), and add methyl chloroformate (0.015g, 0.15mmol) under the cooling of ice water bath, stir the reaction overnight at room temperature, and the reaction was completed. the reaction solution was post-treated after the HPLC showed the end of the reaction The intermediate **RM-IIb-45** (0.049g) was purified by column chromatography.

### Step 3:

To intermediate **RM-IIb-45,** MeOH (2mL), THF (1mL) and aqueous LiOH (1N, 1mL) were added sequentially and stirred at room temperature for 1h, HPLC showed that the reaction solution was neutralized with hydrochloric acid (1N) to pH about 4, water was added, ethyl acetate was added for extraction, then the organic phases were combined, washed with saline and dried, and finally purified by column chromatography. The white solid product **IIb-47** (0.047 g) was obtained in two-step yield:92%.

ESI-MS [(M-H)+]: m/z of **IIb-47**calculated: 400.4, founded: 400.3.

### Example 48

### Synthesis of Compound IIb-48

### Step 1:

**RM-IIb-44** (0.050 g, 0.13 mmol) was dissolved in 2 mL of DCM, DMAP (0.039 g, 0.32 mmol) was added and cyclopentyl chloroformate (0.023 g, 0.15 mmol) was added under cooling in an ice water bath and the reaction was completed by stirring at room temperature overnight.The HPLC showed that the reaction solution was purified by post-treatment column chromatography after the reaction was finished, and intermediate **RM-IIb-46** (0.050g) was obtained.

### Step 2:

To intermediate **RM-IIb-46,** MeOH (2mL), THF (1mL) and aqueous LiOH (1N, 1mL) were added sequentially and stirred at room temperature for 1h, HPLC showed that the reaction solution was neutralized with hydrochloric acid (1N) to pH about 4, water was added, ethyl acetate was added for extraction, then the organic phases were combined, washed with saline and dried, and finally purified by column chromatography. A white solid product **IIb-48** (0.040 g) was obtained in two-step yield:70%.

ESI-MS [(M-H)⁺]: m/z of IIb-48 calculated: 454.5, founded: 454.5.

### Example 49

### Synthesis of Compound IIb-49

### Step 1:

The reaction was completed by dissolving **RM-IIb-44** (0.050g, 0.13mmol) in 2mL of DCM, adding DMAP (0.039g, 0.32mmol), cooling in an ice water bath, adding phenyl chloroformate (0.024g, 0.15mmol), and stirring the reaction overnight at room temperature. The intermediate (0.050g) was obtained by column chromatography separation and purification.

### Step 2:

The intermediate was redissolved in 2 mL of DMF, DMAP (0.025 g, 0.2 mmol) and tert-butylamine (0.015 g, 0.2 mmol) were added, and the reaction was stirred at room temperature for 3 h. The reaction was purified by post-treatment column chromatography after the reaction was shown by HPLC, and intermediate **RM-IIb-47** (0.040 g) was obtained.

### Step 3:

To intermediate **RM-IIb-47,** MeOH (2mL), THF (1mL) and aqueous LiOH (1N, 1mL) were added sequentially and stirred at room temperature for 1h, HPLC showed that the reaction solution was neutralized with hydrochloric acid (1N) to pH about 4, water was added, ethyl acetate was added for extraction, and then the organic phases were combined, washed with saline and dried, and finally purified by column chromatography. A white solid product **IIb-49** (0.020 g) was obtained in three-step yield:35%.

ESI-MS [(M-H)⁺]: m/z of **IIb-49**calculated: 441.5, founded: 441.4.

### Example 50

### Synthesis of Compound IIb-50

### Step 1:

**RM-IIb-44** (0.050g, 0.13mmol) was dissolved in 2mL of DCM, DMAP (0.039g, 0.32mmol) was added, and cyclopentyl chloroformate (0.023g, 0.15mmol) was added under cooling in an ice water bath, and the reaction was stirred at room temperature overnight, and the reaction was completed. After the reaction was completed as indicated by HPLC, the reaction solution was post-treated The intermediate **RM-IIb-48** (0.045g) was purified by column chromatography.

### Step 2:

MeOH (2mL), THF (1mL) and aqueous LiOH (1N, 1mL) were added sequentially to intermediate **RM-IIb-48** and stirred at room temperature for 1h, HPLC showed that the reaction solution was neutralized with hydrochloric acid (1N) to pH about 4, water was added, ethyl acetate was added for extraction, then the organic phases were combined, washed with saline and dried, and finally purified by column chromatography. A white solid product **IIb-50** (0.020 g) was obtained in two-step yield:46%.

ESI-MS [(M-H)⁺]: m/z of IIb-50 calculated: 426.7, founded: 426.4.

### Example 51

### Synthesis of Compound IIb-51

### Step 1:

**RM-IIb-44** (0.050 g, 0.13 mmol) was dissolved in 2 mL of DCM, DMAP (0.039 g, 0.32 mmol) was added, and the reaction was completed by stirring at room temperature overnight under cooling in an ice water bath. The intermediate **RM-IIb-49** (0.015g) was purified by column chromatography.

### Step 2:

MeOH (2mL), THF (1mL) and aqueous LiOH (1N, 1mL) were added sequentially to intermediate **RM-IIb-49** and stirred at room temperature for 1h, HPLC showed that the reaction solution was neutralized with hydrochloric acid (1N) to pH about 4, water was added, ethyl acetate was added for extraction, then the organic phases were combined, washed with saline and dried, and finally purified by column chromatography. The white solid product **IIb-51** (0.009 g) was obtained in two-step yield: 16%.

ESI-MS [(M-H)⁺]: m/z of IIb-51 calculated: 448.5, founded: 448.3.

### Example 52

### Synthesis of Compound IIb-52

The synthesis of compound **IIb-52** was prepared as in Example 2, and the product **IIb-52** was obtained by etherification and hydrolysis reaction, where compound **RM-Ib-30** (0.06 mmol) was used in the reaction instead of compound **RM-Ib-2,** and compound **SM1-2** (0.06 mmol) was used instead of compound **SM1-1** to obtain intermediate RM-IIb-50, which was obtained from intermediate was hydrolyzed to give the white solid product **IIb-52** (0.004g) in two-step yield: 15%.

ESI-MS [(M-H)⁺]: m/z of IIb-52 calculated: 411.1, founded: 411.2.

### Example 53

### Synthesis of compound IIb-53

The synthesis of compound **IIb-53** was prepared as in Example 2, and the product **IIb-53** was obtained by etherification and hydrolysis reaction, where compound **RM-Ib-31** (0.24 mmol) was used in the reaction instead of compound **RM-Ib-2,** and compound **SM1-2** (0.24 mmol) was used instead of compound **SM1-1** to obtain intermediate **RM-IIb-51,** which was obtained from intermediate was hydrolyzed to give the white solid product **IIb-53** (0.018 g) in two-step yield: 19%.

ESI-MS [(M-H)⁺]: m/z of IIb-53 calculated: 395.1, founded: 395.2.

### Example 54

### Synthesis of Compound IIb-54

The synthesis of compound **IIb-54** was prepared as in Example 2, and the product **IIb-54** was obtained by etherification and hydrolysis reaction, where compound **RM-Ib-32** (0.24 mmol) was used in the reaction instead of compound **RM-Ib-2,** and compound **SM1-2** (0.24 mmol) was used instead of compound SM1-1 to obtain intermediate **RM-IIb-52,** which was obtained from intermediate was hydrolyzed to give the white solid product **IIb-54** (0.028 g) in two-step yield:27%.

ESI-MS [(M-H)⁺]: m/z of IIb-54 calculated: 431.0, founded: 431.1.

### Example 55

### Synthesis of compound IIb-55

The synthesis of compound **IIb-55** was prepared as in Example 2, and the product **IIb-55** was obtained by etherification and hydrolysis reaction, where compound **RM-Ib-33** (0.24 mmol) was used in the reaction instead of compound **RM-Ib-2,** and compound **SM1-2** (0.24 mmol) was used instead of compound **SM1-1** to obtain intermediate **RM-IIb-53,** which was obtained from intermediate was hydrolyzed to give the white solid product **IIb-55** (0.020 g) in two-step yield:22%.

ESI-MS [(M-H)⁺]: m/z of IIb-55 calculated: 381.1, founded: 381.2.

### Example 56

### Synthesis of Compound IIb-56

The synthesis of compound **IIb-56** was prepared in the same way as in Example 2, and the product **IIb-56** was obtained by etherification and hydrolysis reaction, where compound **RM-Ib-34** (0.24 mmol) was used in the reaction instead of compound **RM-Ib-2,** and compound **SM1-2** (0.24 mmol) was used instead of compound SM1-1 to obtain intermediate **RM-IIb-54,** which was obtained from intermediate was hydrolyzed to give the white solid product **IIb-56** (0.028 g) in two-step yield:30%.

ESI-MS [(M-H)⁺]: m/z of IIb-56 calculated: 397.0, founded: 397.1.

### Example 57

### Synthesis of Compound IIb-57

The synthesis of compound **IIb-57** was prepared in the same way as in Example 2, and the product **IIb-57** was obtained by etherification and hydrolysis reaction, where compound **RM-Ib-35** (0.23 mmol) was used in the reaction instead of compound **RM-Ib-2** and compound **SM1-11** (0.23 mmol) was used instead of compound **SM1-1** to obtain intermediate **RM-IIb-55** from intermediate was hydrolyzed to give the white solid product **IIb-57** (0.018 g) in two-step yield:21%.

ESI-MS [(M-H)⁺]: m/z of IIb-57 calculated: 363.1, founded: 363.2.

### Example 58

### Synthesis of compound IIb-58

The synthesis of compound IIb-58 was prepared as in Example 2, and the product IIb-58 was obtained by etherification and hydrolysis reaction, where compound RM-Ib-36 (0.23 mmol) was used in the reaction instead of compound RM-Ib-2, and compound SM1-11 (0.23 mmol) was used instead of compound SM1-1 to obtain intermediate RM-IIb-56, which was obtained from intermediate was hydrolyzed to give the white solid product IIb-58 (0.026 g) in two-step yield:30%.

ESI-MS [(M-H)⁺]: m/z of IIb-58 calculated: 379.0, founded: 379.1.

### Example 59

### Synthesis of Compound IIb-59

The synthesis of compound **IIb-59** was prepared as in Example 2, and the product **IIb-59** was obtained by etherification and hydrolysis reaction, where compound **RM-Ib-27** (0.23 mmol) was used in the reaction instead of compound **RM-Ib-2,** and compound **SM1-11** (0.23 mmol) was used instead of compound SM1-1 to obtain intermediate **RM-IIb-57,** which was obtained from intermediate was hydrolyzed to give the white solid product **IIb-59** (0.016 g) in two-step yield:30%.

ESI-MS [(M-H)⁺]: m/z of IIb-59 calculated: 460.1, founded: 460.2.

### Example 60

### Synthesis of compound IIb-60

The synthesis of compound **IIb-60** was prepared in the same way as in Example 2, and the product **IIb-60** was obtained by etherification and hydrolysis reaction, where compound **RM-Ib-27** (0.24 mmol) was used in the reaction instead of compound **RM-Ib-2** and compound **SM1-8** (0.24 mmol) was used instead of compound **SM1-1** to obtain intermediate **RM-IIb-58,** which was obtained from intermediate was hydrolyzed to give the white solid product **IIb-60** (0.021 g) in two-step yield:20%.

ESI-MS [(M-H)⁺]: m/z of IIb-60 calculated: 442.2, founded: 442.1.

### Example 61

### Synthesis of compound IIb-61

The synthesis of compound **IIb-61** was prepared in the same way as in Example 1, and the product **IIb-61** was obtained by etherification and hydrolysis reaction, where compound **RM-Ib-16** (0.24 mmol) was used in the reaction instead of compound **RM-Ib-1,** and compound **SM1-8** (0.24 mmol) was used instead of compound SM1-1 to obtain intermediate **RM-IIb-59,** which was obtained from The intermediate was hydrolyzed to give the white solid product **IIb-61** (0.026 g) in two-step yield: 30%.

ESI-MS [(M-H)⁺]: m/z of IIb-61 calculated: 361.1, founded: 361.2.

### Example 62

### Synthesis of compound IIb-62

The synthesis of compound **IIb-62** was prepared in the same way as in Example 1, and the product **IIb-62** was obtained by etherification and hydrolysis reaction, where compound **RM-Ib-16** (0.24 mmol) was used in the reaction instead of compound **RM-Ib-1,** and compound **SM1-12** (0.24 mmol) was used instead of compound **SM1-1** to obtain intermediate **RM-IIb-60,** which was obtained from The intermediate was hydrolyzed to give the white solid product **IIb-62** (0.015 g) in two-step yield: 16%.

ESI-MS [(M-H)⁺]: m/z of IIb-62 calculated: 373.1, founded: 373.1.

### Example 63

### Synthesis of compound IIb-63

The synthesis of compound **IIb-63** was prepared in the same way as in Example 2, and the product **IIb-63** was obtained by etherification and hydrolysis reaction, where compound **RM-Ib-29** (0.24 mmol) was used in the reaction instead of compound **RM-Ib-2,** and compound **SM1-8** (0.24 mmol) was used instead of compound SM1-1 to obtain intermediate **RM-IIb-61,** which was obtained from The intermediate was hydrolyzed to give the white solid product **IIb-63** (0.020 g) in two-step yield: 17%.

ESI-MS [(M-H)⁺]: m/z of IIb-63 calculated: 476.2, founded: 476.2.

### Sample 64

### Synthesis of compound IIb-64

The synthesis method for the preparation of compound IIb-64 was the same as that of Example 2, and the product **IIb-64** was obtained by etherification and hydrolysis reaction, where compound **RM-Ib-29** (0.24 mmol) was used in the reaction instead of compound **RM-Ib-2** and compound **SM1-12** (0.24 mmol) was used instead of compound **SM1-1** to obtain intermediate **RM-IIb-62,** which was obtained from The intermediate was hydrolyzed to give the white solid product IIb-64 (0.015 g) in two-step yield: 13%.

ESI-MS [(M-H)⁺]: m/z of IIb-64 calculated: 488.2, founded: 488.3.

### Example 65

Assay for GPR40 activity of compounds:
The compounds prepared herein can be initially screened for their effect on GPR40 by the following preclinical in vitro inhibition assays, and then further confirmed in clinical trials for efficacy and safety. Other methods will be apparent to those of ordinary skill in the art.The preclinical and clinical trial results study of TAK-875, a GPR40 agonist previously developed by Takeda, Japan, showed that the results of the ex vivo test were consistent with the results of the relevant in vivo activity test.

The compounds of the present invention, or stereoisomers, tautomer, esterified or amidated precursor drugs, or pharmaceutically acceptable salts thereof, and mixtures thereof, were tested by evaluation experiments of the activity of compounds IIb-01 to IIb-64 and a reference compound Ref-1 (TAK-875) against GPR40, and comparison of the test results revealed that a number of compounds showed better inhibitory activity (EC50) than the reference compound.

Inoculate CHO-K1/GPR40 cells in 384-well plates at a density of 10,000 cells/well. Cells were incubated at 37C for 24 hours in a 5% CO ₂ incubator. After the experiment, discard the cell culture medium, quickly add 30 µl of 1X FLIPR^{®} Calcium 6 dye containing 2.5 mMProbenecid to each well and incubate at 37C for 2 hours protected from light.For the determination, different concentrations of drug (15 µl/well) were added to the wells and the fluorescence values were read. The fluorescence excitation wavelength was 494 nm and emission wavelength was 516 nm. FLIPR instrument data, **EC**₅₀ was derived from the formula Y = Bottom + (Top-Bottom)/(1+(EC50/X)^HillSlope).

The results of the GPR40 agonist activity tests for each compound of novel structure of Formula IIb are listed in Table 3 and Table 2 below; where the GPR40 agonist activity effect range (EC50) of the compounds of the present invention is labeled "A" for <10 nM; "B" for the activity range of 10-100 nM; and "C" for the activity range >100 nM.

**Table 3: Results of GPR40 agonist activity assay for compounds of formula lib**

| No. | Compound | GPR40 activity | No. | Compound | GPR40 activity |
|---|---|---|---|---|---|
| 1 | **IIb-01** | C | 2 | **IIb-02** | C |
| 3 | **IIb-03** | C | 4 | **IIb-04** | B |
| 5 | **IIb-05** | B | 6 | **IIb-06** | B |
| 7 | **IIb-07** | B | 8 | **IIb-08** | C |
| 9 | **IIb-09** | C | 10 | **IIb-10** | C |
| 11 | **IIb-11** | C | 12 | **IIb-12** | C |
| 13 | **IIb-13** | C | 14 | **IIb-14** | B |
| 15 | **IIb-15** | B | 16 | **IIb-16** | C |
| 17 | **IIb-17** | B | 18 | **IIb-18** | A |
| 19 | **IIb-19** | C | 20 | **IIb-20** | B |
| 21 | **IIb-21** | B | 22 | **IIb-22** | C |
| 23 | **IIb-23** | B | 24 | **IIb-24** | C |
| 25 | **IIb-25** | A | 26 | **IIb-26** | C |
| 27 | **IIb-27** | C | 28 | **IIb-28** | C |
| 29 | **IIb-29** | C | 30 | **IIb-30** | C |
| 31 | **IIb-31** | C | 32 | **IIb-32** | C |
| 33 | **IIb-33** | B | 34 | **IIb-34** | C |
| 35 | **IIb-35** | B | 36 | **IIb-36** | C |
| 37 | **IIb-37** | C | 38 | **IIb-38** | B |
| 39 | **IIb-39** | C | 40 | **IIb-40** | B |
| 41 | **IIb-41** | C | 42 | **IIb-42** | C |
| 43 | **IIb-43** | A | 44 | **IIb-44** | C |
| 45 | **IIb-45** | C | 46 | **IIb-46** | A |
| 47 | **IIb-47** | C | 48 | **IIb-48** | B |
| 49 | **IIb-49** | C | 50 | **IIb-50** | C |
| 51 | **IIb-51** | C | 52 | **IIb-52** | C |
| 53 | **IIb-53** | B | 54 | **IIb-54** | B |
| 55 | **IIb-55** | B | 56 | **IIb-56** | B |
| 57 | **IIb-57** | B | 58 | **IIb-58** | B |
| 59 | **IIb-59** | B | 60 | **Ref-1** | B |
| 61 | **IIb-61** | B | 62 | **IIb-62** | B |
| 63 | **IIb-63** | B | 64 | **IIb-64** | B |
| 65 | **Ref-1** | B | | | |

### Example 66

### Compound toxicity screening test:

In order to test the toxicity of some of the more active (EC50 <10nM) compounds (IIb-18, IIb-25 and IIb-43) of the new compounds (IIb-01 to IIb-64), the Acute Toxocity Study (MTD) test was conducted using a single dose of 600mg/kg administered by gavage to healthy mice at 18-22g for 5 consecutive days to assess the toxicity of the subject to the organism, the results showed that the overall toxicity of the compounds was low (LD50: >600), the survival rate of the mice after administration was 100%, no abnormalities in body weight and external and internal observations occurred during the dosing and 5-day recovery period, and the autopsy results did not reveal any abnormal changes in various organs such as heart, liver, lung, kidney and intestine. To this end, several highly active compounds of the present invention have been determined by in vitro experiments to be not only more effective in type 2 diabetes, but also safe and reliable.

### Example 67

BSEP (Bile Salt Export Pump) assay for bile salt transport effect of compounds:
In this experiment, we performed a preliminary study on the inhibition of the uptake of the substrate taurocholic acid (TCA) by the BSEP bile salt transporter by LC/MS/MS. The BSEP protein microvesicles with reverse uptake ability were obtained by transfecting Hi5 cells with the BSEP gene, which is capable of TCA uptake and uptake of taurocholic acid.The principle of this experiment is to perform substrate uptake by BSEP protein microcapsules in the presence of energy ATP, the substrate TCA and the candidate compound to be tested, after a certain transit time, the energy, substrate and candidate compound were completely washed out of the microvesicle, and the absorbed substrate TCA was released by lysis of the microvesicle.

### Sample processing:

1. Dissolve compounds individually to 100 mM using 100% DMSO and store in a -20 degree refrigerator with all compounds completely dissolved.
2. The starting concentration of the sample was 100 mM, diluted in 2-fold gradient with Bravo apparatus, with 11 concentration gradients; the lowest point concentration was 97.65 µM.
3. Control compound (Glyburide) starting at 20 mM, diluted in 2-fold gradient with Bravo instrument, with 11 concentration gradients; the lowest point concentration was 19.53 µM.
4. 300nl of compound was transferred to the middle plate using the ECHO autosampler.
5. Positive control (HPE) wells and negative control (ZPE) wells were also transferred 300nl DMSO each.

### Experimental procedure:

1. Add 14.7 µl of ATP buffer to the corresponding wells of the compound and ZPE, respectively.
2. Add 14.7 µl of AMP buffer to the corresponding wells of the HPE.
3. Shake the plate at 25 degrees C for 10 minutes.
4. Add 15ul of BSEP-Hi5-VT Vesicle solution to all wells separately and shake the plate for 40 minutes at 25 degrees Celsius.
5. Immediately add 5ul of 0.5M EDTA solution to all wells, and then add 65µl of Buffer B. All reactions are completed.
6. Transfer 95 µl of liquid from the end of reaction compound plate to the filter plate using the spiking apparatus.
7. After placing the liquid receiving plate under the filter plate, use a centrifuge to remove the liquid and discard the liquid received in the receiving plate.
8. Add 90 µl of buffer B to the filter plate and repeat step 7. Repeat steps 7 and 8 to wash the filter plate a total of three times.
9. Let the filter plate dry overnight.
10. Add 81 µl of 80% methanol/water solution to the filter plate the next day.
11. Apply membrane to the filter plate and vibrate the plate for 15 minutes.
12. Place a new liquid receiver plate under the filter plate and centrifuge for 5 min to filter all the liquid from the filter plate into the receiver plate.
13. Add 13.5 µl of internal standard solution to each well of the liquid receiver plate and seal the plate with a sealing film.
14. Using LC/MS/MS, detect the content of taurocholic acid in the receiver plate.

**Table 4: Comparison of optimized GPR40 agonist activity and its potential side effects**

| No. | Compound | GPR40 Activity (hEC50, nM) | Potential inhibitory side effects BSEP (IC50, µM) |
|---|---|---|---|
| 1 | **IIb-18** | 7.9 | 161.7 |
| 2 | **IIb-25** | 9.2 | 57.7 |
| 3 | **IIb-43** | 6.8 | 66.8 |
| 4 | **Ref-1 (TAK-875)** | 13.7 | 7.1 |

| | | | |
|---|---|---|---|
| Note: The BSEP inhibition results (IC50) for the compounds shown in Table 4 are below 25 uM [e.g., Ref-1 (TAK-875) has a value of 7.1, which is significantly below 25], then the BSEP inhibition side effects of the compound will likely lead to a potential risk of liver damage in vivo. | | | |

The results of the activity assays in Tables 3 and 4 above show that: (1) the activity (EC50) of the five-membered heterocyclic compounds containing 1-2 oxygen atoms among the various novel benzo-heterocyclic compounds of the present invention is significantly better than that of the six-membered heterocyclic compounds of similar structures containing 1-2 oxygen atoms;; (2) The activity of the benzooxygen-containingfive-membered heterocyclic compounds of the present invention when Z1 is "Oxygen(O)" is better than that of the corresponding compound when Z1 is "CH2" in the same structure;(3) The benzooxygen-containingfive-membered heterocyclic compounds IIb-18, IIb-25, IIb-43 and IIb-46 of the present invention show good activity (EC50: <10nM) against the GPR40 target, which is better than that of the similar control compound TAK-875, and are novel GPR40 agonists with good activity and safety advantages in this research field.

The three benzo oxygen-containing five-membered heterocyclic compounds listed in Table 4 above not only showed better activity but also had BSEP side effect results greater than 25 that could lead to liver damage, which was significantly better than the new drug TAK-875, which was terminated from the Phase III clinical trial by Takeda, Japan, because of liver toxicity problems, and the results of the completed Acute Toxocity Study (MTD) test showed that the three highly active compounds IIb-18, IIb-25 and IIb-43 listed in Table 4 had a better safety profile, the survival rate of the mice was 100% after 5 days of instillation of the new compound at a dose of 600 mg/kg, and no abnormalities occurred during the dosing and 5-day recovery period, and the autopsy results did not reveal any abnormalities. Therefore, several highly active compounds in the novel benzo oxygen-containing five-membered heterocyclic compound IIb designed and synthesized by the present invention are valuable for further animal and clinical testing and promotion of application.

As a result, several compounds with better activity (e.g., **IIb-18, IIb-25** and **IIb-43**) were identified in the innovative study of GPR40 target agonists, and the acute toxicity of the TMD high dose (600 mg/kg/day) test was very low, and the results of BSEP (>25uM), hERG (>30uM) and Ames assays showed no relevant side effects, and the relevant results were all superior to the currently known reference compound TAK-875, the results were better than those of the currently known reference compound TAK-875 and provided a basis for the invention of a new GPR40 target agonists with better GPR40 target selectivity and safe and efficient treatment of type II diabetic patients.

These and other changes to the embodiments may be made in accordance with the detailed description above. In general, the terms used in the claims should not be considered as limiting the claims to the specific embodiments disclosed in this specification and claims, but rather as including all possible embodiments that follow the full scope of the equivalent of the listed claims. Accordingly, the claims are not limited by this disclosure.

## Claims

1. A compound represented by formula **Ib**, or its cis-trans isomer, enantiomer, diastereoisomer, racemate, tautomer, solvate, hydrate, or pharmaceutically acceptable salt, or mixtures thereof; 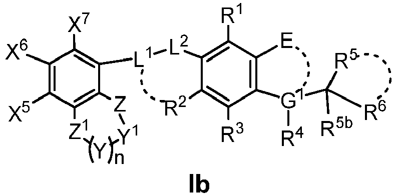
wherein, n = 0, 1 or 2;
when n = 0, Y does not exist, and Y¹ is directly single bonded to Z¹ adjacent (ortho-) to Y to form a five-membered heterocyclic group;
When E is not directly connected to G¹ to form a cyclic compound, E is selected from: hydrogen, deuterium(D), halogen, trifluoromethyl, trifluoromethoxy, nitrile, hydroxyl, carboxyl, amino(NH₂), aminocarbonyl(H₂NCO), alkyl, alkoxy, alkoxycarbonyl, alkylcarbonylamino, alkylaminocarbonyl, aryl, aryloxy, or heterocyclic aryl group; G¹ is CH, or C(Rb), wherein Rb is hydrogen, deuterium(D), alkyl, cycloalkyl, alkenyl, alkynyl, alkoxy, cycloalkoxy, alkoxycarbonyl,alkylaminyl, cycloalkylaminyl, alkylaminylcarbonyl, cycloalkylaminylcarbonyl, aryl, aryloxy, heterocyclic, heterocyclic aryl, or heterocyclic aryloxy, or Rb and R⁴ may be interconnected to form a cycloalkyl, or heterocyclic group;
When E is linked to G¹ to form a cyclic compound, G¹ is -C-; E is -O-, -C(RcRd)-, -OC(RcRd)-, -C(RcRd)O-, or -NRe-; wherein Rc and Rd are hydrogen, deuterium(D), alkyl, cycloalkyl, alkenyl, hydrocarbon, alkoxy, cycloalkoxy, alkoxycarbonyl, alkylaminyl, cycloalkylaminyl, alkylaminylcarbonyl, cycloalkylaminylcarbonyl, aryl, aryloxy, heterocyclic, heterocyclic aryl, or heterocyclic aryloxy, respectively, and Rc and Rd may be interconnected to form cycloalkyl, or heterocyclic groups; Re is hydrogen, deuterium(D), alkyl, cycloalkyl, alkylcarbonyl, alkoxycarbonyl, cycloalkoxycarbonyl, alkylaminylcarbonyl, cycloalkylaminylcarbonyl, alkylsulfonyl, or aryl sulfonyl group;L¹ and L² are each independently -O-, -S-, -C(O)-, -S(O)₂-, -CH₂-, -C R_{f}R_{g})-, -OC(R_{f}R_{g})-, -C(R_{f}R_{g})O-, -N(Re)-, -N(Rc)C(R_{f}R_{g})-, or -C(R_{f}R_{g})N(Re)-; wherein R_{f} and R_{g} are hydrogen, deuterium(D), alkyl, cycloalkyl, alkenyl, alkynyl, alkoxy, cycloalkoxy, alkoxycarbonyl, alkylaminyl, cycloalkylaminyl, alkylaminylcarbonyl, cycloalkylaminylcarbonyl, aryl, aryloxy, heterocyclic, heterocyclic aryl, or heterocyclic aryloxy, respectively, and Re is defined as described above for Re in E;
R¹, R² and R³ are each independently hydrogen, deuterium(D), halogen, trifluoromethyl, trifluoromethoxy, nitrile, hydroxyl, aminocarbonyl (H₂NCO), alkyl, alkoxy, alkoxycarbonyl, alkylaminocarbonyl, alkylcarbonylamino, aryl, aryloxy, or heterocyclic aryl group; wherein R² and L¹ in formula **Ib** can be interconnected to form a 4-8-membered heterocyclic compound, andL¹is -CH-;
R⁴, R⁵ and R^{5b} are each independently hydrogen, deuterium(D), halogen, hydroxyl, amino, nitrile, alkyl, alkoxy, cycloalkyl, heterocycloalkyl, cycloalkoxy, optionally substituted alkenyl, optionally substituted alkynyl, alkoxycarbonyl, alkylaminocarbonyl, alkylcarbonylamino, alkoxycarbonylamino, aryl, aryloxy, or heterocyclic aryl; wherein R⁵ and R^{5b} may be interconnected to become cycloalkyl, heterocyclic, or heterocyclic aryl group;
R⁶ is a carboxyl, alkoxycarbonyl, aryloxycarbonyl, alkylaminylcarbonyl, cycloalkylaminylcarbonyl, alkylsulfonamidocarbonyl, cycloalkylsulfonamido- carbonyl, heterocyclic, or heterocyclic aryl; or R⁶ and the adjacent (ortho-) substituent R⁵ may be interconnected to become heterocyclic, or heterocyclic aryl;
X⁵, X⁶ and X⁷ are each independently hydrogen, deuterium(D), halogen, nitrile, amino, trifluoromethyl, trifluoromethoxy, aminocarbonyl (H₂NCO), alkyl, heterocycloalkyl, alkoxy, heteroatomically substituted alkoxy, alkylamino (NRᵢRⱼ), heteroatomically substituted alkylamino, alkoxycarbonyl, alkylaminocarbonyl, alkylcarbonylamino, alkoxycarbonylamino, cyclo alkoxycarbonylamino group, alkyl sulfonamido group, cycloalkyl sulfonamido group, aryl, aryloxy, aryl amido carbonyl, aryl carbonyl amido, aryloxy carbonyl amido, heterocyclic aryl, heterocyclic aryloxy, or heterocyclic aryl amido group; wherein Rᵢ and Rⱼare each independently hydrogen, deuterium(D), alkyl, heterocycloalkyl, alkylcarbonyl, alkoxycarbonyl, cycloalkoxycarbonyl, alkylaminocarbonyl, alkylsulfonyl, cycloalkylsulfonyl, aryl, aryloxycarbonyl, arylaminocarbonyl, heterocycloaryl group, or Rᵢ and Rⱼ are interconnected to form a 3-8-membered heterocyclic group containing 1-3 heteroatoms;
Y and Y¹ are each independently -O-, -S-, -CH₂-, -CHF-, -CF₂-, -CCl₂-, -C(R_{f}R_{g}-, or -N(Re)-; wherein R_{f} and R_{g} are defined as described above for R_{f} and R_{g} in L¹, respectively, and Re is defined as described above for Re in E;
Z and Z¹ are each independently selected from: -O-, -S-, -CH₂-, -CHF-, -CF₂-, -C (R_{f}R_{g})-, -N(Re)-, or -C(O)-; wherein R_{f} and R_{g} are defined as described above for R_{f} and R_{g} in L¹, respectively, and Re is defined as described above for Re in E.

2. The compound according to claim 1, or its cis-trans isomer, enantiomer, diastereoisomer, racemate, tautomer, solvate, hydrate, or a pharmaceutically acceptable salt, or a mixture thereof, wherein thecompound has the structure of **IIb**; 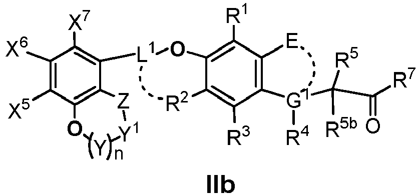 wherein,
The definitions of n, E, G¹, L¹, R¹, R², R³, R⁴, R⁵, R^{5b}, X⁵, X⁶, X⁷, Y, Y¹, Z are the same as the definitions of n, E, G¹, L¹, R¹, R², R³, R⁴, R⁵, R^{5b}, X⁵, X⁶, X⁷, Y, Y¹, Z in claim 1;
R⁷ is a hydroxyl, an alkoxy, an alkyl amino, a cycloalkyl amino, a heterocyclic amino, an alkyl sulfonamido, a cycloalkyl sulfonamido, an aryloxy, a heterocyclic aryloxy, an aryl amino, or a heterocyclic aryl amino; or R⁷and the adjacent (ortho-) substituent R⁵ can be interconnected to become a heterocyclic group.

3. The compound according to claim 2, wherein,
n = 0 or 1;
When n = 0, Y does not exist and Y¹ is directly single bonded to the oxygen adjacent (ortho-) to Y to form a five-membered heterocyclic group;
when n = 1, Y is -CH₂-;
When E is not directly connected to G¹ to form a cyclic compound, E is hydrogen, halogen, trifluoromethyl, trifluoromethoxy, or alkoxy; and G¹ is -CH-, or -C(Rb)-, wherein Rb is hydrogen, alkyl, optionally substituted alkenyl, optionally substituted alkynyl, alkoxy, or Rb and R⁴ are interconnected to form an oxygen-containing heterocyclic group; R⁴ is hydrogen, alkyl, alkoxy, optionally substituted alkynyl, or R⁴ and Rb are interconnected into an oxygen-containing heterocyclic group;
When E is directly connected to G¹ to form a cyclic compound, E is -OC(RcRd)-, G¹ is -C-, and R⁴ is hydrogen, where Rc and Rd are each independently hydrogen;
L¹ is -CH₂-, or L¹ is -OCH- when R² and L¹ in Formula **IIb** are interconnected to form a 5~6-membered heterocyclic compound;
R¹, R² and R³ are each independently hydrogen, halogen, alkyl or alkoxy group;
R⁵ is hydrogen, halogen, hydroxyl, amino, alkylamino, alkyl or alkoxy group;
R^{5b} is hydrogen, halogen, alkyl or alkoxy group;
R⁷ is an alkoxy, hydroxy, alkyl sulfonamido, or cycloalkyl sulfonamido group; X⁵ is hydrogen, deuterium(D), halogen, nitrile, amino, trifluoromethyl, trifluoromethoxy, alkyl, alkoxy, alkylamino (NRᵢRⱼ), alkylcarbonylamino, alkylaminocarbonylamino, alkoxycarbonylamino, cycloalkoxycarbonylamino, alkylsulfonamido, cycloalkylsulfonamido, aryl, or aryloxycarbonylamino group; wherein Rᵢ and Rⱼ are each independently hydrogen, alkyl, alkylcarbonyl, alkoxycarbonyl, or cycloalkoxycarbonyl group;
X⁶ and X⁷ are each independently hydrogen, deuterium(D), halogen, Ci-Cs alkylamine group or C₁-C₈ alkoxycarbonylamino group;
Y is -CH₂-, or Y does not exist when n = 0;
Y¹ is -CH₂-, -CHF-, -CF₂-, or -C(CH₃)₂-;
Z is -O-, or -CH₂-.

4. The compound according to claim 3, wherein,
When n = 0, Y does not exist;
When E is not directly connected to G¹ to form a cyclic compound, E is hydrogen, or halogen; G1 is -CH-, or -C(Rb)-, wherein Rb is alkyl, alkenyl, alkynyl, or alkoxy; and R⁴ is hydrogen, alkyl, or alkoxy group;
When E is directly connected to G¹ to form a cyclic compound, E is -OC(RcRd)-, G¹ is -C-, and R⁴ is hydrogen, wherein Rc and Rd are each independently hydrogen;
L¹ is -CH₂-, or L¹ is -OCH- when R² and L¹ in Formula **IIb** are interconnected to form a 5-6-membered heterocyclic compound;
R¹, R² and R³ are each independently hydrogen, halogen, or alkoxy group;
R⁵ is hydrogen, halogen, hydroxyl, amino, alkyl or alkoxy group;
R^{5b} is hydrogen, halogen, alkyl or alkoxy;
R⁷ is selected from: alkoxy, hydroxy, alkyl sulfonamido, or cycloalkyl sulfonamido group;
X⁵ is hydrogen, deuterium(D), halogen, nitrile, amino, trifluoromethyl, trifluoromethoxy, alkyl, alkoxy, alkylamino (NRᵢRⱼ), alkylcarbonylamino, alkylaminocarbonylamino, alkoxycarbonylamino, cycloalkoxycarbonylamino, alkylsulfonamido, cycloalkylsulfonamido, aryl, or aryloxycarbonylamino group; wherein Rᵢ and Rⱼ are each independently hydrogen, alkyl, alkylcarbonyl, alkoxycarbonyl, or cycloalkoxycarbonyl group;
X⁶ is hydrogen, deuterium(D), halogen, Ci-Cs alkylamino or Ci-Cs alkoxycarbonylamino group;
X⁷ishydrogen;
Y¹ is -CH₂-, -CHF-, -CF₂-, or -C(CH₃)₂-;
Z is -O-.

5. The compound according to claim 2, which is represented by one of the following structures:

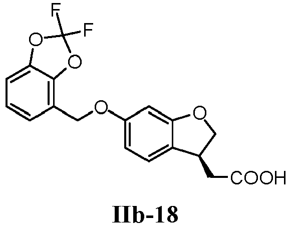

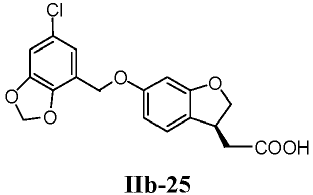

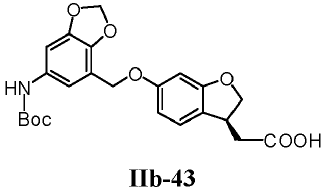

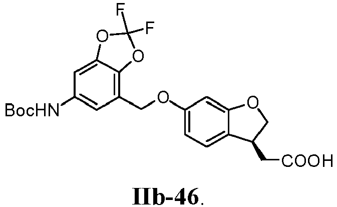

6. A composition comprising a compound according to one of claims 1-5 or a pharmaceutically acceptable salt thereof, and a pharmaceutically acceptable diluent and/or excipient.

7. The use of the compound according to one of claims 1-5 or the composition according to claim 6, which is used as a GPR40 agonist.

8. A method of treating or preventing diabetes or a related metabolic syndrome, **characterized by** administering to a patient an effective dose of the compound according to one of claims 1-5 or a composition according to claim 6.

9. The use of a compound according to one of claims 1-5 or the composition according to claim 6 in the preparation of a medicament for the treatment or prevention of diabetes or a related metabolic syndrome.

10. The use according to claim 9, the diabetes are type II diabetes.
